(19) Europäisches Patentamt
European Patent Office
Office européen des brevets

(11) EP 0 663 393 B1

(12) EUROPÄISCHE PATENTSCHRIFT

(45) Veröffentlichungstag und Bekanntmachung des Hinweises auf die Patenterteilung:
**05.07.2000 Patentblatt 2000/27**

(21) Anmeldenummer: **94120924.9**

(22) Anmeldetag: **30.12.1994**

(51) Int. Cl.7: **C07D 207/12**, C07D 207/14, C07D 211/40, C07D 211/56, C07D 223/12, C07D 223/08, C07D 401/12, C07D 405/12, C07D 417/12, A61K 31/40, A61K 31/445, A61K 31/55

(54) **3-Amino/Hydroxy-4-[4-Benzoyl-Phenyl Carbonylamino/oxy]-Azepane und Homologe als Protein Kinase Hemmer**

3-Amino/hydroxy-4- 4-benzoylphenylcarboxylamino/oxy -azepanes and their homologies as protein kinase inhibitors

3-Amino/hydroxy-4- 4-benzoylphénylcarboxylamino/oxy -azépanes et leurs homologues comme inhibiteurs de protéine kinase

(84) Benannte Vertragsstaaten:
**AT BE CH DE DK ES FR GB GR IE IT LI LU MC NL PT SE**

(30) Priorität: **12.01.1994 CH 8894**

(43) Veröffentlichungstag der Anmeldung:
**19.07.1995 Patentblatt 1995/29**

(73) Patentinhaber:
**F. HOFFMANN-LA ROCHE AG**
**4002 Basel (CH)**

(72) Erfinder:
- **Barbier, Pierre**
  **F-68170 Rixheim (FR)**
- **Huber, Isabelle**
  **CH-1213 Petit-Lancy, Geneva (CH)**
- **Schneider, Fernand**
  **CH-4051 Basle (CH)**
- **Stadlwieser, Josef**
  **CH-4051 Basle (CH)**
- **Taylor, Sven**
  **F-68400 Riedisheim (FR)**

(74) Vertreter:
**Witte, Hubert, Dr. et al**
**F.Hoffmann-La Roche AG**
**Patent Department (PLP),**
**124 Grenzacherstrasse**
**4070 Basel (CH)**

(56) Entgegenhaltungen:
**WO-A-93/03730**

- **LIEBIGS ANNALEN DER CHEMIE, Nr.6, 1993 Seiten 651 - 655 Mueller, Alexander; Takyar, Dinesh K.; Witt, Sandra; Koenig, Wilfried A 'Synthesis of (3R,4R)-3-amino-4-hydroxyhex ahydroazepine, the chiral constituent of the antibiotic ophiocordin'**
- **CHEM. BERICHTE, Bd.113, 1980 Seiten 2221 - 2226 Koenig, Wilfried A.; Sinnwell, Volker; Witt, Sandra; Kneifel, Helmut 'Metabolites of microorganisms. 195. The structure of ophiocordin, an antibiotic with antifungal properties'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Xerox (UK) Business Services
2.16.7 (HRS)/3.6

**Beschreibung**

Die vorliegende Erfindung betrifft neue Azepane und deren Ringhomologe der allgemeinen Formel

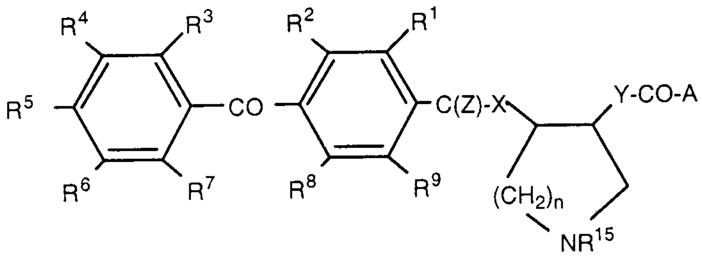

I

worin

A einen Rest

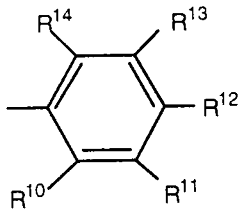

$A^1$

2-, 3- oder 4-Pyridyl oder 2- oder 3-Piperazinyl, oder durch eine oder mehrere nieder-Alkyl-, nieder-Alkoxy- und/oder Hydroxygruppen substituiertes 2-,3- oder 4-Pyridyl oder 2- oder 3-Piperazinyl;

X und Y unabhängig voneinander Sauerstoff oder NH, jedoch nicht beide NH bedeuten;

Z Sauerstoff oder, falls X Sauerstoff ist, Sauerstoff oder H,H bedeutet;

n 1, 2 oder 3 ist;

$R^1$ Wasserstoff oder Fluor;

$R^2$ Wasserstoff, $C_{1-6}$-Alkoxy oder Fluor;

$R^3$ Wasserstoff, Hydroxy, $C_{1-6}$-Alkoxy, Fluor, Trifluormethyl, $C_{2-7}$-Alkoxycarbonyl, Tetrazolyl oder durch $C_{1-6}$-Alkyl, Benzyl, Cyanomethyl oder Carbamoyl-methyl substituiertes Tetrazolyl;

$R^4$ Wasserstoff, Hydroxy, $C_{1-6}$-Alkoxy, $C_{1-6}$-Alkyl, Chlor, Fluor, Trifluormethyl, Acetyl, Di-$C_{1-6}$-alkylamino, oder $C_{1-6}$-Alkoxy-$C_{1-6}$-alkyl, $C_{1-6}$-Alkylthio, $C_{1-6}$-Alkylsulfonyl, Phenyl, Pyrrolidinyl oder Piperidinyl;

$R^5$ Wasserstoff, $C_{1-6}$-Alkoxy, Fluor oder Trifluormethyl;

$R^6$ Wasserstoff, Hydroxy, $C_{1-6}$-Alkoxy, Fluor, 2,4-Difluorphenyl, $C_{1-6}$-Alkoxy-$C_{1-6}$-alkyl, $C_{1-6}$-Alkanoyl, Benzoyl, Nitrilo, Trifluormethyl, $C_{3-6}$-Cycloalkyl, 2- oder 4-Thiazolyl, 2-Thiazolidinyl, 2-Oxazolyl oder 2-Oxazolidinyl, 2- oder 4-Imidazolyl;

$R^7$ Wasserstoff, Hydroxy, $C_{1-6}$-Alkoxy, Carboxy, Amino oder Fluor;

$R^6$ und $R^7$ gemeinsam einen Rest -N=CH-CH=N- oder $-N(CH_3)CH_2CH_2N(CH_3)-$;

$R^8$ Wasserstoff $C_{1-6}$-Alkoxy, $C_{1-6}$-Alkyl oder Fluor;

$R^9$ Wasserstoff oder Fluor;

$R^{10}$ Wasserstoff, Hydroxy, $C_{1-6}$-Alkoxy oder $C_{1-6}$-Alkyl;

$R^{11}$ Wasserstoff, $C_{1-6}$-Alkoxy, $C_{1-6}$-Alkyl, Fluor oder Brom;

$R^{12}$ Wasserstoff, Hydroxy, $C_{1-6}$-Alkoxy, Carboxy, $C_{2-7}$-Alkoxycarbonyl oder Amino;

$R^{13}$ Wasserstoff, Hydroxy, $C_{1-6}$-Alkoxy, $C_{1-6}$-Alkyl, Acetyl oder Fluor;

$R^{14}$ Wasserstoff, $C_{1-6}$-Alkyl oder Fluor;

$R^{15}$ Wasserstoff oder Amidino;

$R^3$ und $R^4$ gemeinsam einen Rest -CH=CH-CH=CH- oder Aethylendioxy;

$R^4$ und $R^5$ gemeinsam einen Rest -CH=CH-CH=CH-, Tetramethylen, Methylendioxy, Aethylendioxy oder einen Rest -N=CH-CH=CH- oder einen Rest (a)

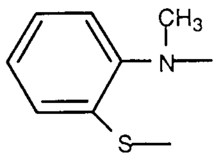

(a)

in der die Bindung zum S durch $R^5$ erfolgt; und

$R^{12}$ und $R^{13}$ gemeinsam einen Rest -CH=CH-CH=CH- oder -C(OH)=CH-CH=CH-, in der die Bindung zu dem die Hydroxygruppe tragenden C-Atom durch $R^{12}$ erfolgt;

bedeuten; und pharmazeutisch anwendbare Salze davon.

Der hier verwendete Ausdruck " $C_{1-6}$" bezeichnet Gruppen mit 1-6, vorzugsweise 1-4 C-Atomen. Alkyl- und Alkoxygruppen können geradkettig oder verzweigt sein, wie Methyl, Ethyl, Propyl, Isopropyl, n-Butyl, sek.- und tert.-Butyl, Pentyl und Hexyl bzw. Methoxy, Ethoxy, Propoxy, Isopropoxy, n-Butoxy, sek. und tert.Butoxy, Pentyloxy und Hexyloxy. Alkanoyl bezeichnet Säurereste aliphatischer, gesättigter und ungesättigter Carbonsäuren deren Kohlenstoffreste geradkettig oder verzweigt sein kann.

Eine bevorzugte Gruppe von Verbindungen der Formel I sind diejenigen, in denen $R^1$ und $R^9$ Wasserstoff, $R^2$ und $R^8$ Wasserstoff oder Fluor, und $R^7$ Wasserstoff, Hydroxy, $C_{1-6}$-Alkoxy, Amino oder Fluor darstellen.

Bevorzugte Verbindungen der Formel I sind ferner solche, in denen X Sauerstoff und Y NH ist, sowie solche, in denen Z Sauerstoff ist.

Weiterhin sind Verbindungen der Formel I bevorzugt, in denen n = 3 ist.

In bezug auf die Reste $R^1$-$R^{14}$ sind Verbindungen bevorzugt, in denen $R^1$, $R^2$, $R^8$ und $R^9$ Wasserstoff sind; oder $R^2$ und $R^8$ Fluor und $R^1$ und $R^9$ Wasserstoff sind; $R^7$ Hydroxy ist; $R^3$ Wasserstoff, $C_{1-6}$-Alkyl, $C_{1-6}$-Alkoxy, Fluor oder Cyanomethyltetrazolyl; $R^4$ Wasserstoff, $C_{1-6}$-Alkyl, $C_{1-6}$-Alkoxy, $C_{1-6}$-Alkylthio, $C_{1-6}$-Alkoxy- $C_{1-6}$-alkyl, Di($C_{1-6}$-alkyl)-amino, $C_{2-7}$-Alkanoyl, Phenyl, Pyrrolidino oder Fluor; $R^5$ Wasserstoff, $C_{1-6}$-Alkoxy, Fluor oder Trifluormethyl; $R^6$ Wasserstoff; $R^4$ und $R^5$ gemeinsam Tetramethylen, Methylendioxy, Aethylendioxy oder einen Rest -CH=CH-CH=CH-, -CH=CH-CH=N- oder

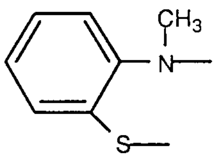

(a); und

$R^3$ und $R^4$ gemeinsam Aethylendioxy bedeuten; $R^{10}$ Wasserstoff, Hydroxy oder $C_{1-6}$-Alkyl; $R^{11}$ Wasserstoff, $C_{1-6}$-Alkyl, $C_{1-6}$-Alkoxy oder Brom; $R^{12}$ Wasserstoff, Hydroxy oder $C_{1-6}$-Alkoxy; $R^{13}$ Wasserstoff, $C_{1-6}$-Alkyl, $C_{1-6}$-Alkoxy, Fluor oder Acetyl; $R^{14}$ Wasserstoff oder $C_{1-6}$-Alkyl; $R^{12}$ und $R^{13}$ gemeinsam einen Rest -CH=CH-CH=CH- oder -C(OH)=CH-CH=CH-, in der die Bindung zu dem die Hydroxygruppe tragenden C-Atom durch $R^{12}$ erfolgt.

Bevorzugte Reste A sind Reste der Formel $A^1$ oder 4-Pyridyl. $R^{15}$ ist vorzugsweise Wasserstoff.

Von besonderem Interesse sind Verbindungen der Formel I, in denen A ein Rest $A^1$ und entweder

$R^{12}$ Hydroxy und mindestens einer der Reste $R^{10}$, $R^{11}$, $R^{13}$ und $R^{14}$ $C_{1-6}$-Alkyl ist; oder

$R^{12}$ Hydroxy und $R^{10}$, $R^{11}$, $R^{13}$ und $R^{14}$ Wasserstoff sind, oder mindestens einer der Reste $R^{10}$-$R^{14}$ $C_{1-6}$ Alkoxy ist; oder einer der Reste $R^{11}$, $R^{13}$ und $R^{14}$ Fluor oder $R^{11}$ Brom ist;
oder $R^{12}$ und $R^{13}$ gemeinsam einen Rest -CH=CH-CH=CH oder -C(OH)=CH-CH=CH, bilden, wobei die Bindung zu dem die Hydroxygruppe tragenden C-Atom durch $R^{12}$ erfolgt.

Ein Beispiel einer Verbindung der oben zuerst erwähnten Gruppe mit $R^{12}$ = Hydroxy und $R^{13}$ und $R^{14}$ = $C_{1-6}$-Alkyl ist der 4-(2-Fluoro-6-hydroxy-3-methoxy-benzoyl)-benzoesäure (3R,4R)-3-(4-hydroxy-3,5-dimethylbenzoylamino)-azepan-4-yl) ester.

Die Substituenten an dem in der Formel I dargestellten heterocyclischen Ring können trans- oder cis-Konfiguration haben. Bevorzugt ist die trans-Konfiguration.

Die Verbindungen der Formel I und ihre Salze können erfindungsgemäss dadurch hergestellt werden, dass man aus einer Verbindung der Formel

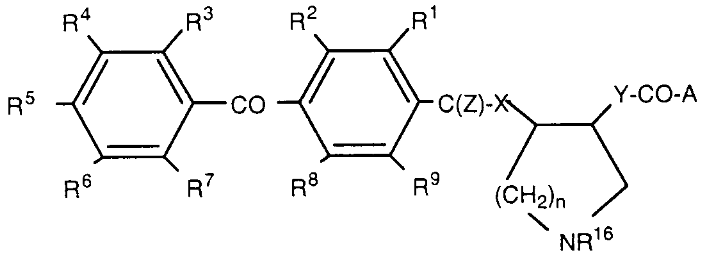

II

worin $R^{16}$ eine Schutzgruppe ist und die übrigen Symbole die oben angegebene Bedeutung haben, wobei durch $R^3$, $R^4$, $R^6$, $R^7$, $R^{12}$ und $R^{13}$ dargestellte Hydroxy- und Aminogruppen in geschützter Form vorliegen können,

die Schutzgruppe $R^{16}$ und gegebenenfalls als $R^3$, $R^4$, $R^6$, $R^7$, $R^{12}$ und $R^{13}$ anwesende Hydroxy- und Aminoschutzgruppen abspaltet,
gewünschtenfalls die erhaltene Verbindung der Formel I, in der $R^{15}$ Wasserstoff darstellt, in eine Verbindung der Formel I, in der $R^{15}$ Amidino darstellt, überführt, und gewünschtenfalls eine erhaltene Verbindung der Formel I in ein pharmazeutisch anwendbares Salz überführt.

Beispiele von Schutzgruppen $R^{16}$ und von in den Substituenten $R^7$ und $R^{12}$ anwesenden Aminoschutzgruppen sind die zum Schutz von Aminogruppen bekannten Gruppen wie tert.-Butoxycarbonyl. Beispiele von Hydroxyschutzgruppen sind Methoxymethyl und Silylgruppen wie tert.Butyldimethyl-silanyl.

Die Abspaltung dieser Schutzgruppen kann in an sich bekannter Weise durch Behandlung mit Säuren, z.B. Mineralsäuren wie HCl in einem inerten organischen Lösungsmittel, z.B. einem Aether wie Dimethoxyethan oder einem Alkohol wie Isopropanol oder Gemischen solcher Lösungsmittel. Die Abspaltung der Schutzgruppen erfolgt zweckmässig bei herabgesetzten Temperaturen, vorzugsweise bei Temperaturen unterhalb Raumtemperatur, insbesondere bei etwa 0°C. Die Einführung einer Amidinogruppe in eine Verbindung der Formel I, in der $R^{15}$ Wasserstoff ist, kann in an sich bekannter Weise, z.B. durch Umsetzung mit Formamidinderivaten wie Formamidinsulfonsäure erfolgen.

Die Verbindungen der Formel I bilden Salze, in die sie in an sich bekannter Weise überführt werden können. Beispiele pharmazeutisch anwendbarer Salze von Verbindungen der Formel I sind Säureadditionssalze von Mineralsäuren, insbesondere Hydrochloride.

Verbindungen der Formel II, in denen Z und X Sauerstoff darstellen, können aus Verbindungen der Formel

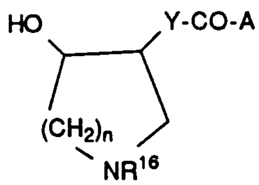

III

in der Y, A, $R^{16}$ und n die oben angegebene Bedeutung haben,
durch Umsetzung mit einer Säure der Formel

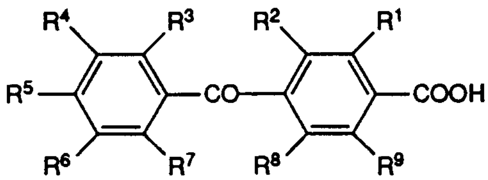

IV

in der $R^1$-$R^9$ die oben angegebene Bedeutung haben,
oder einem reaktiven Derivat davon erhalten werden.

Verbindungen der Formel II, in denen X NH bedeutet, können aus Verbindungen der Formel

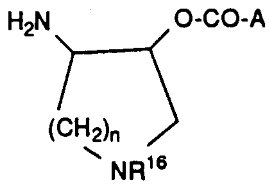

V

in der A, $R^{16}$ und n die oben angegebene Bedeutung haben,
durch Umsetzung mit einer Verbindung der Formel III oder einem reaktiven Derivat davon erhalten werden.

Verbindungen der Formel II, in denen Z H,H und Y NH bedeuten können aus Verbindungen der Formel

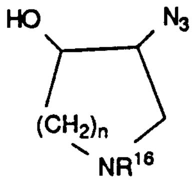

VI

in der $R^{16}$ und n die oben angegebene Bedeutung haben,
durch Umsetzung mit einer Verbindung der Formel

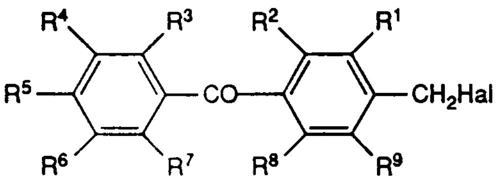

VII

in der Hal Halogen, insbesondere Brom bedeutet, und $R^1$-$R^9$ die oben angegebene Bedeutung haben,
und anschliessende Umsetzung des erhaltenen Aethers mit einer Säure der Formel A-COOH erhalten werden.

Beispiele für die Herstellung der Verbindungen der Formel II sind nachstehend ausführlicher angegeben. Anders substituierte Verbindungen der Formel II können in Analogie zu diesen Beispielen hergestellt werden.

A. Die in den Beispielen 1-64 eingesetzten Ausgangsstoffe wurden durch Veresterung eines Alkohols (siehe Beispiele B) mit einer durch Sulfonylchlorid oder Carbodiimide aktivierten Säure (siehe Beispiele C) wie folgt hergestellt:

Zu einem Gemisch von 445 mg (3R,4R)-3-[4-[dimethyl-(1,1,2-trimethylpropyl)-silanyloxy]-benzoylamino]-4-hydroxy-azepan-1-carbonsäure-tert-butylester 350 mg 3,5-Difluoro-4-(5-methoxy-2-methoxymethoxy-benzoyl)-benzoesäure (Beispiel C 26) und 33 mg N,N-Dimethylaminopyridin in Dichloromethan wurden 207 mg 1-(3-Dimethylaminopropyl)-3-ethylcarbodiimid- hydrochlorid gegeben. Das Gemisch wurde über Nacht bei Raumtemperatur gerührt, in eine pH 7 Pufferlösung gegossen und die organischen Bestandteile mit Aethylazetat extrahiert. Die vereinigten Extrakte wurden mit Kochsalzlösung gewaschen, getrocknet und eingedampft. Der ölige Rückstand wurde durch Säulenchromatographie an Silicagel (Eluens: Hexan/Aethylazetat 2:1) gereinigt und lieferte 760 mg (3R,4R)-4-[(3,5-Difluoro-4-(5-methoxy-2-methoxymethoxy-benzoyl)benzoyloxy]-3-[4-[dimethyl-(1,1,2-trimethylpropyl)-silanyloxy]-benzoylamino]-4-hydroxy-azepan-1-carbonsäure-tert-butylester als farblosen Schaum, der in dieser Form weiter verwendet wurde.

In Analogie wurden hergestellt:

A 2 (3R,4R)-4-[4-(2-Fluoro-4-methoxy-6-methoxymethoxy-benzoyl)-benzoyloxy]-3-(4-methoxymethoxy-benzoylamino)-azepan-1-carbonsäure-tert-butylester als farbloser Schaum aus (3R,4R)-4-Hydroxy-3-(4-methoxymethoxy-benzoylamino)-azepan-1-carbonsäure-tert-butylester (Beispiel B 2) und 4-(2-Fluoro-4-methoxy-6-methoxymethoxy-benzoyl)-benzoesäure (Beispiel C 1)

MS: m/e=711.7 $(M+H)^+$

IR (KBr): 1719, 1668, 1623, 1579, 1501 $cm^{-1}$

A 3 (3R,4R)-4-[4-(2-Fluoro-6-methoxymethoxy-benzoyl)-benzoyloxy]-3-(3-methoxy-4-methoxymethoxy-benzoylamino)-azepan-1-carbonsäure-tert-butylester als farbloser Schaum aus (3R,4R)-4-Hydroxy-3-(3-methoxy-4-methoxymethoxy-benzoylamino)-azepan-1-carbonsäure-tert-butylester (B3) und 4-(2-Fluoro-6-methoxymethoxy-benzoyl)-benzoesäure (C2)

MS: m/e= 711.4 $(M+H)^+$
IR (KBr): 1720, 1683, 1613, 1583, 1503 $cm^{-1}$

A 4 (3R,4R)-4-[4-(2-Fluoro-6-methoxymethoxy-benzoyl)-benzoyloxy]3-(5-methoxy-2-methoxymethoxy-benzoylamino)-azepan-1-carbonsäure-tert-butylester als farbloser Schaum aus (3R,4R)-4-hydroxy-3-(5-methoxy-2-methoxymethoxy-benzoylamino)-azepan-1-carbonsäure-tert-butylester (B4) und 4-(2-Fluoro-3-methoxy-6-methoxymethoxy-benzoyl)-benzoesäure (C3)

A 5 (3R,4R)-3-[4-(tert-Butyldimethylsilanyloxy)-benzoylamino]-4-(3-methoxymethoxy-naphthalin-2-ylcarbonyl)-benzoyloxy]-azepan-1-carbonsäure-tert-butylester als farbloser Schaum aus (3R,4R)-3-[4-(tert-Butyldimethylsilanyloxy)-benzoylamino]-4-hydroxy-azepan-1-carbonsäure-tert-butylester (B5) und 4-(3-Methoxymethoxy-naphthalin-2-ylcarbonyl)-benzoesäure (C4)

A 6 (3R,4R)-4-[4-(2-Fluoro-4-methoxy-6-methoxymethoxy-benzoyl)-benzoyloxy]-3-(3,5-dimethoxy-4-methoxymethoxy-benzoylamino)-azepan-1-carbonsäure-tert-butylester als farbloser Schaum aus (3R,4R)-4-Hydroxy-3-(3,5-dimethoxy-4-methoxymethoxy-benzoylamino)-azepan-1-carbonsäure-tert butylester (B6) und 4-(2-Fluoro-4-methoxy-6-methoxymethoxy-benzoyl)-benzoesäure (C1)

MS: m/e= 771.5 $(M+H)^+$
IR (KBr): 1720, 1672, 1621, 1582, 1495 $cm^{-1}$

A 7 (3R,4R)-4-[4-(2-Fluoro-6-methoxymethoxy-benzoyl)-benzoyloxy]-3-(3,5-dimethoxy-4-methoxymethoxy-benzoylamino)-azepan-1-carbonsäure-tert-butylester als farbloser Schaum aus (3R,4R)-4-Hydroxy-3-(3,5-dimethoxy-4-methoxymethoxy-benzoylamino)-azepan-1-carbonsäure-tert butylester (B6) und 4-(2-Fluoro-6-methoxymethoxy-benzoyl)-benzoesäure (C2)

MS: m/e= 741.5 $(M+H)^+$
IR (KBr): 1720, 1680, 1610, 1586, 1493 $cm^{-1}$

A 8 (3R,4R)-4-[4-(2,3-Difluoro-6-methoxymethoxy-benzoyl)benzoyloxy]-3-(3,5-dimethoxy-4-methoxymethoxy-benzoylamino)-azepan-1-carbonsäure-tert-butylester als farbloser Schaum aus (3R,4R)-4-Hydroxy-3-(3,5-dimethoxy-4-methoxymethoxy-benzoylamino)-azepan-1-carbonsäure-tert butylester (B6) und 4-(2,3-Difluoro-6-methoxymethoxy-benzoyl)-benzoesäure (C5)

MS: m/e= 759.5 $(M+H)^+$
IR (KBr): 1721, 1684, 1587, 1494 $cm^{-1}$

A 9 (3R,4R)-4-[4-(2,4-Difluoro-6-methoxymethoxy-benzoyl)-benzoyloxy]-3-(3,5-dimethoxy-4-methoxymethoxy-benzoylamino)-azepan-1-carbonsäure-tert-butylester als farbloser Schaum aus (3R,4R)-4-Hydroxy-3-(3,5-dimethoxy-4-methoxymethoxy-benzoylamino)-azepan-1-carbonsäure-tert butylester (B6) und 4-(2,4-Difluoro-6-methoxymethoxy-benzoyl)-benzoesäure (C6)

MS: m/e= 759.5 $(M+H)^+$
IR (KBr): 1720, 1673, 1606, 1493 $cm^{-1}$

A 10(3R,4R)-4-[4-(2-Fluoro-3-methoxy-6-methoxymethoxy-benzoyl)-benzoyloxy]-3-(4-methoxymethoxy-benzoylamino)-azepan-1-carbonsäure-tert-butylester als farbloser Schaum aus (3R,4R)-4-Hydroxy-3-(4-methoxymethoxy-benzoylamino)-azepan-1-carbonsäure-tert-butylester (B6) und (2-Fluoro-3-methoxy-6-methoxymethoxy- benzoyl)-benzoesäure (C3)

MS: m/e= 711.6 $(M+H)^+$

IR (KBr): 1720, 1682, 1606, 1493 $cm^{-1}$

A 11 (3R,4R)-4-[4-(2-Fluoro-3-methoxy-6-methoxymethoxy-benzoyl)-benzoyloxy]-3-(3-methoxy-4-methoxymethoxy-benzoylamino)-azepan-1-carbonsäure-tert-butylester als farbloser Schaum aus (3R,4R)-4-Hydroxy-3-(3-methoxy-4-methoxymethoxy-benzoylamino)-azepan-1-carbonsäure-tert-butylester (B3) und 4-(2-Fluoro-3-methoxy-6-methoxymethoxy-benzoyl)-benzoesäure (C3)

MS: m/e= 741.6 $(M+H)^+$
IR (KBr): 1720, 1683, 1600, 1493 $cm^{-1}$

A 12 (3R,4R)-4-[4-(2-Fluoro-3-methoxy-6-methoxymethoxy-benzoyl)-benzoyloxy]-3-(3,5-dimethoxy-4-methoxymethoxy-benzoylamino)-azepan-1-carbonsäure-tert-butylester als farbloser Schaum aus (3R,4R)-4-Hydroxy-3-(3,5-dimethoxy-4-methoxymethoxy-benzoylamino)-azepan-1-carbonsäure-tert butylester (B6) und 4-(2-Fluoro-3-methoxy-6-methoxymethoxy-benzoyl)-benzoesäure (C3)

MS: m/e= 771.6 $(M+H)^+$
IR (KBr): 1721, 1679, 1586, 1493 $cm^{-1}$

A 13 (3R,4R)-4-[4-(2-Fluoro-3-methoxy-6-methoxymethoxy-benzoyl)-benzoyloxy]-3-(3,5-bis-methoxymethoxy-naphthalin-2-ylcarbonylamino)-azepan-1-carbonsäure-tert-butylester als farbloser Schaum aus (3R,4R)-4-Hydroxy-3-(3,5-bis-methoxymethoxy-naphthalin-2-ylcarbonylamino)-azepan-1-carbonsäure-tert-butylester (B7) und 4-(2-Fluoro-3-methoxy-6-methoxymethoxy-benzoyl)-benzoesäure (C3)

A 14 (3R,4R)-3-(3,5-Dimethoxy-4-methoxymethoxy-benzoylamino)-4-[4-(3-methoxymethoxy-naphthalin-2-ylcarbonyl)-benzoyloxy]-azepan-1-carbonsäure-tert-butylester als farbloser Schaum aus (3R,4R)-4-Hydroxy-3-(3,5-dimethoxy-4-methoxymethoxy-benzoylamino)-azepan-1-carbonsäure-tert butylester (B6) und 4-(3-Methoxymethoxy-naphthalin-2-ylcarbonyl)-benzoesäure (C4)

A 15 (3R,4R)-3-(3,5-Dimethoxy-benzoylamino)-4-[4-(5-methoxy-2-methoxymethoxy-benzoyl)-benzoyloxy]-azepan-1-carbonsäure-tert-butylester als gelber Schaum aus (3R,4R)-4-Hydroxy-3-(3,5-dimethoxy-benzoylamino)-azepan-1-carbonsäure-tert-butylester (B8) und 4-(5-methoxy-2-methoxymethoxy-benzoyl)-benzoesäure (C7)

MS: 693.6$(M+H)^+$, 637.5, 605.4
IR: 3426, 1718, 1667, 1595, 1495, 1277, 1158, 840$cm^{-1}$

A 16 (3R,4R)-4-[4-(2-Fluoro-3-methoxy-6-methoxymethoxy-benzoyl)-benzoyloxy]-3-(3-methoxy-2-methoxymethoxy-benzoylamino)-azepan-1-carbonsäure-tert-butylester als farbloser Schaum aus (3R,4R)-4-Hydroxy-3-(3-methoxy-2-methoxymethoxy-benzoylamino)-azepan-1-carbonsäure-tert-butylester (B9) und 4-(2-Fluoro-3-methoxy-6-methoxymethoxy-benzoyl)-benzoesäure (C3)

MS: m/e= 741.6 $(M+H)^+$
IR (KBr): 1722, 1688, 1580, 1492 $cm^{-1}$

A 17 (3R,4R)-4-[4-(2-Fluoro-3-methoxy-6-methoxymethoxy-benzoyl)-benzoyloxy]-3-(3-methoxymethoxy-naphthalin-2-ylcarbonylamino)-azepan-1-carbonsäure-tert-butylester als farbloser Schaum aus (3R,4R)-4-Hydroxy-3-(3-methoxymethoxy-naphthalin-2-ylcarbonylamino)-azepan-1-carbonsäure-tert-butylester((B 10) und 4-(2-Fluoro-3-methoxy-6-methoxymethoxy-benzoyl)-benzoesäure (C3)

A 18 (3R,4R)-3-(4-Methoxymethoxy-benzoylamino)-4-[4-(3-methoxymethoxy-5,6,7,8-tetrahydro-naphthalin-2-ylcarbonyl)-benzoyloxy]-azepan-1-carbonsäure-tert-butylester als farbloser Schaum aus (3R,4R)-4-Hydroxy-3-(4-methoxymethoxy-benzoylamino)-azepan-1-carbonsäure-tert-butylester (B2) und 4-(3-Methoxymethoxy-5,6,7,8-tetrahydro-naphthalin-2-ylcarbonyl)-benzoesäure (C8)

A 19 (3R,4R)-4-[4-(2-Fluoro-3-methoxy-6-methoxymethoxy-benzoyl)-benzoyloxy]-3-(3,5-dimethoxy-benzoylamino)-azepan-1-carbonsäure-tert-butylester als farbloser Schaum aus (3R,4R)-4-hydroxy-3-(3,5-dimethoxy-benzoylamino)-azepan-1-carbonsäure-tert-butylester (B8) und 4-(2-Fluoro-3-methoxy-6-methoxymethoxy-benzoyl)-benzoesäure ((C3)

MS:709.6(M-H)$^-$
IR: 3414, 1721, 1676, 1594, 1528, 1491, 1272, 1157, 1039cm$^{-1}$

A 20 (3R,4R)-4-[4-(2-Fluoro-3-methoxy-6-methoxymethoxy-benzoyl)-benzoyloxy]-3-(4-methoxymethoxy-2,3,6-trimethyl-benzoylamino)-azepan-1-carbonsäure-tert-butylester als farbloser Schaum aus (3R,4R)-4-Hydroxy-3-(4-methoxymethoxy-2,3,6-trimethyl-benzoyl-amino)-azepan-1-carbonsäure-tert-butylester(B11) und 4-(2-Fluoro-3-methoxy-6-methoxymethoxy-benzoyl)-benzoesäure (C3)

MS: m/e= 753.5 (M+H)$^+$
IR (KBr): 1721, 1686, 1587, 1491 cm$^{-1}$

A 21 (3R,4R)-4-[4-(2,3-Difluoro-6-methoxymethoxy-benzoyl)-benzoyloxy]-3-(4-methoxymethoxy-2,3,6-trimethyl-benzoylamino)-azepan-1-carbonsäure-tert-butylester als farbloser Schaum aus (3R,4R)-4-Hydroxy-3-(4-methoxymethoxy-2,3,6-trimethyl-benzoylamino)-azepan-1-carbonsäure-tert-butylester (B11) und 4-(2,3-Difluoro-6-methoxymethoxy-benzoyl)-benzoesäure (C5)

MS: m/e= 741.5 (M+H)$^+$
IR (KBr): 1722, 1688, 1580, 1492 cm$^{-1}$

A 22 (3R,4R)-4-[4-(2,3-Difluoro-6-methoxymethoxy-benzoyl)-benzoyloxy]-3-(3,5-diisopropyl-4-methoxymethoxy-benzoylamino)-azepan-1-carbonsäure-tert-butylester als farbloser Schaum aus (3R,4R)-4-Hydroxy-3-(3,5-diisopropyl-4-methoxymethoxy-benzoyl-amino)-azepan-1-carbonsäure-tert-butylester (B12) und 4-(2,3-Difluoro-6-methoxymethoxy-benzoyl)-benzoesäure (C5)

MS: m/e= 783.6 (M+H)$^+$
IR (KBr): 1722, 1680, 1602, 1492 cm$^{-1}$

A 23 (3R,4R)-4-[4-(5-Methoxy-2-methoxymethoxy-benzoyl)-benzoyloxy]-3-(3,4,5-trimethoxy-benzoylamino)-azepan-1-carbonsäure-tert-butylester als gelber Schaum aus (3R,4R)-4-Hydroxy-3-(3,4,5-trimethoxy-benzoylamino)-azepan-1-carbonsäure-tert-butylester (B13) und 4-(5-Methoxy-2-methoxymethoxy-benzoyl)-benzoesäure (C7)

MS: 723.6(M+H)$^+$, 667.5
IR: 3422, 1720, 1696, 1666, 1586, 1497, 1278, 1235, 1127, 990, 841cm$^{-1}$

A 24 (3R,4R)-4-[4-(5-Dimethylamino-2-methoxymethoxy-benzoyl)-benzoyloxy]-3-(4-methoxymethoxy-benzoyl-amino)-azepan-1-carbonsäure-tert-butylester als oranger Schaum aus (3R,4R)-4-Hydroxy-3-(4-methoxymethoxy-benzoylamino)-azepan-1-carbonsäure-tert-butylester (B2) und 4-(5-Dimethylamino-2-methoxymethoxy-benzoyl)-benzoesäure (C9)

MS: m/e= 706.6 (M+H)$^+$
IR (KBr): 1719, 1665, 1606, 1502 cm$^{-1}$

A 25 (3R,4R)-3-(3-Methoxy-4-methoxymethoxy-benzoylainino)-4-[4[-(2-methoxymethoxy-5-dimethylamino-benzoyl)-benzoyloxy]-azepan-1-carbonsäure-tert-butylester als oranger Schaum aus (3R,4R)-4-Hydroxy-3-(3-methoxy-4-methoxymethoxy-benzoylamino)-azepan-1-carbonsäure-tert-butylester (B3) und 4-(5-Dimethylamino-2-methoxymethoxy-benzoyl)-benzoesäure (C9)

MS: m/e= 736.6 (M+H)$^+$
IR (KBr): 1718, 1665, 1605, 1504 cm$^{-1}$

A 26 (3R,4R)-3-(3,5-Diisopropyl-4-methoxymethoxy-benzoylamino)-4-[4-(2-methoxymethoxy-5-dimethylamino-benzoyl)-benzoyloxy]-azepan-1-carbonsäure-tert-butylester als oranger Schaum aus (3R,4R)-4-Hydroxy-3-(3,5-diisopropyl-4-methoxymethoxy-benzoyl-amino)-azepan-1-carbonsäure-tert-butylester (B12) und 4-(2-Methoxymethoxy-5-dimethylamino-benzoyl)-benzoesäure (C9)

MS: m/e= 790.7 (M+H)$^+$;
IR (KBr): 1719, 1666, 1605, 1503 cm$^{-1}$

A 27 (3R,4R)-4-[4-(2,3-Dihydro-1,4-benzodioxin-6-ylcarbonyl)-benzoyloxy]-3-(4-methoxymethoxy-benzoylamino)-azepan-1-carbonsäure-tert-butylester als farbloser Schaum aus (3R,4R)-4-Hydroxy-3-(4-methoxymethoxy-benzoylamino)-azepan-1-carbonsäure-tert-butylester (B2) und 4-(7-Methoxymethoxy-2,3-dihydro-1,4-benzodioxin-6-ylcarbonyl)benzoesäure (C10)

A 28 (3R,4R)-4-[4-[5-(1-Methoxyethyl)-2-methoxymethoxy-benzoyl]-benzoyloxy-3-(4-methoxymethoxy-benzoylamino)-azepan-1-carbonsäure-tert-butylester als farbloser Schaum aus (3R,4R)-4-Hydroxy-3-(4-methoxymethoxy-benzoylamino)-azepan-1-carbonsäure-tert-butylester (B2) und (RS)-4-[5-(1-Methoxyethyl)-2-methoxymethoxy-benzoyl]-benzoesäure (C 11)

A 29 (3R,4R)-4-[4-(2-Fluoro-3-methoxy-6-methoxymethoxy-benzoyl)-benzoyloxy]-3-(4-methoxymethoxy-3,5-dimethyl-benzoylamino)-azepan-1-carbonsäure-tert-butylester als farbloser Schaum aus (3R,4R)-4-hydroxy-3-(4-methoxymethoxy-3,5-dimethyl-benzoylamino)-azepan-1-carbonsäure-tert-butylester (B14) und 4-(2-Fluoro-3-methoxy-6-methoxymethoxy-benzoyl)-benzoesäure (C3)

MS: m/e= 739.7 $(M+H)^+$;
IR (KBr): 1721, 1677, 1503 $cm^{-1}$

A 30 (3R,4R)-4-[4-(2-Methoxymethoxy-5-dimethylamino-benzoyl)-benzoyloxy]-3-(4-methoxymethoxy-3,5-dimethyl-benzoylamino)-azepan-1-carbonsäure-tert-butylester als oranger Schaum aus (3R,4R)-4-Hydroxy-3-(4-methoxymethoxy-3,5-dimethyl-benzoylamino)-azepan-1-carbonsäure-tert-butylester (B14) und 4-(2-Methoxymethoxy-5-dimethylamino-benzoyl)benzoesäure (C9)

MS: m/e= 734.9 $(M+H)^+$
IR (KBr): 1719, 1666, 1606, 1503 $cm^{-1}$

A 31 (3R,4R)-4-[4-(2-Fluoro-4-methoxy-6-methoxymethoxy-benzoyl)-benzoyloxy]-3-(3,5-diisopropyl-4-methoxymethoxy-benzoylamino)-azepan-1-carbonsäure-tert-butylester als farbloser Schaum aus (3R,4R)-4-Hydroxy-3-(3,5-diisopropyl-4-methoxymethoxy-benzoyl-amino)-azepan-1-carbonsäure-tert-butylester (B12) und 4-(2-Fluoro-4-methoxy-6-methoxymethoxy-benzoyl)-benzoesäure (C1)

MS: m/e= 795.6 $(M+H)^+$
IR (KBr): 1720, 1669, 1622, 1581, 1528 $cm^{-1}$

A 32 (3R,4R)-3-(3-Acetyl-4-methoxymethoxy-benzoylamino)-4-[4-(5-methoxy-2-methoxymethoxy-benzoyl)-benzoyloxy]-azepan-1-carbonsäure-tert-butylester als gelber Schaum aus (3R,4R)-3-(3-Acetyl-4-methoxymethoxy-benzoylamino)-4-hydroxy-azepan-1-carbonsäure-tert-butylester (B15) und 4-(5-Methoxy-2-methoxymethoxy-benzoyl)-benzoesäure (C7)

MS: 735.6$(M+H)^+$, 679.5
IR: 3450, 1719, 1671, 1603, 1491, 1277, 1156, 981$cm^{-1}$

A 33 (3R,4R)-3-(4-Methoxymethoxy-benzoylamino)-4-[4-(6-methoxymethoxy-chinolin-7-ylcarbonyl)-benzoyloxy]-azepan-1-carbonsäure-tert-butylester als gelber Schaum aus (3R,4R)-4-Hydroxy-3-(4-methoxymethoxy-benzoylamino)-azepan-1-carbonsäure-tert-butylester (B2) und 4-(6-Methoxymethoxy-chinolin-7-ylcarbonyl)-benzoesäure (C12)

A 34 (3R,4R)-4-[4-(2,3-Difluoro-6-methoxymethoxy-benzoyl)-benzoyloxy]-3-(4-methoxymethoxy-3,5-dimethyl-benzoylamino)-azepan-1-carbonsäure-tert-butylester als farbloser Schaum aus (3R,4R)-4-Hydroxy-3-(4-methoxymethoxy-3,5-dimethyl-benzoylamino)-azepan-1-carbonsäure-tert-butylester (B14) und 4-(2,3-Difluoro-6-methoxymethoxy-benzoyl)-benzoesäure (C5)

MS: m/e= 727.5 $(M+H)^+$
IR (KBr): 1721, 1683, 1603, 1491 $cm^{-1}$

A 35 (3R,4R)-4-[4-(2,4-Difluoro-6-methoxymethoxy-benzoyl)-benzoyloxy]-3-(4-methoxymethoxy-3,5-dimethyl-benzoylamino)-azepan-1-carbonsäure-tert-butylester als farbloser Schaum aus (3R,4R)-4-Hydroxy-3-(4-methoxymethoxy-3,5-dimethyl-benzoylamino)-azepan-1-carbonsäure-tert-butylester (B14) und 4-(2,4-Difluoro-6-methoxymethoxy-benzoyl)-benzoesäure (C6)

MS: m/e= 727.5 $(M+H)^+$
IR (KBr): 1721, 1675, 1620, 1603, 1482 $cm^{-1}$

A 36 (3R,4R)-3-(3,5-Diethoxy-benzoylamino)-4-[4-(5-methoxy-2-methoxymethoxy-benzoyl)benzoyloxy]-azepan-1-carbonsäure-tert-butylester als gelber Schaum aus (3R,4R)-3-(3,5-diethoxy-benzoylamino)-4-hydroxy-azepan-1-carbonsäure-tert-butylester (B16) und 4-(5-methoxy-2-methoxymethoxy-benzoyl)-benzoesäure (C7)

MS: 721.5$(M+H)^+$, 665.5, 585.4, 409.3
IR: 3429, 1719, 1667, 1593, 1495, 1276, 1172, 990$cm^{-1}$

A 37 (3R,4R)-3-(4-Methoxymethoxy-benzoylamino)-4-[4-(4-methoxymethoxy-biphenyl-3-ylcarbonyl)-benzoyloxy]-azepan-1-carbonsäure-tert-butylester als gelber Schaum aus (3R,4R)-4-Hydroxy-3-(4-methoxymethoxy-benzoylamino)-azepan-1-carbonsäure-tert-butylester (B2) und 4-(4-Methoxymethoxy-biphenyl-3-ylcarbonyl)-benzoesäure (C 13)

A 38 (3R,4R)-3-(4-Methoxymethoxy-3,5-dimethyl-benzoylamino)-4-[4-(6-methoxymethoxy-chinolin-7-ylcarbonyl)-benzoyloxy]-azepan-1-carbonsäure-tert-butylester als gelber Schaum aus (3R,4R)-4-Hydroxy-3-(4-methoxymethoxy-3,5-dimethyl-benzoylamino)-azepan-1-carbonsäure-tert-butylester (B14) und 4-(6-methoxymethoxy-chinolin-7-ylcarbonyl)-benzoesäure (C12)

A 39 (3R,4R)-4-[4-(5-Acetyl-2-methoxymethoxy-benzoyl)-benzoyloxy]-3-(4-methoxymethoxy-benzoylamino)-azepan-1-carbonsäure-tert-butylester als gelber Schaum aus (3R,4R)-4-Hydroxy-3-(4-methoxymethoxy-benzoylamino)-azepan-1-carbonsäure-tert-butylester (B2) und 4-(5-Acetyl-2-methoxymethoxy-benzoyl)-benzoesäure (C 14)

A 40 (3R, 4R)-3-(4-Methoxymethoxy)-benzoylamino)-4-[3-methoxymethoxy-10-methyl-phenothiazin-2-ylcarbonyl)-benzoyl]-azepan-1-carbonsäure-tert-butylester als gelber Schaum aus (3R,4R)-4-Hydroxy-3-(4-methoxymethoxy-benzoylamino)-azepan-1-carbonsäure-tert-butylester (B2) und 4-(3-Methoxymethoxy-10-methyl-phenothiazin-2-ylcarbonyl)-benzoesäure (C15)

MS: 798.6 $(M+H)^+$
IR: 2840, 1718, 1693, 1603, 1536, 1501, 1265, 1153, 847, 752.

A 41 (3R,4R)-3-(3,5-Diethyl-4-methoxymethoxy-benzoylamino)-4-[2-fluoro-3-methoxy-6-methoxymethoxy-benzoyl)-benzoyloxy]-azepan-1-carbonsäure-tert-butylester als farbloser Schaum aus (3R,4R)-3-(3,5-Diethyl-4-methoxymethoxy-benzoylamino)-4-hydroxy-azepan-1-carbonsäure-tert-butylester (B17) und 4-(2-Fluoro-3-methoxy-6-methoxymethoxy-benzoyl)-benzoesäure (C3)

MS: m/e= 767.6 $(M+H)^+$
IR (KBr): 1721, 1672, 1585, 1491 $cm^{-1}$

A 42 (3R,4R)-3-(3,5-Diethyl-4-methoxymethoxy-benzoylamino)-4-[4-(2,3-difluoro-6-methoxymethoxy-benzoyl)-benzoyloxy]-azepan-1-carbonsäure-tert-butylester als farbloser Schaum aus (3R,4R)-3-(3,5-Diethyl-4-methoxymethoxy-benzoylamino)-4-hydroxy-azepan-1-carbonsäure-tert-butylester (B17) und 4-(2,3-Difluoro-6-methoxymethoxy-benzoyl)-benzoesäure (C5)

MS: m/e= 755.6 $(M+H)^+$
IR (KBr): 1721, 1684, 1603, 1492 $cm^{-1}$

A 43 (3R,4R)-3-(3,5-Diethyl-4-methoxymethoxy-benzoylamino)-4-[4-(2-methoxymethoxy-5-dimethylamino-benzoyl)-benzoyloxy]-azepan-1-carbonsäure-tert-butylester als oranger Schaum aus (3R,4R)-3-(3,5-Diethyl-4-methoxymethoxy-benzoylamino)-4-hydroxy-azepan-1-carbonsäure-tert-butylester (B17) und 4-(2-Methoxymethoxy-5-dimethylamino-benzoyl)-benzoesäure (C9)

MS: m/e= 762.7 $(M+H)^+$
IR (KBr): 1719, 1666, 1606, 1503 $cm^{-1}$

A 44 (3R,4R)-3-(3,5-Diethyl-4-methoxymethoxy-benzoylamino)-4-[4-(2,4-difluoro-6-methoxymethoxy-benzoyl)-benzoyloxy-azepan-1-carbonsäure-tert-butylester als farbloser Schaum aus (3R,4R)-3-(3,5-Diethyl-4-methoxymethoxy-benzoylamino)-4-hydroxy-azepan-1-carbonsäure-tert-butylester (B17) und 4-(2,4-Difluoro-6-methoxymethoxy-benzoyl)-benzoesäure [(C6)

MS: m/e= 755.6 $(M+H)^+$
IR (KBr): 1720, 1674, 1619, 1464 $cm^{-1}$

A 45 (3R,4R)-4-[4-(5-Methoxy-2-methoxymethoxy-benzoyl)-benzoyloxy]-3-(4-methoxymethoxy-3,5-dimethyl-benzoylamino)-azepan-1-carbonsäure-tert-butylester aus (3R,4R)-4-Hydroxy-3-(4-methoxymethoxy-3,5-dimethyl-benzoylamino)-azepan-1-carbonsäure-tert-butylester (B 14) und 4-(5-Methoxy-2-methoxymethoxy-benzoyl)-benzoesäure (C7)

A 46 (3R,4R)-4-[4-(5-Methoxy-2-methoxymethoxy-benzoyl)-benzoyloxy]-3(4-pyridinoylamino)-azepan-1-carbonsäure-tert-butylester als gelber Schaum aus (3R,4R)-4-Hydroxy-3-(4-pyridinoylamino)-azepan-1-carbonsäure-tert-butylester (B 22) und 4-(5-Methoxy-2-methoxymethoxy-benzoyl)-benzoesäure (C7)

MS: 634.4$(M+H)^+$, 578.4;
IR: 3390, 1720, 1671, 1532, 1493, 1276, 1159, 991$cm^{-1}$

A 47 (3R, 4R)-3-(4-Methoxymethoxy-benzoylamino)-4-[4-(2-methoxymethoxy-5-methyl-benzoyl)-benzoyloxy]-azepan-1-carbonsäure-tert-butylester als weisser Feststoff aus (3R,4R)-4-Hydroxy-3-(4-methoxymethoxy-benzoylamino)-azepan-1-carbonsäure-tert-butylester (B2) und 4-(2-Methoxymethoxy-5-methyl-benzoyl)-benzoesäure (C16).

MP = 66.2 $^oC$
MS : 677.3 $(M+H)^+$, 621.2, 577.3
IR: 3430, 2788, 1719, 1690, 1665, 1606, 1500, 1279, 994.

A 48 (3R,4R)-4-[4-(6-Methoxymethoxy-1,3-benzodioxol-5-ylcarbonyl)-benzoyloxy]-3-(4-methoxymethoxy-3,5-dimethyl-benzoylamino)-azepan-1-carbonsäure-tert-butylester als farbloser Schaum aus (3R,4R)-4-Hydroxy-3-(4-methoxymethoxy-3,5-dimethyl-benzoylamino)-azepan-1-carbonsäure-tert-butylester (B14) und 4-(6-Methoxymethoxy-1,3-benzodioxol-5-ylcarbonyl)-benzoesäure (C17)

A 49 (3R, 4R)-4-[4-(5-Isopropyl-2-methoxymethoxy-benzoyl)-benzoyloxy]-3-(4-methoxymethoxy-benzoylamino)-azepan-1-carbonsäure-tert-butylester als farbloser Schaum aus (3R,4R)-4-Hydroxy-3-(4-methoxymethoxy-benzoylamino)-azepan-1-carbonsäure-tert-butylester (B2) und 4-(5-Isopropyl-2-methoxymethoxy-benzoyl)-benzoesäure (C18)

MS : 705.5 $(M+H)^+$, 649.5
IR: 3405, 1719, 1666, 1535, 1499, 1278, 1239, 1154, 994,840
Anal. calc. for $C_{39}H_{48}N_2O_{10}$ (704.817); C 66.46, H 6.86, N 3.97; found: C 66.27, H 6.97, N 3.74.

A 50 (3R,4R)-4-[4-(6-Methoxymethoxy-(1,4-benzodioxin-5-ylcarbonyl)-benzoyloxy]-3-(4-methoxymethoxy-3,5-dimethyl-benzoylamino) azepan-1-carbonsäure-tert-butylester als gelber Feststoff aus (3R,4R)-4-hydroxy-3-(4-methoxymethoxy-3,5-dimethyl-benzoylamino)-azepan-1-carbonsäure-tert-butylester (B14) und 4-(6-Methoxymethoxy-1,4-benzodioxin-5-ylcarbonyl)-benzoesäure (C19)

MS : 749.5 $(M+H)^+$, 693.4
IR : 3428, 7120, 1672, 1602, 1529, 1268, 1157, 1098, 1021.

A 51 (3R,4R)-4-[4-(2-Fluoro-3-dimethylamino-6-methoxymethoxy-benzoyl)-benzoyloxy]-3-(4-methoxymethoxy-benzoylamino)-azepan-1-carbonsäure-tert-butylester als gelber Schaum aus (3R,4R)-4-Hydroxy-3-(4-methoxymethoxy-benzoylamino)-azepan-1-carbonsäure-tert-butylester (B2) und 4-(2-Fluoro-3-dimethylamino-6-methoxymethoxy-benzoyl)-benzoesäure (C20)

MS: m/e= 724.5 $(M+H)^+$
IR (KBr): 1720, 1680, 1606, 1498 $cm^{-1}$

A 52 (3R,4R)-4-[4-(2-Fluoro-3-dimethylamino-6-methoxymethoxy-benzoyl)-benzoyloxy]-3-(4-methoxymethoxy-3,5-dimethyl-benzoylamino)-azepan-1-carbonsäure-tert-butylester als gelber Schaum aus (3R,4R)-4-Hydroxy-3-(4-methoxymethoxy-3,5-dimethyl-benzoylamino)-azepan-1-carbonsäure-tert-butylester (B14) und 4-(2-Fluoro-3-dimethylamino-6-methoxymethoxy-benzoyl)-benzoesäure (C20)

MS: m/e= 752.5 $(M+H)^+$
IR (KBr): 1721, 1672, 1608, 1490 $cm^{-1}$

A 53 (3R,4R)-4-[4-(2-Fluoro-3-dimethylamino-6-methoxymethoxy-benzoyl)-benzoyloxy]-3-(3,5-dimethoxy-4-methoxymethoxy-benzoylamino)-azepan-1-carbonsäure-tert-butylester als gelber Schaum aus (3R,4R)-4-Hydroxy-3-(3,5-dimethoxy-4-methoxymethoxy-benzoylamino)-azepan-1-carbonsäure-tert butylester (B6) und 4-(2-Fluoro-3-dimethylamino-6-methoxymethoxy-benzoyl)-benzoesäure (C20)

MS: m/e= 784.5 $(M+H)^+$
IR (KBr): 1721, 1673, 1587, 1495 $cm^{-1}$

A 54 (3R,4R)-4-[4-(5-acetyl-2-methoxymethoxy-benzoyl)-benzoyloxy]-3-(4-methoxymethoxy-3,5-dimethyl-benzoylamino)-azepan-1-carbonsäure-tert-butylester als gelber Schaum aus (3R,4R)-4-Hydroxy-3-(4-methoxymethoxy-3,5-dimethyl-benzoylamino)-azepan-1-carbonsäure-tert-butylester (B14) und 4-(5-Acetyl-2-methoxymethoxy-benzoyl)-benzoesäure (C 14)

A 55 (3R,4R)-4-[4-(2-Fluoro-3-methoxy-6-methoxymethoxy-benzoyl)-benzoyloxy]-3-(4-pyridinoylamino)-azepan-1-carbonsäure-tert-butylester als farbloser Schaum aus (3R,4R)-4-Hydroxy-3-(4-pyridinoylamino)-azepan-1-carbonsäure-tert-butylester (B22) und 4-(2-Fluoro-3-methoxy-6-methoxymethoxy-benzoyl)-benzoesäure (C3)

MS:652.5$(M+H)^+$, 596.3
IR: 3340, 1729, 1679, 1532, 1491, 1282, 1157, 821, 749$cm^{-1}$

A 56 (3R,4R)-3-(3-tert-Butyl-4-hydroxy-benzoylamino)-4-[4-(5-methoxy-2-methoxymethoxy-benzoyl)-benzoyloxy]-azepan-1-carbonsäure-tert-butylester als weisser Feststoff aus (3R,4R)-3-(3-tert-Butyl-4-hydroxy-benzoylamino)-4-hydroxy-azepan-1-carbonsäure-tert-butylester (B18) und 4-(5-Methoxy-2-methoxymethoxy-benzoyl)-benzoesäure (C7)

MS:705.5$(M+H)^+$, 649.5
IR: 3426, 1742, 1665, 1537, 1489, 1259, 1193, 1158, 988$cm^{-1}$

A 57 (3R,4R)-3-(3-Bromo-4-methoxymethoxy-benzoylamino)-4-[4-(2-fluoro-3-methoxy-6-methoxymethoxy-benzoyl)-benzoyloxy]-azepan-1-carbonsäure-tert-butylester als gelber Schaum aus (3R,4R)-3-(3-Bromo-4-methoxymethoxy-benzoylamino)-4-hydroxy-azepan-1-carbonsäure-tert-butylester (B19) und 4-(2-Fluoro-3-methoxy-6-methoxyrnethoxy-benzoyl)-benzoesäure (C3)

A 58 (3R,4R)-3-(3-Isopropyl-4-methoxymethoxy-benzoylamino)-4-[4-(5-methoxy-2-methoxymethoxy-benzoyl)-benzoyloxy]-azepan-1-carbonsäure-tert-butylester als gelber Schaum aus (3R,4R)-4-Hydroxy-3-(3-isopropyl-4-methoxymethoxy-benzoylamino)-azepan-1-carbonsäure-tert-butylester (B20) und 4-(5-Methoxy-2-methoxymethoxy-benzoyl)-benzoesäure (C7)

MS: 735.5$(M+H)^+$, 679.5
IR: 3432, 1719, 1666, 1532, 1493, 1277, 1151, 994$cm^{-1}$

A 59 (3R,4R)-3-(3-sec-Butyl-4-methoxymethoxy-benzoylamino)-4-[4-(5-methoxy-2-methoxymethoxy-benzoyl)-benzoyloxy]-azepan-1-carbonsäure-tert-butylester als gelber Schaum aus (3R,4R)-3-(3-sec-Butyl-4-methoxymethoxy-benzoylamino)-4-hydroxy-azepan-1-carbonsäure-tert-butylester (B21) und 4-(5-Methoxy-2-methoxymethoxy-benzoyl)-benzoesäure (C7)

MS: 749.5$(M+H)^+$, 693.4
IR: 3443, 1720, 1666, 1532, 1493, 1277, 1150, 995$cm^{-1}$

A 60 (3R,4R)-4-[4-(2-Fluoro-3-isopropyl-6-methoxymethoxy-benzoyl)-benzoyloxy]-3-(4-methoxymethoxy-3,5-dimethyl-benzoylamino)-azepan-1- carbonsäure-tert-butylester als gelber Schaum aus (3R,4R)-4-Hydroxy-3-(4-methoxymethoxy-3,5-dimethyl-benzoylamino)-azepan-1-carbonsäure-tert-butylester (B14) und 4-(2-Fluoro-3-isopropyl-6-methoxymethoxy-benzoyl)-benzoesäure (C2)

A 61 (3R,4R)-4-[4(2-Fluoro-6-methoxymethoxy-3-pyrrolidin-1-yl-benzoyl)-benzoyloxy]-3-(4-methoxymethoxy-benzoylamino)-azepan-1-carbonsäure-tert-butylester als oranger Schaum aus (3R,4R)-4-Hydroxy-3-(4-methoxymethoxy-benzoylamino)-azepan-1-carbonsäure-tert-butylester (B2) und 4-(2-Fluoro-6-methoxymethoxy-pyrrolidin-1-yl-benzoyl)-benzoesäure (C22)

MS: m/e= 750.7 $(M+H)^+$
IR (KBr): 1721, 1683, 1574, 1499 $cm^{-1}$

A 62 (3R,4R)-4-[4-(2-Fluoro-6-methoxymethoxy-3-pyrrolidin-1-yl-benzoyl)-benzoyloxy]-3-(4-methoxymethoxy-3,5-dimethyl-benzoylamino)-azepan-1-carbonsäure-tert-butylester als oranger Schaum aus (3R,4R)-4-Hydroxy-3-(4-methoxymethoxy-3,5-dimethyl-benzoylamino)-azepan-1-carbonsäure-tert-butylester (B14) und 4-(2-Fluoro-6-methoxymethoxy-pyrtolidin-1-yl-benzoyl)-benzoesäure (C22)

MS: m/e= 778.6 $(M+H)^+$
IR (KBr): 1721, 1675, 1603, 1486 $cm^{-1}$

A 63 (3R, 4R)-3-(4-Methoxymethoxy)-benzoylamino)-4-[4-(2-fluoro-6-methoxymethoxy-3-methyl-benzoyl)-benzoyloxy]-azepan-1-carbonsäure-tert-butylester als farbloses Oel aus (3R,4R)-4-Hydroxy-3-(4-methoxymethoxy-benzoylamino)-azepan-1-carbonsäure-tert-butylester (B2) und 4-(2-Fluoro-6-methoxymethoxy-3-methyl-benzoyl)-benzoesäure (C23)

MS: 695.5 $(M+H)^+$, 639.5
IR : 3380, 2934, 1719, 1671, 1613, 1605, 1535, 1500, 1241, 1153, 993, 895.
Anal. calc. for $C_{37}H_{43}N_2O_{10}F$ (694.753): C 63.97, 6.24, 4.03; found: C 63.69, 6.57, 3.73.

A 64 (3R, 4R)-4-[4-(3-Ethyl-2-fluoro-6-methoxymethoxy-benzoyl)-benzoyloxy]-3-(4-methoxymethoxy-3,5-dimethyl-benzoylamino) azepan-1-carbonsäure-tert-butylester als farbloses Oel aus (3R,4R)-4-Hydroxy-3-(4-methoxymethoxy-3,5-dimethyl-benzoylamino)-azepan-1-carbonsäure-tert-butylester (B14) und 4-(3-Ethyl-2-fluoro-6-methoxymethoxy-benzoyl)-benzoesäure (C24)

MS : 737.5 $(M+H)^+$ 681.5
IR: 3335, 1720, 1682, 1530, 1269, 1158, 1095, 1036.

A 65 (3R,4R)-4-[4-(2-Fluoro-6-methoxymethoxy-3-piperidin-1-yl-benzoyl)-benzoyloxy]-3-(4-methoxymethoxy-benzoylamino)-azepan-1-carbonsäure-tert-butylester als oranger Schaum aus (3R,4R)-4-Hydroxy-3-(4-methoxymethoxy-benzoylamino)-azepan-1-carbonsäure-tert-butylester (B14) und 4-(2-Fluoro-6-methoxymethoxy-piperidin-1-yl-benzoyl)-benzoesäure (C25)

MS: m/e = 792.6 $(M+H)^+$
IR (KBr): 1721, 1683, 1604, 1573, 1530, 1486, 1269, 1158, 1038, 971 $cm^{-1}$

B. Die in Beispiel A eingesetzten Alkohole wurden hergestellt durch

a) hydrogenolytische Spaltung von Alkoholschutzgruppen aus entsprechend geschützten Verbindungen; oder

b) Acylierung eines entsprechenden Amins mit einer Carbonsäure oder einem Acetonoximester

Die folgenden Verbindungen wurden erhalten:

B 1 (Methode a) (3R,4R)-3-[4-[Dimethyl(1,1,2-trimethylpropyl)siloxy]-benzoylamino)-4-hydroxy-azepan-1-carbonsäure-tert-butylester als farbloser Schaum aus (3R,4R)-4-Benzyloxy-3-[4-[dimethyl-(1,1,2-trimethylpropyl)-siloxy]benzoylamino]azepan-1-carbonsäure-tert-butylester (R2)

MS:493$(M+H)^+$,437, 393, 263
IR: 3421, 1692, 1667, 1604, 1501, 1264, 1171, 911$cm^{-1}$

B 2 (Methode a) (3R,4R)-4-Hydroxy-3-(4-methoxymethoxy-benzoyl-amino)-azepan-1-carbonsäure-tert-butylester als farbloser Schaum aus (3R,4R)-4-Benzyloxy-3-(4-methoxymethoxy-benzoyl-amino)-azepan-1-carbonsäure-tert-butylester (R1)

MS: m/e= 395 $(M+H)^+$
IR (Flm): 3341, 1688, 1665, 1636, 1606, 1541, 1155, 1079 $cm^{-1}$

B 3 (Methode a) (3R,4R)-4-Hydroxy-3-(3-methoxy-4-methoxymethoxy-benzoylamino)-azepan-1-carbonsäure-tert-butylester als farbloser Schaum aus (3R,4R)-4-Benzyloxy-3-(3-methoxy-4-methoxymethoxy-benzoylamino)-azepan-1-carbonsäure-tert-butylester (R3)

MS: m/e= 425.2 $(M+H)^+$
IR (KBr): 3369, 1690, 1664, 1638, 1605, 1544, 1162, 1077 $cm^{-1}$

B 4 (Methode a) (3R,4R)-4-hydroxy-3-(5-methoxy-2-methoxymethoxy-benzoylamino)-azepan-1-carbonsäure-tert-butylester als farbloser Schaum aus (3R,4R)-4-benzyloxy-3-(5-methoxy-2-methoxymethoxy-benzoylamino)-azepan-1-carbonsäure-tert-butylester (R4)

B 5 (Methode a) (3R,4R)-3-[4-(tert-butyldimethylsilanyloxy)-benzoylamino]-4-hydroxy-azepan-1-carbonsäure-tert-butylester als farbloser Schaum aus (3R,4R)-4-benzyloxy-3-[4-(tert-butyldimethylsilanyloxy)-benzoylamino]-azepan-1-carbonsäure-tert-butylester (R5)

B 6 (Methode a) (3R,4R)-4-Hydroxy-3-(3,5-dimethoxy-4-methoxymethoxy-benzoylamino)-azepan-1-carbonsäure-tert butylester als farbloser Schaum aus (3R,4R)-4-Benzyloxy-3-(3,5-dimethoxy-4-methoxymethoxy-benzoylamino)-azepan-1-carbonsäure-tert-butylester (R6)

MS: m/e= 455 $(M+H)^+$
IR (KBr): 3378, 1693, 1663, 1640, 1587, 1543, 1162, 1079 $cm^{-1}$

B 7 (Methode a) (3R,4R)-4-hydroxy-3-(3,5-bis-methoxymethoxy-naphthalen-2-ylcarbonylamino)-azepan-1-carbonsäure-tert-butylester als farbloser Schaum aus (3R,4R)-4-benzyloxy-3-(3,5-bis-methoxymethoxy-naphthalen-2-ylcarbonylamino)-azepan-1-carbonsäure-tert-butylester (R7)

B 8 (Methode a) (3R,4R)-4-hydroxy-3-(3,5-dimethoxy-benzoylaniino)-azepan-1-carbonsäure-tert-butylester als farbloser Schaum aus (3R,4R)-4-benzyloxy-3-(3,5-dimethyl-benzoylamino)azepan-1-carbonsäure-tert-butylester (R8)

MS:395$(M+H)^+$
IR: 3335, 1664, 1594, 1536, 1156, 1064$cm^{-1}$

B 9 (Methode a) (3R,4R)-4-Hydroxy-3-(3-methoxy-2-methoxymethoxy-benzoylamino)-azepan-1-carbonsäure-tert-butylester als farbloser Schaum aus (3R,4R)-4-Benzyloxy-3-(3-methoxy-2-methoxymethoxy-benzoyl-amino)-azepan-1-carbonsäure-tert-butylester (R9)

MS: m/e= 368 $(M-C_4H_8)^{+\bullet}$
IR (Flm): 3376, 1687, 1660, 1578, 1527, 1163, 1077 $cm^{-1}$

B 10 (Methode a) (3R,4R)-4-Hydroxy-3-(3-methoxymethoxy-naphthalen-2-ylcarbonylamino)-azepan-1-carbonsäure-tert-butylester als farbloser Schaum aus (3R,4R)-4-Benzyloxy-3-(3-methoxymethoxy-naphthalen-2-ylcarbonylamino)-azepan-1-carbonsäure-tert-butylester (R10)

IR: 3335, 1664, 1594, 1536, 1156, 1064$cm^{-1}$

B 11 (Methode a) (3R,4R)-4-Hydroxy-3-(4-methoxymethoxy-2,3,6-trimethyl-benzoyl-amino)-azepan-1-carbonsäure-tert-butylester als farbloser Schaum aus (3R,4R)-4-Benzyloxy-3-(4-methoxymethoxy-2,3,6-trimethyl-benzoylamino)-azepan-1-carbonsäure-tert-butylester (R11)

MS: m/e= 437.6 $(M+H)^+$
IR (KBr): 3402, 1692, 1673, 1638, 1597, 1533, 1160, 1054 $cm^{-1}$

B 12 (Methode a) (3R,4R)-4-Hydroxy-3-(3,5-diisopropyl-4-methoxymethoxy-benzoyl-amino)-azepan-1-carbonsäure-tert-butylester [Ro 47-6143] als farbloser Schaum aus (3R,4R)-4-Benzyloxy-3-(3,5-diisopropyl-4-methoxymethoxy-benzoyl-amino)-azepan-1-carbonsäure-tert-butylester (R12)

MS: m/e= 479.6 $(M+H)^+$
IR (KBr): 3426, 1695, 1665, 1640, 1603, 1537, 1162, 1071 $cm^{-1}$

B 13 (Methode a) (3R,4R)-4-Hydroxy-3-(3,4,5-trimethoxy-benzoylamino)-azepan-1-carbonsäure-tert-butylester als farbloses Pulver aus (3R,4R)-4-benzyloxy-3-(3,4,5-trimethoxy-benzoylamino)azepan-1-carbonsäure-tert-butylester (R13)

MS:447.6$(M+H)^+$, 425.6, 369.5, 325.4
IR: 3381, 1664, 1583, 1546, 1237, 1172, 1126, 1010$cm^{-1}$

B 14 (Methode a) (3R,4R)-4-Hydroxy-3-(4-methoxymethoxy-3,5-dimethyl-benzoylamino)-azepan-1-carbonsäure-tert-butylester als farbloser Schaum aus (3R,4R)-Benzyloxy-3-(4-methoxymethoxy-3,5-dimethyl-benzoylamino)-azepan-1-carbonsäure-tert-butylester (R14)

MS: m/e= 423.4 $(M+H)^+$
IR (Flm): 3339, 1695, 1665, 1640, 1602, 1536, 1162, 1073 $cm^{-1}$

B 15 (Methode a) (3R,4R)-3-(3-Acetyl-4-methoxymethoxy-benzoylamino)4-hydroxy-azepan-1-carbonsäure-tert-butylester als farbloser Schaum aus (3R,4R)-3-(3-Acetyl-4-methoxymethoxy-benzoylamino)4-benzyloxy-azepan-1-carbonsäure-tert-butylester (R15)

MS:437$(M+H)^+$, 381, 305
IR: 3340, 1675, 1602, 1540, 1488, 1232, 1165, 977$cm^{-1}$

B 16 (Methode a) (3R,4R)-3-(3,5-Diethoxy-benzoylamino)-4-hydroxy-azepan-1-carbonsäure-tert-butylester als farbloser Schaum aus (3R,4R)-4-benzyloxy-3-(3,5-diethoxy-benzoylamino)azepan-1-carbonsäure-tert-butylester (R16)

MS: 423.5$(M+H)^+$, 367.4, 323.4
IR: 3329, 1664, 1593, 1536, 1172, 1058$cm^{-1}$

B 17 (Methode a) (3R,4R)-3-(3,5-Diethyl-4-methoxymethoxy-benzoylamino)-4-hydroxy-azepan-1-carbonsäure-tert-butylester als farbloser Schaum aus (3R,4R)-Benzyloxy-3-(3,5-diethyl-4-methoxymethoxy-benzoyl-amino)-azepan-1-carbonsäure-tert-butylester (R17)

MS: m/e= 451.4 $(M+H)^+$
IR (Flm): 3334, 1692, 1665, 1638, 1602, 1536, 1162, 1075 $cm^{-1}$

B18 (Methode b) (3R,4R)-4-Hydroxy-3-(4-pyridinoylamino)-azepan-1-carbonsäure-tert-butylester als gelbes Oel aus (3R,4R)-3-Amino-4-hydroxy-azepan-1-carbonsäure-tert-butylester

MS: 336.2$(M+H)^+$; IR: 3425, 1666, 1539, 1417, 1165, 847$cm^{-1}$

B 19 (Methode a) (3R,4R)-3-(3-tert-Butyl-4-hydroxy-benzoylamino)-4-hydroxy-azepan-1-carbonsäure-tert-butylester als farbloses Pulver aus (3R,4R)-4-benzyloxy-3-(3-tert-butyl-4-hydroxy-benzoylamino)azepan-1-carbonsäure-tert-butylester (R18)

MS: 405.3$(M-H)^-$
IR: 3294, 1668, 1599, 1545, 1419, 1269, 1168$cm^{-1}$

B 20 (Methode b) (3R,4R)-3-(3-Bromo-4-methoxymethoxy-benzoylamino)-4-hydroxy-azepan-1-carbonsäure-tert-butylester als farbloser Schaum aus 3-Bromo-4-methoxymethoxy-benzoesäure

B 21 (Methode a) (3R,4R)-4-Hydroxy-3-(3-isopropyl-4-methoxymethoxy-benzoylamino)-azepan-1-carbonsäure-tert-butylester als farbloser Schaum aus (3R,4R)-4-Benzyloxy-3-(3-isopropyl-4-methoxymethoxy-benzoylamino)-azepan-1-carbonsäure-tert-butylester (R19)

MS: 437.4$(M+H)^+$, 381.4, 337.3
IR: 3333, 1665, 1637, 1604, 1539, 1492, 1243, 1162, 977$cm^{-1}$

B 22 (Methode a) (3R,4R)-3-(3-sec-Butyl-4-methoxymethoxy-benzoylamino)-4-hydroxy-azepan-1-carbonsäure-tert-butylester als farbloser Schaum aus (3R,4R)-4-Benzyloxy-3-(3-sec-butyl-4-methoxymethoxy-benzoylamino)azepan-1-carbonsäure-tert-butylester (R20)

MS: 451.5$(M+H)^+$, 395.4, 351.3
IR: 3335, 1665, 1638, 1604, 1539, 1491, 1241, 1162, 1074, 998$cm^{-1}$

B 23 (Methode b) (3R,4R)-3-(2-Benzyloxy-5-fluoro-benzoylamino)-4-hydroxy-azepan-1-carbonsäure-tert-butylester als hellgelber Schaum aus 2-Benzyloxy-5-fluoro-benzoesäure-acetonoxim ester

MS: 459.1$(M+H)^+$, 403.1,359.2
IR: 3389, 1686, 1657, 1532, 1488, 1417, 1274, 1187, 1003, 812, 744, 698$cm^{-1}$

Das in B 18 eingesetzte Ausgangsmaterial wurde wie folgt hergestellt:

Ethyl 2,3-dideoxy-α-D-erythrohexopyranosid wurde mit p-Toluolsulfochlorid in Pyridin in 6-O-(p-Tolylsalfonyl)-2,3-dideoxy-α-D-erythrohexopyranosid übergeführt. Umsetzung mit Natriumazid in Dimethylformamid lieferte Ethyl 6-Azido-2,3,6-trideoxy-α-D-erythrohexopyranosid, aus dem mit 4-Nitrobenzoesäure unter Mitsunobu-Reaktionsbedingungen das 6-Azido-2,3,6-trideoxy-4-O-(4-nitrobenzoyl)-α-D-threohexopyranosid erhalten wurde. Hydrolyse mit methanolischer NaOH lieferte 6-Azido-2,3,6-trideoxy-α-D-galactopyranosid, aus dem durch Benzylierung und anschliessende saure Hydrolyse die 6-Azido-5-O-benzyl-2,3,6-trideoxy-D-galaktopyranose erhalten wurde. Hydrierung mit PtO und Umsetzung mit Bis-tert.-butylcarbonat lieferte tert.-Butyl (3R,4R)-4-(benzyloxy)-hexahydro-3-hydroxy-1H-azepin-1-carboxylat. Unter Mitsunobu-Bedingungen wurde daraus das tert.Butyl-(3S,4R)-4-benzyloxy)-hexahydro-3-O-(4-nitrobenzoyl)-1H-azepin-1-carboxylat und daraus durch basische Hydrolyse und Umsetzung mit Hydrazinsäure unter Mitsunobu-Bedingungen das tert.-Butyl-(3R,4R)-3-azido-4-(benzyloxy)-hexahydro-1H-azepin-1-carboxylat erhalten. Hydrierung dieser Verbindung über Pd/C bei Raumtemperatur und Atmosphärendruck lieferte (3R,4R)-3-Amino-4-benzyloxy-azepan-1-carbonsäure-tert.-butylester, weitere Hydrierung bei 80°C und 10 Bar lieferte den (3R,4R)-3-Amino-4-hydroxy-azepan-1-carbonsäure-tert.-butylester als farbloses Oel.

Das in B 23 eingesetzte Ausgangsmaterial wurde ausgehend von 5-Fluor-2-hydroxy-benzoesäure durch Umsetzung mit Benzylbromid in Dimethylformamid zu 2-Benzyloxy-5-fluor-benzoesäure-benzylester, basische Hydrolyse und Veresterung mit Acetonoxim hergestellt.

C. Die in Beispiel A eingesetzten Säuren wurden hergestellt durch

a) basische Hydrolyse entsprechender Ester;oder

b) Oxidation entsprechender Aldehyde mit Oxidationsmitteln wie Natriumchlorit, Persäuren oder $KMnO_4$; oder

c) Oxidation entsprechender Alkohole mit Oxidationsmitteln wie $MnO_2$

Die folgenden Verbindungen wurden erhalten:

C 1 (Methode a) 4-(2-Fluoro-4-methoxy-6-methoxymethoxy-benzoyl)-benzoesäure als farblose Kristalle, M.p. 136°C aus 4-(2-Fluoro-4-methoxy-6-methoxymethoxy-benzoyl)-benzoesäure methyl ester (D4)

C 2 (Methode a) 4-(2-Fluoro-6-methoxymethoxy-benzoyl)-benzoesäure als farblose Kristalle, M.p. 164°C aus 4-(2-Fluoro-6-methoxymethoxy-benzoyl)-benzoesäure methyl ester (D5)

C 3 (Methode a) 4-(2-Fluoro-3-methoxy-6-methoxymethoxy-benzoyl)-benzoesäure als farblose Kristalle, M.p. 167°C aus 4-(2-Fluoro-3-methoxy-6-methoxymethoxy-benzoyl)-benzoesäure methyl ester (D6)

C 4 (Methode b) 4-(3-m-Methoxymethoxy-naphthalen-2-ylcarbonyl)-benzoesäure aus 4-(3-Methoxymethoxy-naphthalen-2-ylcarbonyl)-benzaldehyde (L1)

C 5 (Methode a) 4-(2,3-Difluoro-6-methoxymethoxy-benzoyl)-benzoesäure als farblose Kristalle, M.p. 181°C aus 4-(2,3-Difluoro-6-methoxymethoxy-benzoyl)-benzoesäure-methyl ester (D7)

C 6 (Methode a) 4-(2,4-Difluoro-6-methoxymethoxy-benzoyl)-benzoesäure als farblose Kristalle, M.p. 139°C aus 4-(2,4-Difluoro-6-methoxymethoxy-benzoyl)-benzoesäure methyl ester (D8)

C 7 (Methode b) 4-(5-Methoxy-2-methoxymethoxy-benzoyl)-benzoesäure aus 4-(5-Methoxy-2-methoxymethoxy-benzoyl)-benzaldehyd (L2)

C 8 (Methode b) 4-(3-Methoxymethoxy-5,6,7,8-tetrahydro-naphthalin-2-ylcarbonyl)-benzoesäure aus 4-(3-methoxymethoxy-5,6,7,8-tetrahydro-naphthalin-2-ylcarbonyl)benzaldehyd (L3)

C 9 (Methode a) 4-(2-Methoxymethoxy-5-dimethylamino-benzoyl)-benzoesäure als rote Kristalle, M.p. 166°C aus 4-(2-Methoxymethoxy-5-dimethylamino-benzoyl)-benzoesäure methyl ester (D9)

C 10 (Methode b) 4-(7-Methoxymethoxy-2,3-dihydro-1,4-benzodioxin-6-ylcarbonyl)benzoesäure aus 4-(7-Methoxymethoxy-2,3-dihydro-1,4-benzodioxin-6-ylcarbonyl)-benzaldehyd (L4))

C 11 (Methode b) (RS)-4[5-(1-Methoxyethyl)-2-methoxymethoxy-benzoyl]-benzoesäure aus (RS)-4-[5(1-Methoxyethyl)-2-methoxymethoxy-benzoyl]-benzaldehyd (L5)

C 12 (Methode a) 4-(6-Methoxymethoxy-chinolin-7-ylcarbonyl)-benzoesäure aus 4-(6-Methoxymethoxy-chinolin-7-ylcarbonyl)-benzoesäure methyl ester ((D10)

C 13 (Methode a) 4-(4-Methoxymethoxy-biphenyl-3-ylcarbonyl)-benzoesäure aus 4(4-Methoxymethoxy-biphenyl-3-ylcarbonyl)-benzoesäure methyl ester (D11)

C 14 (Methode b) 4-(5-Acetyl-2-methoxymethoxy-benzoyl)-benzoesäure aus 4-(5-Acetyl-2-methoxymethoxy-benzoyl)-benzaldehyd (L6)

C 15 (Methode a) 4-(3-Methoxymethoxy-10-methyl-phenothiazin-2-ylcarbonyl)-benzoesäure als gelber Feststoff aus 4-(3-Methoxymethoxy-10-methyl-phenothiazin-2-ylcarbonyl)-benzoesäure methyl ester (D12)

MP = 219 $^{o}$C. MS : 422 $(M)^+$
IR: 3425, 1716, 1662, 1600, 1502, 1411, 1266, 1018.

C 16 (Methode a) 4-(2-Methoxymethoxy-5-methyl-benzoyl)-benzoesäure als weisser Feststoff aus 4-(2-Methoxymethoxy-5-methyl-benzoyl)-benzoesäure methyl ester (D 13)

MP: 128.2 $^{o}$C. MP : 300 $(M)^{+,}$ 269, 238
IR: 3431, 1693, 1668, 1230, 1147, 1110, 998, 806.

C 17 (Methode a) 4-(6-Methoxymethoxy-1,3-benzodioxol-5-ylcarbonyl)-benzoesäure aus 4-(6-Methoxymethoxy-1,3-benzodioxol-5-ylcarbonyl)-benzoesäure methyl ester (D14)

C 18 (Methode a) 4-(5-Isopropyl-2-methoxymethoxy-benzoyl)-benzoesäure als weisser Feststoff aus 4-(5-Isopropyl-2-methoxymethoxy-benzoyl)-benzoesäure methyl ester (D15)

MP: 122 $^{o}$C. MS : 328 $(M)^+$, 297, 283
IR: 2963, 1693, 1661, 1605, 1495, 1292, 1002, 823.

C 19 (Methode a) 4-(6-Methoxymethoxy-1,4-benzodioxin-5-ylcarbonyl)-benzoesäure als gelber Feststoff aus 4-(6-Methoxymethoxy-1,4-benzodioxin-5-ylcarbonyl)benzoesäure methyl ester (D 16)

MP: 124.2 °C. MS344 $(M)^+$, 299, 282
IR: 2937, 1677, 1599, 1483, 1270, 1073, 829.

C 20 (Methode a) 4-(2-Fluoro-3-dimethylamino-6-methoxymethoxy-benzoyl)-benzoesäure als gelbe Kristalle, M.p. 156°C aus 4-(2-Fluoro-3-dimethylamino-6-methoxymethoxy-benzoyl)-benzoesäure methyl ester (D17)

C 21 (Methode b) 4-(2-Fluoro-3-isopropyl-6-methoxymethoxy-benzoyl)-benzoesäure aus 4-(2-Fluoro-3-isopropyl-6-methoxymethoxy-benzoyl)-benzaldehyd ((L6)

C 22 (Methode a) 4-(2-Fluoro-6-methoxymethoxy-pyrrolidin-1-yl-benzoyl)-benzoesäure als orange Kristalle, M.p. 153°C aus 4-(2-Fluoro-6-methoxymethoxy-3-pyrrolidin-1-yl-benzoyl)-benzoesäure methyl ester (D18)

C 23 (Methode a) 4-(2-Fluoro-6-methoxyymethoxy-3-methyl-benzoyl)-benzoesäure als gelber Feststoff aus 4-(2-Fluoro-6-methoxymethoxy-3-methyl-benzoyl)-benzoesäure methyl ester (D19)

MP: 148.5 °C. MS : 318 $(M\ )^+$, 256
IR: 2827, 1699, 1675, 1487, 1263, 1055, 809.

C 24 (Methode a) 4-(3-Ethyl-2-fluoro-6-methoxymethoxy-benzoyl)-benzoesäure als hellgelber Feststoff aus 4-(3-Ethyl-2-fluoro-6-methomethoxy-benzoyl)benzoesäure methyl ester (D2)

MP: 148.5 °C. MS : 332 $(M)^+$,270
IR: 2934, 1703, 1679, 1468, 1265, 1150, 1036 809.

C 25 (Methode a) 4-(2-Fluoro-6-methoxymethoxy-piperidin-1-yl-benzoyl)-benzoesäure als gelbe Krisalle, M.p. 148°C aus 4-(2-Fluoro-6-methoxymethoxy-3-piperidin-1-yl-benzoyl)-benzoesäure methyl ester (D3)

C 26 (Methode c) 3,5-Difluoro-4-(5-methoxy-2-methoxymethoxy-benzoyl)-benzoesäure, Mp: 130-133(AcOEt/hexane) aus (RS)-3,5-Difluoro-4-[hydroxy-(5-methoxy-2-methoxymethoxy-phenyl)-methyl]benzoesäure (hergestellt durch nucleophile Addition von lithiierter 3,5-Difluorbenzoesäure an 5-Methoxy-2-methoxymethoxybenzaldehyd)

C 27 (Methode a) 4-(2-Methoxymethoxy-5-methylthio-benzoyl)-benzoesäure als gelber Feststoff aus 4-(2-Methoxymethoxy-5-methylthio-benzoyl)-benzoesäure methyl ester (D20)

Mp:129-130
MS: $332(M)^+$, 302, 270, 45
IR: 2909, 1693, 1667, 1482, 1290, 1232, 995, 804$cm^{-1}$

D. Die in Beispiel C eingesetzten Ester, Aldehyde und Alkohole wurden hergestellt durch

a) Methoxymethylierung der Verbindungen aus Beispiel (E) mittels Chlormethyl-methyläther und NaH; oder

b) Oxidation der Verbindungen aus Beispiel (F) mit $MnO_2$; oder

c) nucleophile Addition der Verbindungen aus Beispiel (G) oder entsprechender bekannter Verbindungen an Terephthalsäure-1-methylester 4-propan-2-on-oximester; oder

d) Oxidation der Verbindungen von Beispiel (F) mit Oxalylchlorid/DMSO.

Die folgenden Verbindungen wurden hergestellt:

D 2 (Methode c) 4-(3-Ethyl-2-fluoro-6-methoxymethoxy-benzoyl)-benzoesäuremethylester als farbloses Oel aus 4-Ethyl-2-fluoro-4-methoxymethoxy-benzol (G7) (Nucleophil) und Terephthalsäure-1-methylester 4-propan-2-on-oximester (Elektrophil);

MS: 346 (M)+, 315
IR: 2962, 1727, 1681, 1621, 1485, 1277, 1155, 1038, 816 $cm^{-1}$.

D 3 (Methode d) 4-(2-Fluoro-6-methoxymethoxy-3-piperidin-1-yl-benzoyl)-benzoesäuremethylester als gelbes viskoses Oel aus (RS)-4-[(2-Fluoro-6-methoxymethoxy-3-piperidin-1-yl-phenyl)-hydroxy-methyl]-benzoesäuremethylester

MS: m/e= 401 $(M)^{+\bullet}$
IR (Film): 1728, 1683, 1619, 1574, 1487, 1273 1038 $cm^{-1}$

D 4 (Methode a) 4-(2-Fluoro-4-methoxy-6-methoxymethoxy-benzoyl)-benzoesäuremethylester als hellgelbe Kristalle, M.p. 66°C aus 4-(2-Fluoro-6-hydroxy-4-methoxy-benzoyl)-benzoesäuremethylester (E3)

D 5 (Methode a) 4-(2-Fluoro-6-methoxymethoxy-benzoyl)-benzoesäure-methylester als farblose Kristalle, M.p. 75°C aus 4-(2-Fluoro-6-hydroxy-benzoyl)-benzoesäuremethylester (E2)

D 6 (Methode b) 4-(2-Fluoro-3-methoxy-6-methoxymethoxy-benzoyl)-benzoesäuremethylester als hellgelbe Kristalle aus (RS)-4-[(2-Fluoro-3-methoxy-6-methoxymethoxy-phenyl)-hydroxy-methyl]-benzoesäuremethylester (F1)

D 7 (Methode b) 4-(2,3-Difluoro-6-methoxymethoxy-benzoyl)-benzoesäuremethylester als farblose Kristalle, M.p. 82°C aus (RS)-4-[(2,3-Difluoro-6-methoxymethoxy-phenyl)-hydroxy-methyl]-benzoesäuremethylester (F2)

D 8 (Methode b) 4-(2,4-Difluoro-6-methoxymethoxy-benzoyl)-benzoesäure-methylester als farblose Kristalle, M.p. 73°C aus (RS)-4-[(2,4-Difluoro-6-methoxymethoxy-phenyl)-hydroxy-methyl]-benzoesäuremethylester (F3)

D 9 (Methode c) 4-(2-Methoxymethoxy-5-dimethylamino-benzoyl)-benzoesäuremethylester aus 1-Dimethylamino-4-methoxymethoxy-benzol (G1)

D 10 (Methode c) 4-(6-Methoxymethoxy-chinolin-7-ylcarbonyl)-benzoesäuremethylester aus 8-Bromo-7-methoxymethoxy-chinolin (G2)

D 11 (Methode b) 4-(4-Methoxymethoxy-biphenyl-3-ylcarbonyl)-benzoesäuremethylester aus (RS)-4-[Hydroxy-(4-methoxymethoxy-biphenyl-3-yl)-methyl]-benzoesäuremethylester (F4)

D 12 (Methode c) 4-(3-Methoxymethoxy-10-methyl-phenothiazin-2-ylcarbonyl)-benzoesäure methylester als Oel aus 3-Methoxymethoxy-10-methyl-phenothiazin (G3)

MS : 435 (M), 163
IR: 2948, 1713,1651, 1600, 1500, 1329, 1138, 830, 734.

D 13 (Methode c) 4-(2-Methoxymethoxy-5-methyl-benzoyl)-benzoesäuremethylester als weisser Feststoff aus 4-Methoxymethoxy-toluol; M.p. : 56 °C

MS : 314(M), 283
IR: 2929, 1720, 1662, 1607, 1495, 1281, 991, 820 $cm^{-1}$.

D 14 (Methode b) 4-(6-Methoxymethoxy-1,3-benzodioxol-5-ylcarbonyl)-benzoesäuremethylester aus (RS)-4-[Hydroxy-(6-methoxymethoxy-1,3-benzodioxol-5-yl)-methyl]-benzoesäuremethylester (F5)

D 15 (Methode c) 4-(5-Isopropyl-2-methoxymethoxy-benzoyl)-benzoesäure-methylester aus 1-Isopropyl-4-methoxymethoxy-benzene (G4)

MS: 342(M), 311, 297
IR: 2959, 2825, 1725, 1662, 1606, 1497, 1279, 1237, 992, 820 $cm^{-1}$

D 16 (Methode c) 4-(6-Methoxymethoxy-1,4-benzodioxin-5-ylcarbonyl)-benzoesäure als gelber Feststoff aus 2,3-Dihydroxy-6-(methoxymethoxy )-1,4-benzodioxin

MS : 358 (M), 327
IR: 2954, 1725, 1676, 1483, 1267, 800 $cm^{-1}$.

D 17 (Methode c) 4-(2-Fluoro-6-methoxymethoxy-3-dimethylamino-benzoyl)-benzoesäuremethylester aus 1-Fluoro-2-dimethylamino-4-methoxymethoxy-benzene (G5)

D 18 (Methode d) 4-(2-Fluoro-6-methoxymethoxy-3-pyrrolidin-1-yl-benzoyl)-benzoesäuremethylester als gelbes viskoses Oel aus (RS)-4-[(2-Fluoro-6-methoxymethoxy-3-pyrrolidin-1-yl-phenyl)-hydroxy-methyl]-benzoesäuremethylester (F6)

MS: m/e= 387 $(M)^{+\bullet}$
IR (Flm): 1726, 1681, 1620, 1571, 1493, 1281 1041 $cm^{-1}$

D 19 (Methode c) 4-(2-Fluoro-6-methoxymethoxy-3-methyl-benzoyl)benzoesäuremethylester als weisser Feststoff aus 2-Fluoro-4-methoxymethoxy-toluene (G6)

MS : 332 (M), 301
IR: 2952, 1730, 1679, 1627, 1483, 1279, 1155, 1024, 819 $cm^{-1}$.

D 20 (Methode d) Methyl 4-(2-methoxymethoxy-5-methylthio-benzoyl)-benzoate als gelbes Oel aus Methyl (RS)-4-[hydroxy-(2-methoxymethoxy-5-methylthio-phenyl)-methyl]benzoat ((F8)

MS: $346(M)^{+}$, 316, 270, 45
IR: 3433, 1734, 1724, 1664, 1485, 1284, 1237, 985 814$cm^{-1}$

D 21 (Methode b) 4-(2-Fluoro-4,6-dimethoxy-benzoyl)-benzoesäuremethylester als farblose Kristalle, M.p. 99°C aus (RS)-4-[(2-Fluoro-4,6-dimethoxy-phenyl)-hydroxy-methyl]-benzoesäuremethylester (F10)

Die Verbindungen der Beispiele D1 und D21 wurden wie folgt weiter umgesetzt:

E 1 Zu einer Lösung von 28.83 g 4-(5-Fluoro-2-methoxy-benzoyl)benzoesäuremethylester (D1) in 250 ml Dichlormethan wurden bei -78°C 11 ml Bortribromid zugetropft. Das Reaktiongemisch wurde eine Stunde bei -78°C unter Argon gerührt. Nach Zugabe von 100 ml Methanol wurde das Lösungsmittel weitgehend abgezogen. Nach erneuter Zugabe von 100 ml eiskaltem Methanol wurde das ausgefallene Produkt abgenutscht, mit 50 ml eiskaltem Methanol gewaschen, weitgehend trockengesaugt und aus Essigester/Hexan umkristallisiert. Man erhielt 26 g 4-(2-Fluoro-6-hydroxy-benzoyl)-benzoesäuremethylester als gelbe Kristalle. Smp. 101°C.
In analoger Weise wurden dargestellt:

E 2 4-(2-Fluoro-6-hydroxy-4-methoxy-benzoyl)-benzoesäuremethylester als farblose Kristalle, M.p. 154°C aus 4-(2-Fluoro-4,6-dimethoxy-benzoyl)-benzoesäuremethylester (D21)

F. Die Ausgangsstoffe für die Beispiele D3, D6. D7, D8, D11, D14, D18, D20, D21 und D22 wurden durch nucleophile Addition von Verbindungen der Beispiele (G) an Methyl 4-formylbenzoat wie folgt hergestellt:

F 1 (RS)-4-[(2-Fluoro-3-methoxy-6-methoxymethoxy-phenyl)-hydroxy-methyl]-benzoesäuremethylester, farblose Kristalle, Smp. 66°C aus 1-Fluoro-2-methoxy-5-methoxymethoxy-benzol (G8)

F 2 (RS)-4-[(2,3-Difluoro-6-methoxymethoxy-phenyl)-hydroxy-methyl]-benzoesäuremethylester aus 1,2-Difluoro-4-methoxymethoxy-benzene (G9)

F 3 (RS)-4-[(2,4-Difluoro-6-methoxymethoxy-phenyl)-hydroxy-methyl]-benzoesäuremethylester aus 1,3-Difluoro-5-methoxymethoxy-benzol (G10)

F 4 (RS)-4-[Hydroxy-(4-methoxymethoxy-biphenyl-3-yl)methyl]-benzoesäuremethylester aus 4-Methoxymethoxy-biphenyl

F 5 (RS)-4-[Hydroxy-(6-methoxymethoxy-1,3-benzodioxol-5-yl)-methyl]-benzoesäuremethylester aus 5-Bromo-6-methoxymethoxy-1,3-benzodioxol F 6 (RS)-4-[(2-Fluoro-6-methoxymethoxy-3-pyrrolidin-1-yl-phenyl)-hydroxy-

methyl]-benzoesäuremethylester aus 1-(2-Fluoro-4-methoxymethoxy-phenyl)-pyrrolidin (G11)

F 7 (RS)-4-[(2-Fluoro-6-methoxymethoxy-3-piperidin-1-yl-phenyl)-hydroxy-methyl]-benzoesäuremethylester aus 1-(2-Fluoro-4-methoxymethoxy-phenyl)-piperidin ((G12)

F 8 Methyl (RS)-4-[hydroxy-(2-methoxymethoxy-5-methylthio-phenyl)-methyl]-benzoat als gelbes Oel aus 1-Methoxymethoxy-4-methylthiobenzol

MS: 348$(M)^+$, 286, 271, 227, 45
IR: 3456, 1721, 1484, 1282, 1110, 995, 815$cm^{-1}$

F 9 (RS)-4-[(2-Fluoro-6-methoxy-phenyl)-hydroxy-methyl]-benzoesäure-methylester als farblose Kristalle, M.p. 115°C aus 3-Fluoro-anisol

F 10 (RS)-4-[(2-Fluoro-4,6-dimethoxy-phenyl)-hydroxy-methyl]-benzoesäuremethylester aus 5-Fluoro-1,3-dimethoxybenzol.

G. Die Ausgangsstoffe für die Beispiele D2, D9, D10,D12, D15, D17, D19, F1, F2, F3, F6 und F7 wurden durch O-Alkylierung der entsprechenden Phenole hergestellt:

G 1 1-Dimethylamino-4-methoxymethoxy-benzol als hellgelbes Oel aus 4-Hydroxy-N,N-dimethylanilin

B.p. 100°C/0.2 mbar
MS: m/e= 181 $(M)^{+\bullet}$, 136
IR (Flm): 1615, 1515, 1235, 1201, 1151, 1077, 1017 $cm^{-1}$

G 2 8-Bromo-7-methoxymethoxy-chinolin aus 7-Bromo-chinolin-6-ol

G 3 3-Methoxymethoxy-10-methyl-phenothiazine als Oel aus 10-Methyl-phenothiazin-3-ol

G 4 1-Isopropyl-4-methoxymethoxy-benzol als farbloses Oel aus 4-Isopropyl-phenol

MS: 180 (M), 165, 135
IR: 2960, 1610, 1512, 1234, 1009, 831.

G 5 1-Fluoro-2-dimethylamino-5-methoxymethoxy-benzol als farbloses Oel aus 3-Fluoro-4-dimethylamino-phenol (H3)

MS: m/e= 199 $(M)^{+\bullet}$, 154, 45
IR (KBr): 1575, 1512, 1273, 1250, 1150, 1011 $cm^{-1}$

G 6 2-Fluoro-4-methoxymethoxy-toluol als Oel aus 3-Fluoro-4-methyl-phenol (H2).

MS : 170 (M)
IR : 2956, 1630, 1590, 1510, 1265, 1153, 1012, 850
Anal. calc. for $CgH_{11}O_2F$ (170.183): C 63.52, H 6.52; found: C 63.25, H 6.52.

G 7 1-Ethyl-2-fluoro-4-methoxymethoxy-benzol als Oel aus 4-Ethyl-3-fluoro-phenol (H1).

G 8 1-Fluoro-2-methoxy-5-methoxymethoxy-benzol als farbloses Oel aus 3-Fluoro-4-methoxyphenol

B.p. 165°C/10 mbar
MS: m/e= 186 $(M)^{+\bullet}$, 45
IR (Flm): 1599, 1512, 1266, 1223, 1153, 1121, 1077, 1030, 1009 $cm^{-1}$

G 9 1,2-Difluoro-4-methoxymethoxy-benzol als farbloses Oel, B.p. 86°C/10 mbar aus 3,4-Difluorophenol

MS: m/e= 174 $(M)^{+\bullet}$, 45
IR (Flm): 1616, 1516, 1256, 1220, 1204, 1153, 1077, 1007 $cm^{-1}$

G 10 1,3-Difluoro-5-methoxymethoxy-benzol als farbloses Oel aus 3,5-Difluorophenol

B.p. 79°C/10 mbar
MS: m/e= 174 $(M)^{+\bullet}$, 45
IR (Flm): 1628, 1600, 1472, 1224, 1156, 1138, 1116, 1080, 1028 $cm^{-1}$

G 11 1-(2-Fluoro-4-methoxymethoxy-phenyl)-pyrrolidin als hellgelbes Oel aus 3-Fluoro-4-(pyrrolidin-1-yl)-phenol (H4)

B.p. 135°C/0.2 mbar
MS: m/e= 225 $(M)^{+\bullet}$, 180
IR (Flm): 1579, 1516, 1270, 1152, 1015 $cm^{-1}$

G 12 1-(2-Fluoro-4-methoxymethoxy-phenyl)-piperidin als farbloses Oel aus 3-Fluoro-4-(piperidin-1-yl)-phenol (H2)

B.p. 135°C/0.2 mbar
MS: m/e= 239 $(M)^{+\bullet}$, 194, 45
IR (Flm): 1628, 1582, 1508, 1272, 1257, 1154, 1012 $cm^{-1}$

H. Die Ausgangsstoffe für die Beispiele G5, G7,G11, und G12 wurden durch O-Dealkylierung entsprechender Vorstufen

a) mit Phosphortribromid, oder

b HBr/Eisessig

wie folgt hergestellt:

H 1 (Methode a) 4-Ethyl-3-fluoro-phenol als Flüssigkeit aus 1-Ethyl-2-fluoro-4-methoxy-benzol (I1)

MS : 141, 125
IR : 3343, 2971, 1626, 1598, 1509, 1283, 1148, 844 $cm^{-1}$.

H 2 (Methode b) 3-Fluoro-4-(piperidin-1-yl)-phenol als farbloser Feststoff aus 1-(2-Fluoro-4-methoxy-phenyl)-piperidin (I3) Mp 134°C

H 3 (Methode b) 3-Fluoro-4-dimethylamino-phenol als grauweisser Feststoff aus 1-Fluoro-2-dimethylamino-5-methoxy-benzol (I2)

H 4 (Methode b) 1-(2-Fluoro-4-hydroxy-phenyl)-pyrrolidin als grauweisser Feststoff aus 1-(2-Fluoro-4-methoxy-phenyl)-pyrrolidin

I . Die Ausgangsstoffe für die Beispiele H wurden durch Reduktion

a) eines entsprechenden Acetophenons; oder

b) eines entsprechenden Amids

wie folgt hergestellt:

I 1 (Methode a) 1-Ethyl-2-fluoro-4-methoxy-benzene als gelbe Flüssigkeit aus 2-Fluoro-4-methoxy-acetophenone

MS : 154 (M), 139 ($M-CH_3$)
IR : 2400, 1627, 1585, 1285, 1154, 1034, 833 $cm^{-1}$.

I 2 (Methode b) 1-Fluoro-2-dimethylamino-5-methoxy-benzol als farblose Kristalle aus N-(2-Fluoro-4-methoxy-phenyl)-N-methyl-formamid (J1)

Mp.: 125-126°C.

I 3 (Methode b) 1-(2-Fluoro-4-methoxy-phenyl)-piperidin als hellgelbes Oel (B.p. 125°C/0.2 mbar) aus 1-(2-Fluoro-4-methoxy-phenyl)-piperidin-2-on (J2)

MS: m/e= 209 $(M)^{+\bullet}$, 208 $(M-H)^+$, 194
IR (KBr): 1626, 1580, 1510, 1230, 1157, 1140, 1120, 1043 $cm^{-1}$

I 4 (Methode b) 1-(2-Fluoro-4-methoxy-phenyl)-pyrrolidin als farblose Kristalle, M.p. 143°C aus 1-(2-Fluoro-4-methoxy-phenyl)-pyrrolidin-2-on (J3)

J. Die Ausgangsstoffe für die Beispiele I2,I3, und I4 wurden durch

a) Methylierung eines entsprechenden N-Formanilids, oder

b) basische Cyclisierung eines entsprechenden N-Bromanilids wie folgt hergestellt:

J 1 (Methode a) N-(2-Fluoro-4-methoxy-phenyl)-N-methyl-formamid als farbloses viskoses Oel aus N-(2-Fluoro-4-methoxy-phenyl)-formamid (K1)

MS: m/e= 183 $(M)^{+\bullet}$, 140
IR (Flm): 1683, 1624, 1587, 1516, 1289, 1160 $cm^{-1}$

J 2 (Methode b) 1-(2-Fluoro-4-methoxy-phenyl)-piperidin-2-on als farblose Kristalle, M.p.: 92°C aus 5-Bromo-N-(2-fluoro-4-methoxy-phenyl)-valeriansäureamid (K2)

J 3 (Methode b) 1-(2-Fluoro-4-methoxy-phenyl)-pyrrolidin-2-on als hellgelbes viskoses Oel aus 4-Bromo-N-(2-fluoro-4-methoxy-phenyl)-butyramid (K3)

MS: m/e= 209 $(M)^{+\bullet}$, 154
IR (Flm): 1700, 1624, 1588, 1516, 1287, 1158 $cm^{-1}$

K. Die Ausgangsstoffe für die Beispiele J wurden hergestellt durch

a) Formylierung eines entsprechenden Anilins; oder

b) Acylierung eines entsprechenden Anilins :

K 1 (Methode a) N-(2-Fluoro-4-methoxy-phenyl)-formamid, farblose Kristalle,aus 2-Fluoro-4-methoxyanilin

MS: m/e= 169 $(M)^{+\bullet}$, 126

IR (KBr): 1658, 1646, 1621, 1590, 1525, 1460, 1429, 1390, 1300, 1223, 1180, 1110, 1035, 872, 802 $cm^{-1}$

K 2 (Methode b) 5-Bromo-N-(2-fluoro-4-methoxy-phenyl)-valeriansäureamid, farblose Kristalle, M.p. 99°C aus 2-Fluoro-4-methoxyanilin und 5-Bromvaleriansäure

K 3 (Methode b) 4-Bromo-N-(2-fluoro-4-methoxy-phenyl)-butyramid, farblose Kristalle, Mp 88°C aus 2-Fluoro-4-methoxyanilin und 4-Brombuttersäure

L. Die in Beispiel B, Methode b) eingesetzten Aldehyde wurden nach dem Verfahren von Beispiel D wie folgt hergestellt:

L 1 4-(3-Methoxymethoxy-naphthalin-2-ylcarbonyl)benzaldehyd (aus (4-Hydroxymethyl-phenyl)-(3-methoxymethoxy-naphthalen-2-yl)-methanon (M1)

L 2 4-(5-Methoxy-2-methoxymethoxy-benzoyl)-benzaldehyd aus (RS)-(4-Hydroxymethyl-phenyl)-(5-methoxy-2-methoxymethoxy-phenyl)-methanol (M2)

L 3 4-(3-Methoxymethoxy-5,6,7,8-tetrahydro-naphthalin-2-ylcarbonyl)-benzaldehyde aus (RS)-(4-Hydroxymethyl-phenyl)-(3-methoxymethoxy-5,6,7,8-tetrahydro-naphthalin-2-yl)-methanol (M3)

L 4 4-(7-Methoxymethoxy-2,3-dihydro-1,4-benzodioxin-6-ylcarbonyl)-benzaldehyd aus (RS)-(4-Hydroxymethyl-phenyl)-(7-methoxymethoxy-2,3-dihydro-1,4-benzodioxin-6-yl)methanol (M4)

L 5 (RS)-4-[5-(1-methoxyethyl)-2-methoxymethoxy-benzoyl]-benzaldehyde aus (SR)-(4-hydroxymethyl-phenyl)-[5-[(RS) und (SR)-1-methoxyethyl]-2-methoxymethoxyphenyl]-methanol (M5)

L 6 4-(5-Acetyl-2-methoxymethoxy-benzoyl)-benzaldehyd (aus (RS)-1-[3-[(RS) und (SR)-Hydroxy-(4-hydroxymethyl-phenyl)-methyl]-4-methoxymethoxy-phenyl]-ethanol (M6)

L 7 4-(2-Fluoro-3-isopropyl-6-methoxymethoxy-benzoyl)-benzaldehyd aus (RS)-(2-Fluoro-3-isopropyl-6-methoxymethoxy-phenyl)-(4-hydroxymethyl)-phenyl)-methanol (M7)

M. Die in Beispiel L eingesetzten Verbindungen wurden hergestellt durch

a) O-Desilylierung von Verbindungen aus Beispiel N; oder

b) nucleophile Addition des Li-Salzes von 4-Brombenzylalkohol an die Verbindungen von Beispiel O oder entsprechende bekannte Verbindungen; oder

c) Oxidation von Verbindungen von Beispiel N und anschliessende O-Desilylierung.

Die folgenden Verbindungen wurden so hergestellt:

M 1 (Methode a) (4-Hydroxymethyl-phenyl)-(3-methoxymethoxy-naphthalen-2-yl)-methanone aus [4-(tert-Butyl-dimethyl-silanyloxymethyl)-phenyl]-(3-methoxymethoxy-naphthalen-2-yl)-methanon (N1)

M 2 (Methode b) (RS)-(4-Hydroxymethyl-phenyl)-(5-methoxy-2-methoxymethoxy-phenyl)-methanol aus 5-Methoxy-2-methoxymethoxy-benzaldehyd.

M 3 (Methode a) (RS)-(4-Hydroxymethyl-phenyl)-(3-methoxymethoxy-5,6,7,8-tetrahydro-naphthalin-2-yl)-methanol aus (RS)-[4-(tert-butyl-dimethylsilanyloxymethyl)-phenyl]-(3-methoxymethoxy-5,6,7,8-tetrahydro-naphthalin-2-yl)-methanol (N2)

M 4 (Methode b) (RS)-(4-Hydroxymethyl-phenyl)-(7-methoxymethoxy-2,3-dihydro-1,4-benzodioxin-6-yl)methanol

aus 7-mMethoxymethoxy-2,3-dihydro-1,4-benzodioxin-6-carbaldehyd (O1)

M 5 (Methode b) (SR)-(4-Hydroxymethyl-phenyl)-[5-[(RS) and (SR)-1-methoxyethyl]-2-methoxymethoxyphenyl]-methanol aus (RS)-5-(1-methoxy-ethyl)-2-methoxymethoxy-benzaldehyd (O2)

M 6 (Methode a)(RS)-1-[3-[(RS) and (SR)-Hydroxy-(4-hydroxymethyl-phenyl)-methyl]-4-methoxymethoxyphenyl]-ethanol aus (RS)-[5-[(RS) and (SR)-1-(tert-Butyldimethylsilanyloxy)-ethyl]-2-methoxymethoxyphenyl]-(4-hydroxy-methyl-phenyl)-methanol (N3)

M 7 (Methode a) (RS)-(2-Fluoro-3-isopropyl-6-methoxymethoxy-phenyl)-(4-hydroxymethyl)-phenyl)-methanol aus (RS)-[4-(tert-butyldimethylsilanyloxymethyl)-phenyl](2-fluoro-3-isopropyl-6-methoxymethoxy-phenyl)-methanol (N4)

M 8 (Methode c) (4-Hydroxymethyl-phenyl)-(5-methoxy-2-methoxymethoxy-phenyl)-methanon aus (RS)-[4-(tert-Butyldimethylsilanyloxymethyl)-phenyl]-(5-methoxy-2-methoxymethoxy-phenyl)-methanol (N5)

N. Die in Beispiel M bei den Methoden a) und c) eingesetzten Verbindungen wurden hergestellt durch

a) nucleophile Addition von lithiiertem [(4-Bromobenzyl)oxy]-tert-butyldimethylsilan an 3-Ethoxymethoxy-naphthalin-2-carbonsäuremethyl ester; oder

b) nucleophile Addition des in a) verwendeten Li-Reagenzes an Verbindungen von Beispiel O oder entsprechende bekannte Verbindungen; oder

c) nucleophile Addition von lithiiertem 4-Brombenzylalkohol an Verbindungen von Beispiel O; oder

d) nucleophile Addition von Verbindungen von Beispiel 53 an (4-Formylbenzyl)oxy]tert-butyldimethylsilan

Die folgenden Verbindungen wurden so hergestellt:

N 1 (Methode a) [4-(tert-Butyl-dimethyl-silanyloxymethyl)-phenyl]-(3-methoxymethoxy-naphthalen-2-yl)-methanon aus [(4-Bromobenzyl)oxy]tert-butyldimethylsilan und 3-Methoxymethoxy-naphthalin-2-carbonsäure-methylester.

N 2 (Methode b) (RS)-[4-(tert-Butyldimethylsilanyloxymethyl)-phenyl]-(3-methoxymethoxy-5,6,7,8-tetrahydro-naphthalin-2-yl)-methanol aus 3-Methoxymethoxy-5,6,7,8-tetrahydro-naphthaline-2-carbaldehyd (O3)

N 3 (Methode c) (RS)-[5-[(RS) and (SR)-1-(tert-Butyldimethylsllanyloxy)-ethyl]-2-methoxymethoxyphenyl]-(4-hydroxymethyl-phenyl)-methanol aus (RS)-5-[(1-tertButyldimethylsilanyloxy)ethyl]-2-methoxymethoxy-benzaldehyd (O4)

N 4 (Methode d) (RS)-[4-(tert-Butyldimethylsilanyloxymethyl)-phenyl]-(2-fluoro-3-isopropyl-6-methoxymethoxy-phenyl)-methanol (aus 2-Fluoro-4-methoxymethoxy-1-(1-methylethyl)benzol und (4-Formylbenzyl)oxy]tert-butyldimethylsilan

N 5 (Methode b) (RS)-[4-(tert-Butyldimethylsilanyloxymethyl)-phenyl]-(5-methoxy-2-methoxymethoxy-phenyl)-methanol aus 5-Methoxy-2-methoxymethoxy-benzaldehyd

O. Die in Beispiel M und N eingesetzten Verbindungen wurden hergestellt durch

a) Methoxymethylierung bekannter Phenole; oder

b) Oxidation von Verbindungen Beispiel P

Die folgenden Verbindungen wurden so hergestellt:

O 1 (Methode a) 7-Methoxymethoxy-2,3-dihydro-1,4-benzodioxin-6-carbaldehyd (aus 7-Hydroxy-2,3-dihydro-1,4-benzodioxin-6-carbaldehyd

O 2 (Methode b) 5-(1-Methoxyethyl)-2-methoxymethoxy-benzaldehyde aus (RS)-[5-(1-Methoxyethyl)-2-methoxymethoxy-phenyl]-methanol ((P1)

O 3 (Methode a) 3-Methoxymethoxy-5,6,7,8-tetrahydro-naphthalin-2-carbaldehyd aus 3-Hydroxy-5,6,7,8-tetrahydro-naphthalin-2-carbaldehyd

O 4 (Methode b) (RS)-5-[(1-tert Butyldimethylsilanyloxy)ethyl]-2-methoxymethoxy-benzaldehyd aus (RS)-5-[(1-tert Butyldimethylsilanyloxy)-ethyl]-2-methoxymethoxy-phenyl]methanol (P2)

Die in N 4 eingesetzten Ausgangsstoffe wurden wie folgt hergestellt:

a) 2-Fluor-4-hydroxy-acetophenon wurde mit Chlormethyl-methylether in Gegenwart von NaH zu 2-Fluor-4-methoxymethoxy-acetophenon umgesetzt, das mit Methyltriphenylphosphoniumbromid in einer Wittig-Reaktion 2-Fluor-4-methoxymethoxy-1-(1-methylvinyl)benzol lieferte. Hydrierung über Pd/C lieferte 2-Fluor-4-methoxymethoxy-1-(1-methylethyl)benzol.

b) [4-(Formylbenzyl)oxy]tert.-butyldimethylsilan wurde durch Formylierung von [(4-Brombenzyl)oxy]tert.-butyldimethylsilan mit BuLi/Dimethylformamid bei -78°C erhalten.

P. Die in Beispiel O2 und O4 eingesetzten Verbindungen wurden durch Reduktion von Verbindungen von Beispiel Q hergestellt:

P 1 (RS)-[5-(1-Methoxyethyl)-2-methoxymethoxy-phenyl]-methanol aus (RS)-5-(1-Methoxyethyl)-2-methoxymethoxybenzoesäuremethylester (Q2)

P 2 (RS)-5-[(1-tert Butyldimethylsilanyloxy)-ethyl]-2-methoxymethoxy-phenyl]methanol aus (RS)-5-[(1-tert-Butyl-dimethyl-silanyloxy)-ethyl]-2-methoxymethoxy-benzoesäuremethylester (Q1)

Q. Die Verbindungen P1 und P2 wurden wie folgt hergestellt:

5-Acetyl-2-hydroxybenzoesäuremethylester wurde in 5-Acetyl-2-methoxymethoxy-benzoesäuremethylester übergeführt, aus dem durch Reduktion mit Natriumborhydrid der (RS)-5-(1-Hydroxyethyl)-2-methoxymethoxy-benzoesäuremethylester erhalten wurde. Daraus wurden durch O-Silylierung

Q 1 (RS)-5-[(1-tert-Butyl-diniethyl-silanyloxy)-ethyl]-2-methoxymethoxy-benzoesäuremethylester und durch O-Alkylierung

Q 2 (RS)-5-(1-Methoxyethyl)-2-methoxymethoxy-benzoesäuremethylester

erhalten.

R. Die in Beispiel B eingesetzten Verbindungen wurden hergestellt durch Acylierung von tert-Butyl-(3R,4R)-4-benzyloxy-azepan-1-carboxylat (S) mit bekannten oder in an sich bekannter Weise hergestellten aktivierten Carbonsäuren.

Die folgenden Verbindungen wurden so hergestellt:

R 1 (3R,4R)-4-Benzyloxy-3-(4-methoxymethoxy-benzoylamino)-azepan-1-carbonsäure-tert-butylester aus 4-(Methoxymethoxy)benzoesäure;

R 2 (3R,4R)-4-Benzyloxy-3-[4-[dimethyl-(1,1,2-trimethylpropyl)siloxy]-benzoylamino]azepan-1-carbonsäure-tert-butylester als farbloses Oel aus 4-[Dimethyl(1,1,2-trimethylpropyl)siloxy] benzoesäure

IMS: 583$(M+H)^+$, 527, 483, 263

IR: 3343, 1693, 1663, 1605, 1534, 1491, 1416, 1243, 1165, 1148, 1072, 996, 737, 698cm$^{-1}$

R 3 (3R,4R)-4-Benzyloxy-3-(3-methoxy-4-methoxymethoxy-benzoylamino)-azepan-1-carbonsäure-tert-butylester als farbloses viskoses Oel aus 3-Methoxy-4-methoxymethoxy benzoesäure

R 4 (3R,4R)-4-Benzyloxy-3-(5-methoxy-2-methoxymethoxy-benzoylamino)-azepan-1-carbonsäure-tert-butylester aus 5-Methoxy-2-methoxymethoxy-benzoesäure

R 5 (3R,4R)-4-Benzyloxy-3-[4-(tert-butyldimethylsilanyloxy)-benzoylamino]-azepan-1-carbonsäure-tert-butylester aus 4-(tert-Butyldimethylsilanyloxy)-benzoesäure

R 6 (3R,4R)-4-Benzyloxy-3-(3,5-dimethoxy-4-methoxymethoxy-benzoylamino)-azepan-1-carbonsäure-tert-butylester als farbloser Schaum aus 3,5-Dimethoxy-4-methoxymethoxy benzoesäure

MS: 545.4 (M+H)$^+$
IR (Flm): 3326, 1693, 1663, 1585, 1539, 1159, 1079 cm$^{-1}$

R 7 (3R,4R)-4-Benzyloxy-3-(3,5-bis-methoxymethoxy-naphthalin-2-ylcarbonylamino)-azepan-1-carbonsäure-tert-butylester aus 3,5-bis(Methoxymethoxy)-2-naphthoesäure

R 8 (3R,4R)-4-Benzyloxy-3-(3,5-dimethyl-benzoylamino)azepan-1-carbonsäure-tert-butylester als farbloses Oel aus 3,5-Dimethoxybenzoesäure

MS: 513.7(M+H)$^+$, 457.6, 413.5
IR: 3330, 1694, 1665, 1593, 1531, 1416, 1172, 1059, 764, 699cm$^{-1}$

R 9 (3R,4R)-4-Benzyloxy-3-(3-methoxy-2-methoxymethoxy-benzoyl-amino)-azepan-1-carbonsäure-tert-butylester als farbloses viskoses Oel aus 3-Methoxy-2-(methoxymethoxy) benzoesäure

MS: 515.5 (M+H)$^+$
IR (Flm): 3378, 1690, 1658, 1579, 1523, 1166, 1077 cm$^{-1}$

R 10 (3R,4R)-4-Benzyloxy-3-(3-methoxymethoxy-naphthalen-2-ylcarbonyl-amino)-azepan-1-carbonsäure-tert-butylester (Ro-48-4163) aus 3-Methoxymethoxy-2-naphthalin-2-carbonsäure

R 11 (3R,4R)-4-Benzyloxy-3-(4-methoxymethoxy-2,3,6-trimethyl-benzoyl-amino)-azepan-1-carbonsäure-tert-butylester als farbloser Schaum aus 4-Methoxymethoxy-2,3,6-trimethyl-benzoesäure

MS: 527.6 (M+H)$^+$
IR (KBr): 3326, 1694, 1638, 1597, 1524, 1156, 1065 cm$^{-1}$

R 12 (3R,4R)-4-Benzyloxy-3-(3,5-diisopropyl-4-methoxymethoxy-benzoyl-amino)-azepan-1-carbonsäure-tert-butylester als farbloser Schaum aus 4-Methoxymethoxy-3,5-bis-(1-methylethyl) benzoesäure

MS: 569.9 (M+H)$^+$
IR (Flm): 3330, 1695, 1664, 1603, 1531, 1162, 1070 cm$^{-1}$

R 13 (3R,4R)-4-Benzyloxy-3-(3,4,5-trimethoxy-benzoylamino)azepan-1-carbonsäure-tert-butylester als farbloses Pulver aus 3,4,5-trimethoxy-benzoesäure

MS: 519.4(M+H)$^+$ 441.4, 397.
IR: 3273, 1696, 1664, 1632, 1584, 1542, 1416, 1234, 1128, 736, 697cm$^{-1}$

R 14 (3R,4R)-Benzyloxy-3-(4-methoxymethoxy-3,5-dimethyl-benzoylamino)-azepan-1-carbonsäure-tert-butylester als farbloser Schaum aus 4-Methoxymethoxy-3,5-dimethylbenzoesäure

MS: 511.8 (M+H)$^+$
IR (Flm): 3342, 1693, 1665, 1602, 1530, 1161, 1072 cm$^{-1}$

R 15 (3R,4R)-3-(3-Acetyl-4-methoxymethoxy-benzoylamino)-4-benzyloxy-azepan-1-carbonsäure-tert-butylester als farbloser Schaum aus 3-Acetyl-4-methoxymethoxybenzoesäure

MS: 527.5$(M+H)^+$, 471.6
IR: 3334, 1680, 1663, 1603, 1535, 1487, 1415, 1165, 1086, 977, 738, 698$cm^{-1}$

R 16 (3R,4R)-4-Benzyloxy-3-(3,5-diethoxy-benzoylamino)azepan-1-carbonsäure-tert-butylester, hellgelbes Oel aus 3,5-Diethoxybenzoesäure

MS: 485.7$(M+H)^+$, 429.5, 385.6
IR: 3334, 1737, 1665, 1595, 1532, 1419, 1363, 1158, 1066, 765, 700$cm^{-1}$

R 17 (3R,4R)-Benzyloxy-3-(3,5-diethyl-4-methoxymethoxy-benzoyl-amino)-azepan-1-carbonsäure-tert-butylester, farbloser Schaum, aus 3,5-Diethyl-4-methoxymethoxy benzoesäure

[MS: 541.5 $(M+H)^+$
IR (Flm): 3335, 1694, 1665, 1604, 1532, 1162, 1074 $cm^{-1}$

R 18 (3R,4R)-4-Benzyloxy-3-(3-tert-butyl-4-hydroxy-benzoylamino)azepan-1-carbonsäure-tert-butylester, farbloses Pulver, aus 3-(1,1-Dimethylethyl)-4-hydroxy-benzoesäure

MS: 513.4$(M-H)^-$
IR: 3324, 1665, 1637, 1601, 1551, 1417, 1272, 1156, 735, 697$cm^{-1}$

R 19 (3R,4R)4-benzyloxy-3-(3-isopropyl-4-methoxymethoxy-benzoylamino)-azepan-1-carbonsäure-tert-butylester, farbloses Oel, aus 4-Methoxymethoxy-2-(1-methylethyl)benzoesäure

MS: 527.5$(M+H)^+$, 471.5, 427.4
IR: 3346, 2960, 1695, 1665, 1604, 1537, 1500, 1259, 1165, 1100, 909$cm^{-1}$

R 20 (3R,4R)-4-Benzyloxy-3-(3-sec-butyl-4-methoxymethoxy-benzoylamino)-azepan-1-carbonsäure-tert-butylester, farbloses Oel, aus 4-methoxymethoxy-2-(1-methylpropyl)benzoesäure

MS: 541.5$(M+H)^+$, 485.5, 441.5
IR: 3341, 1694, 1663, 1605, 1534, 1491, 1416, 1241, 1165, 1150, 1074, 996, 736, 698$cm^{-1}$

S. Das in Beispiel (R) eingesetzte tert-Butyl (3R,4R)-3-Amino-4-benzyloxy-azepan-1-carboxylat wurde wie folgt hergestellt:

Ethyl-2,3,6-trideoxy-alpha-D-erythrohexopyranosid wurde mit p-Toluol-sulfochlorid zu Ethyl 6-O-(p-tolylsulfonyl)-2,3-dideoxy-alpha-D-erythrohexopyranosid umgesetzt. Durch Reaktion mit Natriumazid wurde daraus das Ethyl 6-Azido-2,3,6-trideoxy-alpha-D-erythrohexopyranosid und daraus mit 4-Nitrobenzoesäure unter Mitsunobu-Bedingungen das Ethyl 6-Azido-2,3,6-trideoxy-4-O(4-nitrobenzoyl)-alpha-D-threohexopyranosid erhalten. Basische Hydrolyse der letzteren Verbindung lieferte Ethyl 6-Azido-2,3,6-trideoxy-alpha-D-galaktopyranosid, das durch Benzylierung and anschliessende saure Hydrolyse in 6-Azido-5-O-bnzyl-2,3,6-trideoxy-D-galactopyranose übergeführt wurde. Katalytische Hydrierung (PtO/Raumtemperatur) und nachfolgende Umsetzung mit bis-tert-Butylcarbonat lieferte tert Butyl (3S,4R)-4(benzyloxy)-hexahydro-3-hydroxy-1H-azepin-1-carboxylat. Acylierung unter Mitsunobu-Bedingungen zu tert Butyl (3S,4R)-4-(benzyloxy)-hexahydro-3-O-(4-nitrobenzoyl)-1H-azepin-1-carboxylat, basische Hydrolyse und Umsetzung mit Hydrazinsäure unter Mitsunobu-Bedingungen lieferte das tert-Butyl (3R,4R)-3-Azido-4-(benzyloxy)-hexahydro-1H-azepin-1-carboxylat, aus dem durch Hydrierung (Pd/C) das in Beispiel S eingesetzte Amin erhalten wurde.

T. Die in den Beispielen 65-67 eingesetzten Verbindungen wurden hergestellt aus Verbindungen der Beispiele A durch

a) Hydrierung einer Benzylgruppe; oder

b) Desilylierung.

Auf diese Weise wurden hersgestellt:

T 1 (Methode a) (3R,4R)-3-(5-Fluoro-2-hydroxy-benzoylamino)-4-[4-(5-methoxy-2-methoxymethoxy-benzoyl)-benzoyloxy]-azepin-1-carbonsäure-tert-butylester, farbloser Schaum, aus (3R,4R)-3-(2-Benzyloxy-5-fluoro-benzoylamino)-4-[4-(5-methoxy-2-methoxymethoxy-benzoyl)-benzoyloxy]-azepin-1-carbonsäure-tert-butylester (letztere Verbindung hergestellt aus (B23) und (C7);

T 2 (Methode b)(3R,4R)-4-[3,5-Difluoro-4-(5-methoxy-2-methoxymethoxy-benzoyl)-benzoyloxy]-3-(4-hydroxy-benzoylamino)-azepan-1-carbonsäure-tert-butylester (Ro-47-2629), hellgelber Schaum, aus (3R,4R)-4-[(3,5-Difluoro-4-(5-methoxy-2-methoxymethoxy-benzoyl)-benzoyloxy]-3-[4-[dimethyl-(1,1,2-trimethylpropyl)-silanyloxy]-benzoylamino]-4-hydroxy-azepan-1-carbonsäure-tert-butylester (A1)

T 3 (Methode b) (3R,4R)-3-(4-Hydroxy-benzoylamino)-4-[4-(2-methoxymethoxy-5-methylthio-benzoyl)-benzoyloxy]-azepan-1-carbonsäure-tert-butylester, gelber Schaum, aus (3R,4R)-4-[4-(2-Methoxymethoxy-5-methylthio-benzoyl)-benzoyloxy]-3-[4-[dimethyl(1,1,2-trimethylpropyl)siloxy]-benzoylamino)-azepan-1-carbonsäure-tert-butylester

MS: 665.4$(M+H)^+$, 609.4
IR: 3391, 1720, 1666, 1609, 1505, 1277, 1158, 1105, 983, 848$cm^{-1}$

U. Die in den Beispielen 68-76 eingesetzten Verbindungen wurden durch Reduktion eines Azids zum Amin und Acylierung mit bekannten aktivierten Benzoesäurederivaten hergestellt.

Auf diese Weise wurden erhalten:

U 1 (3RS,4SR)-3-[4-(5-Methoxy-2-methoxymethoxy-benzoyl)-benzoyloxy]-4-(4-methoxymethoxy-3,5-dimethyl-benzoylamino)-pyrrolidin-1-carbonsäure-tert-butylester, farbloser Schaum, aus (3RS,4SR)-3-Azido-4-[4-(5-methoxy-2-methoxymethoxy-benzoyl)-benzoyloxy]-pyrrolidin-1-carbonsäure-tert-butylester (V1) und 4-Methoxymethoxy-3,5-dimethylbenzoesäure

U 2 (3RS,4RS)-3-[4-(5-Methoxy-2-methoxymethoxy-benzoyl)-benzoyloxy]-4-(4-methoxymethoxy-3,5-dimethyl-benzoylamino)-pyrrolidin-1-carbonsäure-tert-butylester, farbloser Schaum aus (3RS,4RS)-3-Azido-4-[4-(5-methoxy-3-methoxymethoxy-benzoyl)-benzoyloxy]-pyrrolidin-1-carbonsäure-tert-butylester (V2) und 4-methoxymethoxy-3,5-dimethyl-benzoesäure

U 3 (3RS,4RS)-3-[4-(5-Methoxy-2-methoxymethoxy-benzoyl)-benzoyloxy]-4-(4-methoxymethoxy-benzoylamino)-pyrrolidin-1-carbonsäure-tert-butylester, farbloser Schaum, aus (3RS,4RS)-3-Azido-4-[4-(5-methoxy-3-methoxymethoxy-benzoyl)-benzoyloxy]-pyrrolidin-1-carbonsäure-tert-butylester (V2) und 4-Methoxymethoxy-benzoesäure

U 4 (3RS,4RS)-3-[4-(2-Fluoro-3-methoxy-6-methoxymethoxy-benzoyl)-benzoyloxy]-4-(4-methoxymethoxy-3,5-dimethyl-benzoylamino)-pyrrolidin-1-carbonsäure-tert-butylester aus (3RS,4RS)-3-Azido-4-[4-(2-fluoro-3-methoxy-6-methoxymethoxy-benzoyl)-benzoyloxy]-pyrrolidin-1-carbonsäure-tert-butylester (V3) und 4-Methoxymethoxy-3,5-diiiiethyl-benzoesäure

U 5 (3RS,4RS)-3-[4-(2-Fluoro-3-methoxy-6-methoxymethoxy-benzoyl)-benzoyloxy]-4-pyridin-4-ylcarbonylamino-pyrrolidin-1-carbonsäure-tert-butylester (Ro-48-4206), farbloser Schaum, aus (3RS,4RS)-3-Azido-4-[4-(2-fluoro-3-methoxy-6-methoxymethoxy-benzoyl)-benzoyloxy]-pyrrolidin-1-carbonsäure-tert-butylester (V3), aus Isonicotinsäure

U 6 (3RS,4RS)-3-[4-(2-Fluoro-3-methoxy-6-methoxymethoxy-benzoyl)-benzoyloxy]-4-(4-methoxymethoxy-benzoylamino)-pyrrolidin-1-carbonsäure-tert-butylester, farbloser Schaum, aus (3RS,4RS)-3-Azido-4-[4-(2-fluoro-3-methoxy-6-methoxymethoxy-benzoyl)-benzoyloxy]-pyrrolidin-1-carbonsäure-tert-butylester (V3) und 4-Methoxymethoxy-benzoesäure 4-methoxymethoxy-benzoesäure

U 7 (3RS,4RS)-3-[4-(6-Methoxymethoxy-1,3-benzodioxol-5-ylcarbonyl)-benzoyloxy]-4-(4-methoxymethoxy-benzoylamino)-pyrrolidin-1-carbonsäure-tert-butylester aus (3RS,4RS)-3-Azido-4-[4-(6-methoxymethoxy-1,3-benzodioxol-5-ylcarbonyl)-benzoyloxy]-pyrrolidin-1-carbonsäure-tert-butylester (V4) und 4-Methoxymethoxy-benzoesäure

U 8 (3R,4R)-3-(4-Methoxymethoxy-benzoylamino)-4-[4-(5-methoxy-2-methoxymethoxy-benzoyl)-benzyloxy]-azepan-1-carbonsäure-tert-butylester, farbloses Oel, aus (3R,4R)-3-Azido-4-[4-(5-methoxy-2-methoxymethoxy-benzoyl)-benzyloxy]-azepan-1-carbonsäure-tert-butylester (W) und 4-methoxymethoxy-benzoesäure

U 9 (3R,4R)-3-(5-Methoxy-2-methoxymethoxy-benzoylamino)-4-[4-(5-methoxy-2-methoxymethoxy-benzoyl)-benzyloxy]-azepan-1-carbonsäure-tert-butylester, farbloses Oel, aus (3R,4R)-3-Azido-4-[4-(5-methoxy-2-methoxymethoxy-benzoyl)-benzyloxy]-azepan-1-carbonsäure-tert-butylester (W) und 5-Methoxy-2-methoxymethoxy-benzoesäure

V. Die in den Beispielen (U1) bis (U7) eingesetzten Azide wurden hergestellt durch Veresterung der Alkoholgruppe des (3RS,4RS)-3-azido-4-hydroxy-pyrrolidin-1-carbonsäure-tert-butylesters mit einer Säure der Beispiele (C)

a) unter Bedingungen der Mitsunobu-Reaktion; oder

b) unter Aktivierung durch Sulfonylchlorid oder Carbodiimide.

Die folgenden Verbindungen wurden so hergestellt:

V 1 (Methode a) (3RS,4SR)-3-Azido-4-[4-(5-methoxy-2-methoxymethoxy-benzoyl)-benzoyloxy]-pyrrolidin-1-carbonsäure-tert-butylester aus 4-(5-Methoxy-2-methoxymethoxy-benzoyl)-benzoesäure (C7)

V 2 (Methode b) (3RS,4RS)-3-Azido-4-[4-(5-methoxy-2-methoxymethoxy-benzoyl)-benzoyloxy]-pyrrolidin-1-carbonsäure-tert-butylester aus 4-(5-methoxy-2-methoxymethoxy-benzoyl)-benzoesäure (C7)

V 3 (Methode b) (3RS,4RS)-3-Azido-4-[4-(2-fluoro-3-methoxy-6-methoxymethoxy-benzoyl)-benzoyloxy]-pyrrolidin-1-carbonsäure-tert-butylester aus 4-(2-fluoro-3-methoxy-6-methoxymethoxy-benzoyl)-benzoesäure (C3).

V 4 (Methode b) (3RS,4RS)-3-Azido-4-[4-(6-methoxymethoxy-1,3-benzodioxol-5-ylcarbonyl)-benzoyloxy]-pyrrolidin-1-carbonsäure-tert-butylester aus 4-(6-Methoxymethoxy-1,3-benzodioxol-5-ylcarbonyl)-benzoesäure (C17).

W. Die in Beispiel (U8) und (U9) eingesetzte Verbindung (W) wurde aus tert-Butyl(3R,4R)-3-Azido-4-(benzyloxy)-hexahydro-1H-azepin-1-carboxylat (siehe Beispiel (S) durch Debenzylierung mit Trifluoressigsäure zu (3R,4R)-3-azido-4-hydroxy-azepan-1-carbonsäure-tert-butylester und O-Alkylierung dieser Verbindung mit (4-Bromomethyl-phenyl)-(5-methoxy-2-methoxymethoxy-phenyl)-methanon erhalten. Letztere Verbindung wurde durch Bromierung von (4-Hydroxymethyl-phenyl)-(5-methoxy-2-methoxymethoxy-phenyl)-methanon (M8) erhalten.

Die Verbindungen der Formel I und ihre pharmazeutisch anwendbaren Salze sind Proteinkinase-Hemmer; sie hemmen zelluläre Prozesse, wie Zellproliferation und -sekretion und können zur Kontrolle oder Verhütung von durch Proteinkinasen mediierten Erkrankungen verwendet werden, beispielsweise von entzündlichen Erkrankungen wie Arthritis, Immunerkrankungen, Psoriasis, Kontaktdermatitis, in Verbindung mit Organtransplantationen, wie auch in der Onkologie. Sie hemmen Zellinfektionen mit HIV (human immunodeficiency virus) oder Epstein-Bart Virus und sind daher zur Behandlung von AIDS und infektiöser Mononucleose geeignet. Die erfindungsgemässen Verbindungen hemmen weiterhin die Kontraktion der glatten Muskulatur und können daher bei cardiovasculären und bronchopulmonären Erkrankungen eingesetzt werden. Ferner sind sie bei der Asthmatherapie von Wert. Die erfindungsgemässen Verbindungen hemmen auch die Blutplättchenaggragation und können zur Kontrolle oder Verhütung von Thrombosen verwendet werden. Weiterhin hemmen sie die Freisetzung von Mediatoren aktivierter Neutrophiler und können daher zur Kontrolle ischämischer Schäden, z.B. in Herz oder Hirn verwendet werden. Ferner hemmen sie die durch erhöhte Glucosespiegel verursachte Neurotoxizität und sind daher bei der Behandlung diabetischer Komplikationen von Wert. Schliesslich stimulieren die erfindungsgemässen Verbindungen, insbesondere der 4-(2-Fluoro-6-hydroxy-3-methoxy-benzoyl)-benzoesäure (3R,4R)-3-(4-hydroxy-3,5-dimethyl-benzoylamino)-azepan-4-yl ester das Haarwachstum und können daher zur Verhütung oder Unterdrückung von Haarausfall eingesetzt werden.

In vielen Zellfunktionen spielen Proteinkinasen eine wichtige Rolle als Signaltransmitter. Neben Tyrosinkinasen sind Serin/Threoninkinasen wie Proteinkinase C (PKC) und cyclische AMP-abhängige Proteinkinase (PKA) Schlüsselenzyme in der Signalübermittlungskette von der Zellmembran zum Zellkern.

Die erfindungsgemässen Verbindungen hemmen Serin/Proteinkinasen wie PKC und PKA sowohl als isolierte Enzyme wie auch in der Zelle. Sie hemmen daher wichtige Zellfunktionen wie oben erwähnt, insbesondere die

Aktivierung und Proliferation von T-Lymphocyten und die Proliferation von Keratinocyten.

Hemmstoffe der T-Zell-Aktivierung können als Immunsuppressiva zur Anwendung bei Erkrankungen wie rheumatoider Arthritis, Psoriasis und anderen entzündlichen Hauterkrankungen (atopisches Ekzem, Kontaktekzem), bei Autoimmunerkrankungen, Transplantationen und immunmediierter Alopezie eingesetzt werden.

Hemmstoffe der Keratinocytenproliferation sind zur Anwendung bei Hauterkrankungen mit einer hyperproliferativen Komponente in der Epidermis, insbesondere Psoriasis, von Wert. Hemmstoffe der Zellproliferation können in der Onkologie Anwendung finden.

Die oben erwähnten Aktivitäten können mittels der nachstehend beschriebenen Versuchsanordnungen beobachtet werden:

A: Hemmung der Protein Kinase C (PKC) (isoliertes Enzym):

Protein Kinase C (PKC)-Aktivität wird bestimmt durch Messung des Transfers von $^{32}$P-markiertem Phosphat von $^{32}$P-γ-ATP (10 μM) auf Histon H1 (200 μg/ml) als Substrat. Als Enzymquelle wird partiell gereinigte PKC aus Schweinehirn verwendet [DEAE-Chromatographie nach der Methode von U. Kikkawa et al. (Methods Enzymol. 99, 288, 1983)]. Aktivierung der PKC erfolgt durch Phospholipidvesikel, die durch Ultraschallbehandlung von einer Mischung aus 0.05 ml Phosphatidylserin (10 mg/ml) und 0.005 ml Diolein (10 mg/ml) in 5 ml Tris-HCl-Puffer (20 mM, pH 7.4) hergestellt werden. Die Testsubstanzen werden in Dimethylsulfoxid (DMSO)/Puffer im Konzentrationsbereich 0.001 - 100 μM eingesetzt. Der Test wird durch die Zugabe von Enzym gestartet; nach 2 Minuten Inkubation bei 32°C wird die Reaktion durch Zugabe von 20%-iger Trichloressigsäure (mit 1% SDS und 1% Natrium-pyrophosphat) abgestoppt. Das präzipitierte radioaktive Histon-Protein wird durch Filtration über Nitrocellulose-Membranen von überschüssigem ATP abgetrennt und die Radioaktivität auf dem Filter im Szintillationszähler gemessen. Die Hemmwirkung der Testsubstanz wird als mikromolare Konzentration angegeben, die nötig ist, die PKC-Aktivität um 50% zu reduzieren ($IC_{50}$ [μM]).

B: Hemmung der cAMP-abhängigen Protein Kinase (PKA):

PKA-Aktivität wird bestimmt durch Messung des Transfers von $^{32}$P-markiertem Phosphat von $^{32}$P-γ-ATP (10 μM) auf Histon H1 (333 μg/ml) als Substrat. Als Enzymquelle wird partiell gereinigte PKA aus Schweinehirn verwendet [DEAE-Chromatographie nach der Methode von U. Kikkawa et al. (Methods Enzymol. 99, 288, 1983)]. Aktivierung der PKA erfolgt durch zyklisches AMP (2 μM) in Tris-HCl-Puffer (20 mM, pH 7.4). Die Testsubstanzen werden in Dimethylsulfoxid (DMSO)/Puffer im Konzentrationsbereich 0.001 - 100 μM eingesetzt. Der Test wird durch die Zugabe von Enzym gestartet; nach 2 Minuten Inkubation bei 32°C wird die Reaktion durch Zugabe von 20%-iger Trichloressigsäure (mit 1% SDS und 1% Natriumpyrophosphat) abgestoppt. Das präzipitierte radioaktive Histon-Protein wird durch Filtration über Nitrozellulose-Membranen von überschüssigem ATP abgetrennt und die Radioaktivität auf dem Filter im Szintillationszähler gemessen. Die Hemmwirkung der Testsubstanz wird als mikromolare Konzentration angegeben, die nötig ist, die PKA-Aktivität um 50% zu reduzieren ($IC_{50}$ [μM]).

C: Hemmung der Protein Kinase C (PKC) (in der Zelle):

Die intrazelluläre Protein Kinase C (PKC)-Aktivität wird in 3T3-Fibroblasten bestimmt durch Messung der Phosphorylierung eines PKC-spezifischen Substrats, dem 80 kD-MARCKS-Protein (myristoylated alanine-rich C-kinase substrate). Hierbei wird zunächst das endogene ATP durch 4-stündige Inkubation der Zellen mit 80-200 μCi/ml anorganischem $^{32}$P-markiertem Phosphat bei 37°C markiert. Dann erfolgt die Aktivierung der PKC durch Phorbolester (Tetradecanoyl-phorbol 13-azetat (TPA); 100 nM). Die Testsubstanzen werden 15 Minuten vor TPA in Dimethylsulfoxid (DMSO)/Puffer im Konzentrationsbereich 0.1 - 100 μM eingesetzt. Nach 15 Minuten Inkubation bei 37°C wird die Reaktion durch Entfernen des Mediums abgestoppt und die Zellen durch Zugabe von 1% Triton X-100 in Phosphatpuffer lysiert. Der Zellextrakt wird einer Hitzebehandlung (20 Minuten bei 90°C) unterzogen und anschliessend zentrifugiert, wobei das MARCKS-Protein gelöst im Ueberstand verbleibt. Dieser wird dann mittels diskontinuierlicher, eindimensionaler SDS-Gelelektrophorese aufgetrennt und die Radioaktivität in der 80 kD-Proteinbande mittels Phosphoimager gemessen. Die Hemmwirkung der Testsubstanz wird als mikromolare Konzentration angegeben, die nötig ist, die PKC-Aktivität im Vergleich zu Kontrollproben ohne Inhibitor um 50% zu reduzieren ($IC_{50}$ [μM]).

D: Hemmung der T-Lymphozyten-Proliferation:

Menschliche mononukleäre Zellen weden aus venösem Blut gesunder Spender isoliert und eine Suspension von 5 x $10^5$ Zellen/ml in RPMI 1640 Medium, das 10% FCS enthält, hergestellt. Die Zellen werden mit 1 μg/ml T-Zell-spezifischem Mitogen Phytohämagglutinin (PHA) stimuliert. Je 200 μl Zellsuspension werden in Mikrotiterplatten

mit den Testsubstanzen in Reihenverdünnungen im Konzentrationsbereich 0.03 - 10 μM versetzt. Die Zellproliferation wird am 3. und 4. Tag gemessen durch Inkubation mit 1 μCi [$^3$H]Thymidin während der letzten 6 Stunden des jeweiligen Tages. Die aufgenommene Radioaktivität wird mit einem Flüssigkeitsszintillationszähler gemessen.

E. Hemmung der Keratinozyten-Proliferation:

a) HaCaT-Zellen (immortalisierte humane Zellinie):

HaCaT-Zellkultur erfolgt in Medium DMEM/F12 (Mischungsverhältnis 3/1), welches Serum (FCS, 5%), epidermalen Wachstumsfaktor (EGF), Hydrocortison, Cholera-Toxin, Insulin, L-Glutamin und Penicillin/Streptomycin enthält. In Mikrotiterplatten werden 5000 Zellen in 0.2 ml Medium pro Loch eingesetzt. Die Testsubstanzen werden in Dimethylsulfoxid (DMSO)/Medium verdünnt und im Konzentrationsbereich 0.01 - 10 μM beim Start der Kultur eingesetzt. Die Zellen werden dann 48 Stunden bei 37°C inkubiert; während der letzten 6 Stunden wird radioaktives [$^3$H]Thymidin zugegeben. Nach dem Ablösen der Zellen mit Trypsin wird inkorporierte Radioaktivität im Szintillationszähler gemessen. Die Hemmwirkung der Testsubstanz wird als mikromolare Konzentration angegeben, die nötig ist, die Thymidin-Einbaurate um 50% zu reduzieren ($IC_{50}$ [μM]).

b) primäre Keratinozyten:

Keratinozyten, isoliert aus humaner Vorhaut, werden in Primärkultur bis zur Passage 7 verwendet. Die Kultur- und Testbedingungen sind identisch mit denen der HaCaT-Zellen mit Ausnahme der Verwendung von 10000 Zellen pro Loch.

Die mit typischen Verbindungen der Formel I in diesen Versuchsanordnungen erhaltenen Resultate sind in der nachstehenden Tabelle zusammengefasst:

Tabelle

| Verbindung von Beispiel | Versuchsanordnung* | | | | |
|---|---|---|---|---|---|
| | A | B | C | D | E |
| 20 | 0,011 | 0,015 | 0,34 | 0,1-6 | a:3-10 |
| 25 | 0,040 | 0,014 | 0,8 | 1 | |
| 41 | 0,040 | 0,013 | >1 | 0,3 | |
| 57 | 0,867 | 0,005 | 2,0 | | a: 0,3-0,6<br>b: 0,6 |
| 60 | 1,2 | 0,009 | 2,7 | 2-5 | a: 0,3-0,5<br>b: 0,3-6 |

* die Versuchsdaten geben die jeweilige $IC_{50}$ [μM] an

Die Verbindungen der Formel I und deren Salze können als Heilmittel, z.B. in Form pharmazeutischer Präparate, Anwendung finden.

Die Mittel können enteral, parenteral oder topisch verabreicht werden. Für die enterale Applikation eignen sich z.B. Mittel in Form von Tabletten, Kapseln, Dragées, Sirupen, Suspensionen, Lösungen und Suppositorien. Für die parenterale Applikation sind Mittel in Form von Infúsions- oder Injektionslösungen geeignet.

Die Dosierungen, in denen die Präparate verabreicht werden, können je nach Anwendungsart und Anwendungsweg sowie nach den Bedürfnissen der Patienten variieren.

Bei oraler Verabreichung der erfindungsgemässen Verbindungen kommen beim Erwachsenen Dosierungen von etwa 0,1-100 mg/kg, vorzugsweise 0,5-50 mg/kg pro Tag in Betracht.

Die Präparate können in einer oder mehreren Dosierungen verabreicht werden. Eine bevorzugte Darreichungsform sind Kapseln mit einem Gehalt von ca. 5-500 mg Wirkstoff.

Die Präparate können inerte oder auch pharmakodynamisch aktive Zusätze enthalten. Tabletten oder Granulate z.B. können eine Reihe von Bindemitteln, Füllstoffen, Trägersubstanzen oder Verdünnungsmitteln enthalten. Flüssige Präparate können beispielsweise in Form einer sterilen, mit Wasser mischbaren Lösung vorliegen. Kapseln

können neben dem Wirkstoff zusätzlich ein Füllmaterial oder Verdickungsmittel enthalten. Des weiteren können geschmacksverbessernde Zusätze, sowie die üblicherweise als Konservierungs-, Stabilisierungs-, Feuchthalte- und Emulgiermittel verwendeten Stoffe, ferner auch Salze zur Veränderung des osmotischen Druckes, Puffer und andere Zusätze vorhanden sein.

Die vorstehend erwähnten Trägersubstanzen und Verdünnungsmittel können aus organischen oder anorganischen Stoffen, z.B. aus Wasser, Gelatine, Milchzucker, Stärke, Magnesiumstearat, Talkum, Gummi arabicum, Polyalkylenglykolen und dergleichen bestehen. Voraussetzung ist, dass alle bei der Herstellung der Präparate verwendeten Hilfsstoffe untoxisch sind.

Zur topischen Anwendung werden die Wirkstoffe zweckmässig in Form von Salben, Tinkturen, Crèmen, Lösungen, Lotionen, Sprays, Suspensionen und dergleichen verwendet. Bevorzugt sind Salben und Crèmen sowie Lösungen. Diese zur topischen Anwendung bestimmten Präparate können dadurch hergestellt werden, dass man die Verfahrensprodukte als wirksamen Bestandteil nichttoxischen, inerten, für topische Behandlung geeigneten, an sich in solchen Präparaten üblichen festen oder flüssigen Trägern zumischt.

Für die topische Anwendung sind zweckmässig ca. 0,1-5%ige, vorzugsweise 0,3-2%ige Lösungen, sowie ca. 0,1-5%ige, vorzugsweise ca. 0,3-2%ige Salben und Crèmen geeignet.

Den Präparaten kann gegebenenfalls ein Antioxydationsmittel, z.B. Tocopherol, N-Methyl-γ-tocopheramin sowie t-Butyl-Hydroxyanisol oder t-Butyl-Hydroxytoluol beigemischt sein.

Die nachstehenden Beispiele erläutern die Erfindung weiter.

Beispiel 1

639.7mg (3R,4R)-4-[4-(2-Fluor-3-methoxy-6-methoxymethoxy-benzoyl)-benzoyloxy]-3-(4-methoxymethoxy-benzoylamino)-azepan-1-carbonsäure-tert-butylester wurde in 9 ml Dimethoxyethan und 9 ml Isopropanol gelöst und nach Kühlen auf 0°C langsam mit HCl-Gas gesättigt und 24 Stunden im Kühlschrank aufbewahrt. Das Lösungsmittel wurde vollständig unter vermindertem Druck entfernt und das Produkt mit Diethylether verrieben. Die erhaltene Suspension wurde mehrere Stunden gerührt; das Lösungsmittel dekantiert und das Rühren nach Zusatz eines weiteren Anteils von frischem Lösungsmittel über Nacht fortgesetzt. Das Produkt wurde abfiltriert, mehrmals mit Diethylether gewaschen und bei 0.1 mbar und 50°C getrocknet. Man erhielt 479 mg 4-(2-Fluoro-6-hydroxy-3-methoxy-benzoyl)-benzoesäure (3R,4R)-3-(4-hydroxy-benzoylamino)azepan-4-yl ester hydrochlorid (1:1) als gelbes Pulver.

MS: m/e= 523.1 $(M+H)^+$
IR (KBr): 1720, 1638, 1607 $cm^{-1}$

In analoger Weise wurden die Verbindungen der Beispiele 2 - 76 erhalten :

Beispiel 2

4-(2-Fluor-6-hydroxy-3-methoxy-benzoyl)-benzoesäure (3R,4R)-3-(4-hydroxy-3-methoxy-benzoylamino)-azepan-4-yl ester hydrochlorid (1:1) als gelbes Pulver aus (3R,4R)-4-[4-(2-Fluor-3-methoxy-6-methoxymethoxy-benzoyl)benzoyloxy]-3-(3-methoxy-4-methoxymethoxy-benzoylamino)-azepan-1-carbonsäure-tert-butylester

MS: m/e= 553.3 $(M+H)^+$
IR (KBr): 1721, 1641, 1600 $cm^{-1}$

Beispiel 3

4-(2-Fluor-6-hydroxy-3-methoxy-benzoyl)-benzoesäure (3R,4R)-3-(4-hydroxy-3,5-dimethoxy-benzoylamino)-azepan-4-yl ester hydrochlorid (1:1) als gelbes Pulver aus (3R,4R)-4-[4-(2-Fluoro-3-methoxy-6-methoxymethoxy-benzoyl)-benzoyloxy]-3-(3,5-dimethoxy-4-methoxymethoxy-benzoylamino)-azepan-1-carbonsäure-tert-butylester

MS: m/e= 583.2 $(M+H)^+$
IR (KBr): 1720, 1642, 1605 $cm^{-1}$

Beispiel 4

4-(2-Fluoro-6-hydroxy-3-methoxy-benzoyl)-benzoesäure (3R,4R)-3-(3,5-dihydroxy-naphthalen-2-ylcarbonylamino)-azepan-4-yl ester hydrochlorid (1:1) als gelbes Pulver aus (3R,4R)-4-[4-(2-Fluoro-3-methoxy-6-methoxymethoxy-benzoyl)-benzoyloxy]-3-(3,5-bis-methoxymethoxy-naphthalin-2-ylcarbonylamino)-azepan-1-carbonsäure-tert-butylester

MS: 589.5 $(M+H)^+$
IR: 3392, 3269, 1722, 1655, 1622, 1573, 1530, 1501 $cm^{-1}$

Beispiel 5

4-(3-Hydroxy-naphtalin-2-ylcarbonyl)-benzoesäure (3R,4R)-3-(4-hydroxy-3,5-dimethoxy-benzoylamino)-azepan-4-yl ester hydrochlorid(1:1) als gelbes Pulver aus (3R,4R)-3-(3,5-Dimethoxy-4-methoxymethoxy-benzoyl-amino)-4-[4-(3-methoxymethoxy-naphthalin-2-ylcarbonyl)-benzoyloxy]-azepan-1-carbonsäure-tert-butylester

MS: 585,3 $(M+H)^+$
IR: 3413, 2778, 1721, 1642, 1599, 1540, 1505 $cm^{-1}$

Beispiel 6

4-(2-Hydroxy-5-methoxy-benzoyl)-benzoesäure (3R,4R)-3-(3,5-dimethoxy-benzoylamino)-azepan-4-yl ester hydrochlorid (1:1) als gelbes Pulver aus (3R,4R)-3-(3,5-Dimethoxy-benzoylamino)-4-[4-(5-methoxy-2-methoxymethoxy-benzoyl)-benzoyloxy]-azepan-1-carbonsäure-tert-butylester

MS:549.4$(M+H)^+$
IR: 3421, 1721 1636, 1594, 1273, 1042, 838$cm^{-1}$

Beispiel 7

4-(2-Fluoro-6-hydroxy-3-methoxy-benzoyl)-benzoesäure (3R,4R)-3-(2-hydroxy-3-methoxy-benzoylamino)-azepan-4-yl ester hydrochlorid (1:1) als gelbes Pulver aus (3R,4R)-4-[4-(2-Fluoro-3-methoxy-6-methoxymethoxy-benzoyl)-benzoyloxy]-3-(3-methoxy-2-methoxymethoxy-benzoylamino)-azepan-1-carbonsäure-tert-butylester

MS: m/e= 553.4 $(M+H)^+$
IR (KBr): 1722, 1642, 1586 $cm^{-1}$

Beispiel 8

3-(2-Fluoro-6-hydroxy-3-methoxy-benzoyl)-benzoesäure (3R,4R)-3-((3-hydroxy-naphtalin-2-ylcarbonyl-amino)-azepan-4-yl ester hydrochlorid (1:1) als gelbes Pulver aus (3R,4R)-4-[4-(2-Fluoro-3-methoxy-6-methoxymethoxy-benzoyl)-benzoyloxy]-3-(3-methoxymethoxy-naphthalin-2-ylcarbonylamino)- azepan-1-carbonsäure-tert-butylester

MS
IR

Beispiel 9

3-(3-Hydroxy-5,6,7,8-tetrahydro-naphtalin-2-ylcarbonyl)-benzoylamino)-azepan-4-yl ester hydrochlorid (1:1) als gelbes Pulver aus (3R,4R)-3-(4-Methoxymethoxy-benzoylamino)-4-[4-(3-methoxymethoxy-5,6,7,8-tetrahydro-naphthalin-2-ylcarbonyl)-benzoyloxy]-azepan-1-carbonsäure-tert-butylester

MS
IR

Beispiel 10

4-(2-Fluoro-6-hydroxy-3-methoxy-benzoyl)-benzoesäure (3R,4R)-3-(3,5-dimethoxy-benzoylamino)-azepan-4-yl ester hydrochlorid (1:1) als gelbes Pulver aus (3R,4R)-4-[4-(2-Fluoro-3-methoxy-6-methoxymethoxy-benzoyl)-benzoyloxy]-3-(3,5-dimethoxy-benzoylamino)-azepan-1-carbonsäure-tert-butylester

MS:565.3$(M-H)^-$
IR: 3405, 1722 1644, 1593, 1275, 1157, 1018, 821$cm^{-1}$

Beispiel 11

4-(2-Fluoro-6-hydroxy-3-methoxy-benzoyl)-benzoesäure (3R,4R)-3-(4-hydroxy-2,3,6-trimethyl-benzoylamino)-azepan-4-yl ester hydrochlorid (1:1) als gelbes Pulver aus (3R,4R)-4-[4-(2-Fluoro-3-methoxy-6-methoxymethoxy-benzoyl)-benzoyloxy]-3-(4-methoxymethoxy-2,3,6-trimethyl-benzoylamino)-azepan1-carbonsäure-tert-butylester

MS: m/e= 565 $(M+H)^+$
IR (KBr): 1722, 1639 $cm^{-1}$

Beispiel 12

4-(2,3-Difluoro-6-hydroxy-benzoyl)benzoesäure (3R,4R)-3-(4-hydroxy-2,3,6-trimethyl-benzoylamino)-azepan-4-yl ester hydrochlorid (1:1) als gelbes Pulver aus (3R,4R)-4-[4-(2,3-Difluoro-6-methoxymethoxy-benzoyl)-benzoyloxy]-3-(4-methoxymethoxy-2,3,6-trimethyl-bensoylamino)-azepan-1- carbonsäure-tert-butylester

MS: m/e= 553 $(M+H)^+$
IR (KBr): 1723, 1632 $cm^{-1}$

Beispiel 13

4-(2,3-Difluoro-6-hydroxy-benzoyl)-benzoesäure (3R,4R)-3-(4-hydroxy-3,5-diisopropyl-benzoylamino)-azepan-4-yl ester hydrochlorid (1:1) als gelbes Pulver aus (3R,4R)-4-[4-(2,3-Difluoro-6-methoxymethoxy-benzoyl)-benzoyloxy]-3-(3,5-diisopropyl-4-methoxymethoxy-benzoylamino)-azepan-1-carbonsäure-tert-butylester

MS: m/e= 595.5 $(M+H)^+$
IR (KBr): 1719, 1632, 1602 $cm^{-1}$

Beispiel 14

4-(2-Hydroxy-5-methoxy-benzoyl)-benzoesäure (3R,4R)-3-(3,4,5-trimethoxy-benzoylamino)-azepan-4-yl ester hydrochlorid (1:1) als gelbes Pulver aus (3R,4R)-4-[4-(5-Methoxy-2-methoxymethoxy-benzoyl)-benzoyloxy]-3-(3,4,5-trimethoxy-benzoylamino)-azepan-1-carbonsäure-tert-butylester

MS:579.4$(M+H)^+$, 307.3
IR: 3425, 1717 1635, 1585, 1500, 1276, 1126, 837$cm^{-1}$

Beispiel 15

4-(2-Hydroxy-5-dimethylamino-benzoyl)-benzoesäure (3R,4R)-3-(4-hydroxy-benzoylamino)-azepan-4-yl ester hydrochlorid (1:2) als lachsrosa Pulver aus (3R,4R)-4-[4-(5-Dimethylamino-2-methoxymethoxy-benzoyl)-benzoyloxy]-3-(4-methoxymethoxy-benzoylamino)-azepan-1-carbonsäure-butylester

MS: m/e= 518.5 $(M+H)^+$
IR (KBr): 1719, 1639, 1608 $cm^{-1}$

Beispiel 16

4-(2-Hydroxy-5-dimethylamino-benzoyl)-benzoesäure (3R,4R)-3-(4-hydroxy-3-methoxy-benzoylamino)-azepan-4-yl ester hydrochlorid (1:2) als lachsrosa Pulver aus (3R,4R)-3-(3-Methoxy-4-methoxymethoxy-benzoylamino)-4-[4[-(2-methoxymethoxy-5-dimethylamino-benzoyl)-benzoyloxy]- azepan-1-carbonsäure-tert-butylester

MS: m/e= 548.4 $(M+H)^+$
IR (KBr): 1717, 1638, 1605 $cm^{-1}$

Beispiel 17

4-(2-Hydroxy-5-dimethylamino-benzoyl)-benzoesäure (3R,4R)-3-(4-hydroxy-3,5-diisopropyl-benzoylamino)-azepan-4-yl ester hydrochlorid (1:2) als lachsrosa Pulver aus (3R,4R)-3-(3,5-Diisopropyl-4-methoxymethoxy-benzoylamino)-4-[4-(2-methoxymethoxy-5-dimethylamino-benzoyl)-benzoyloxy]-azepan-1-carbonsäure-tert-butylester

MS: m/e= 602.6 $(M+H)^+$
IR (KBr): 1720, 1637, 1606 $cm^{-1}$

Beispiel 18

4-(7-Hydroxy-1,4-benzodioxin-6-ylcarbonyl)-benzoesäure (3R,4R)-3-(4-hydroxy-benzoylamino)-azepan-4-yl ester hydrochlorid (1:1) als gelbes Pulver aus (3R,4R)-4-[4-(2,3-Dihydro-1,4-benzodioxin-6-ylcarbonyl)-benzoyloxy]-3-(4-methoxymethoxy-benzoylamino)-azepan-1-carbonsäure-tert-butylester

MS: 533.4 $(M+H)^+$
IR: 3417, 3275, 1720, 1639, 1608, 1539, 1502 $cm^{-1}$

Beispiel 19

4-[2-Hydroxy-5-(1-methoxy-ethyl)-benzoyl]-benzoesäure (3R,4R)-3-(4-hydroxy-benzoylamino)-azepan-4-yl ester hydrochlorid (1:1) als hellgelbes Pulver aus (3R,4R)-4-[4-[5-(1-Methoxyethyl)-2-methoxymethoxy-benzoyl]-benzoyloxy-3-(4-methoxymethoxy-benzoylamino)-azepan-1-carbonsäure-tert-butylester

MS: 533.4 $(M+H)^+$
IR: 3424m 1680, 1565, 1506 $cm^{-1}$

Beispiel 20

4-(2-Fluoro-6-hydroxy-3-methoxy-benzoyl)-benzoesäure (3R,4R)-3-(4-hydroxy-3,5-dimethyl-benzoylamino)-azepan-4-yl ester hydrochlorid (1:1) als gelbes Pulver aus (3R,4R)-4-[4-(2-Fluoro-3-methoxy-6-methoxymethoxy-benzoyl)-benzoyloxy]-3-(4-methoxymethoxy-3,5-dimethyl-benzoylamino)- azepan-1-carbonsäure-tert-butylester

MS: m/e= 549.5 $(M+H)^+$
IR (KBr): 1722, 1642, 1605 $cm^{-1}$

Beispiel 21

4-(2-Hydroxy-5-dimethylamino-benzoyl)-benzoesäure (3R,4R)-3-(4-hydroxy-3,5-dimethyl-benzoylamino)-azepan-4-yl ester hydrochlorid (1:2) als lachsrosa Pulver aus (3R,4R)-4-[4-(2-Methoxymethoxy-5-dimethylamino-benzoyl)-benzoyloxy-3-(4-methoxymethoxy-3,5-dimethyl-benzoylamino)-azepan-1-carbonsäure-tert-butylester

MS: m/e= 544.5 $(M-H)^-$
IR (KBr): 1720, 1638, 1605 $cm^{-1}$

Beispiel 22

4-(2-Fluoro-6-hydroxy-4-methoxy-benzoyl)-benzoesäure (3R,4R)-3-(4-hydroxy-3,5-diisopropyl-benzoylamino)-azepan-4-yl ester hydrochlorid (1:1) als farbloses Pulver aus 3R,4R)-4-[4-(2-Fluoro-4-methoxy-6-methoxymethoxy-benzoyl)-benzoyloxy]-3-(3,5-diisopropyl-4-methoxymethoxy-benzoylamino)-azepan-1-carbonsäure-tert-butylester

MS: m/e= 605.4 $(M-H)^-$
IR (KBr): 1722, 1639, 1600 $cm^{-1}$

Beispiel 23

4-(2-Hydroxy-5-methoxy-benzoyl)-benzoesäure (3R,4R)-3-(3-acetyl-4-hydroxy-benzoylamino)-azepan-4-yl

ester hydrochlorid (1:1) als gelbes Pulver aus (3R,4R)-3-(3-Acetyl-4-methoxymethoxy-benzoylamino)-4-[4-(5-methoxy-2-methoxymethoxy-benzoyl)-benzoyloxy]-azepan-1-carbonsäure-tert-butylester

MS:547.7$(M+H)^+$
IR: 3429, 1717 1642, 1608, 1533, 1484, 1282 ,1040, 826$cm^{-1}$

Beispiel 24

4-(6-Hydroxy-chinolin-7-ylcarbonyl)-benzoesäure (3R,4R)-3-(4-hydroxy-benzoylamino)-azepan-4-yl ester hydrochlorid (1:1) als gelbes Pulver aus (3R,4R)-3-(4-Methoxymethoxy-benzoylamino)-4-[4-(6-methoxymethoxy-chinolin-7-ylcarbonyl)-benzoyloxy]-azepan-1-carbonsäure-tert-butylester

MS: m/e = 539.4 $(M+H)^+$
IR (KBr): 1720, 1634, 1605 $cm^{-1}$

Beispiel 25

4-(2,3-Difluoro-6-hydroxy-benzoyl)-benzoesäure (3R,4R) -3-(4-hydroxy-3,5-dlmethyl-benzoylamino)-azepan-4-yl ester hydrochlorid (1:1) als hellgelbes Pulver aus (3R,4R)-4-[4-(2,3-Difluoro-6-methoxymethoxy-benzoyl)-benzoyloxy]-3-(4-methoxymethoxy-3,5-dimethyl-benzoylamino)-azepan-1-carbonsäure-tert-butylester

MS: m/e= 539.4 $(M+H)^+$
IR (KBr): 1720, 1634, 1605 $cm^{-1}$

Beispiel 26

4-(2,4-Difluoro-6-hydroxy-benzoyl)-benzoesäure (3R,4R) -3-(4-hydroxy-3,5-dimethyl-benzoylamino)-azepan-4-yl ester hydrochlorid (1:1) als farbloses Pulver aus (3R,4R)-4-[4-(2,4-Difluoro-6-methoxymethoxy-benzoyl)-benzoyloxy]-3-(4-methoxymethoxy-3,5-dimethyl-benzoylamino)-azepan-1-carbonsäure-tert-butylester

MS: m/e= 539.5 $(M+H)^+$
IR (KBr): 1718, 1635, 1608 $cm^{-1}$

Beispiel 27

4-(2-Hydroxy-5-methoxy-benzoyl)-benzoesäure (3R,4R)-3-(3,5-diethoxy-benzoylamino)-azepan-4-yl ester hydrochlorid (1:1) als gelbes Pulver aus (3R,4R)-3-(3,5-diethoxy-benzoylamino)-4-[4-(5-Methoxy-2-methoxymethoxy-benzoyl)-benzoyloxy]-azepan-1-carbonsäure-tert-butylester

MS:577.4$(M+H)^+$, 305.7,193.5
IR: 3404, 1721, 1636, 1593, 1531, 1484, 1273, 1058, 831$cm^{-1}$

Beispiel 28

4-(4-Hydroxy-biphenyl-3-ylcarbonyl)-benzoesäure (3R, 4R)-3-(4-hydroxy-benzoylamino)-azepan-4-yl ester hydrochlorid (1:1) als gelbes Pulver aus (3R,4R)-3-(4-Methoxymethoxy-benzoylamino)-4-[4-(4-methoxymethoxy-biphenyl-3-ylcarbonyl)-benzoyloxy]-azepan-1-carbonsäure-tert-butylester

MS: 551.3 $(M+H)^+$
IR: 3255, 2796, 1722, 1631, 1607, 1539, 1505 $cm^{-1}$

Beispiel 29

4-(6-Hydroxy-chinolin-7-ylcarbonyl)-benzoesäure (3R,4R) -3-(4-hydroxy-3,5-dlmethyl-benzoylamino)-azepan-4-yl ester hydrochlorid (1:1) als gelbes Pulver aus (3R,4R)-3-(4-Methoxymethoxy-3,5-dimethyl-benzoylamino)-4-[4-(6-methoxymethoxy-chinolin-7-ylcarbonyl)-benzoyloxy]-azepan-1-carbonsäure-tert-butylester

MS: 554.5 $(M+H)^+$

IR: 3411, 1720, 1639, 1606, 1536, 1485 $cm^{-1}$

Beispiel 30

4-(5-Acetyl-2-hydroxy-benzoyl)-benzoesäure (3R, 4R)-3-(4-hydroxy-benzoylamino)-azepan-4-yl ester hydrochlorid (1:1) als gelbes Pulver aus (3R,4R)-4-[4-(5-Acetyl-2-methoxymethoxy-benzoyl)-benzoyloxy]-3-(4-methoxymethoxy-benzoylamino)-azepan-1-carbonsäure-tert-butylester

MS: 517.3 $(M+H)^+$
IR: 3410, 1722, 1628, 1606 $cm^{-1}$

Beispiel 31

4-(2-Hydroxy-10-methyl-phenothiazin-1-ylcarbonyl)-benzoesäure (3R, 4R)-3-(4-hydroxy-benzoylamino)-azepan-4-yl ester hydrochlorid (1:2) als oranges Pulver aus (3R, 4R)-3-(4-Methoxymethoxy)-benzoylamino)-4-[4-(3-methoxymethoxy-10-methyl-phenothiazin-2-ylcarbonyl)-benzoyl]-azepan-1-carbonsäure-tert-butylester

MS : 610.4 $(M+H)^+$
IR : 3376, 2669, 1723, 1612, 1540, 1507, 1268, 849, 822, 755.

Beispiel 32

4-(2-Fluoro-6-hydroxy-3-methoxy-benzoyl)-benzoesäure (3R,4R)-3-(3,5-diethyl-4-hydroxy-benzoylamino)-azepan-4-yl ester hydrochlorid (1:1) als gelbes Pulver aus (3R,4R)-3-(3,5-Diethyl-4-methoxymethoxy-benzoylamino)-4-[2-fluoro-3-methoxy-6-methoxymethoxy-benzoyl)-benzoyloxy]azepan-1- carbonsäure-tert-butylester

MS: m/e= 579.5 $(M+H)^+$
IR (KBr): 1722, 1639, 1603 $cm^{-1}$

Beispiel 33

4-(2,3-Difluoro-6-hydroxy-benzoyl)-benzoesäure (3R,4R)-3-(3,5-diethyl-4-hydroxy-benzoylamino)-azepan-4-yl ester hydrochlorid (1:1) als hellgelbes Pulver aus (3R,4R)-3-(3,5-Diethyl-4-methoxymethoxy-benzoylamino)-4-[4-(2,3-difluoro-6-methoxymethoxy-benzoyl)-benzoyloxy]-azepan-1-carbonsäure-tert-butylester

MS: m/e= 567.4 $(M+H)^+$
IR (KBr): 1722, 1634, 1604 $cm^{-1}$

Beispiel 34

4-(2-Hydroxy-5-dimethylamino-benzoyl)-benzoesäure (3R,4R)-3-(3,5-diethyl-4-hydroxy-benzoylamino)-azepan-4-yl ester hydrochlorid (1:2) als lachsrosa Pulver aus (3R,4R)-3-(3,5-Diethyl-4-methoxymethoxy-benzoyl-amino)-4-[4-(2-methoxymethoxy-5-dimethylamino-benzoyl)benzoyloxy]-azepan-1-carbonsäure-tert-butylester

MS: m/e= 574.5 $(M+H)^+$
IR (KBr): 1720, 1638, 1605 $cm^{-1}$

Beispiel 35

4-(2,4-Difluoro-6-hydroxy-benzoyl)-benzoesäure (3R,4R)-3-(3,5-diethyl-4-hydroxy-benzoylamino)-azepan-4-yl ester hydrochlorid (1:1) als farbloses Pulver aus (3R,4R)-3-(3,5-Diethyl-4-methoxymethoxy-benzoylamino)-4-[4-(2,4-difluoro-6-methoxymethoxy-benzoyl)-benzoyloxy-azepan-1-carbonsäure-tert-butylester

MS: m/e= 567.4 $(M+H)^+$
IR (KBr): 1722, 1636, 1603 $cm^{-1}$

Beispiel 36

4-(2-Hydroxy-5-methoxy-benzoyl)-benzoesäure (3R,4R) -3-(4-hydroxy-3,5-dimethyl-benzoylamino)-azepan-4-yl ester hydrochlorid (1:1) als gelbes Pulver aus (3R,4R)-4-[4-(5-Methoxy-2-methoxymethoxy-benzoyl)-benzoyloxy]-3-(4-methoxymethoxy-3,5-dimethyl-benzoylamino)-azepan-1-carbonsäure-tert-butylester

MS: 531.2 $(M+H)^+$
IR: 3405, 1721, 1636, 1606, 1532, 1485 $cm^{-1}$

Beispiel 37

4-(2-Hydroxy-5-methoxy-benzoyl)-benzoesäure (3R,4R)-3-(4-pyridinoylamino)-azepan-4-yl ester hydrochlorid (1:2) als gelbes Pulver aus (3R,4R)-4-[4-(5-Methoxy-2-methoxymethoxy-benzoyl)-benzoyloxy]-3-(4-pyridinoylamino)-azepan-1-carbonsäure-tert-butylester

MS:490.4$(M+H)^+$
IR: 3428, 1723, 1673, 1636, 1606, 1548, 1483, 1269, 831$cm^{-1}$

Beispiel 38

4-(2-Hydroxy-5-methyl-benzoyl)-benzoesäure (3R, 4R)-3-(4-hydroxy-benzoylamino)-azepan-4-yl ester hydrochlorid (1:1) als gelbes Pulver aus (3R,4R)-3-(4-Methoxymethoxy-benzoylamino)-4-[4-(2-methoxymethoxy-5-methyl-benzoyl)-benzoyloxy]-azepan-1-carbonsäure-tert-butylester

MS : 489.2 $(M+H)^+$.
R : 3258, 1721, 1832, 1608, 1540, 1505, 1275, 851.

Beispiel 39

4-(6-Hydroxy-1,3-benzodioxol-5-ylcarbonyl)-benzoesäure (3R,4R) -3-(4-hydroxy-3,5-dimethyl-benzoylamino)-azepan-4-yl ester hydrochlorid (1:1) als gelbes Pulver aus (3R,4R)-4-[4-(6-Methoxymethoxy-1,3-benzodioxol-5-ylcarbonyl)-benzoyloxy]-3-(4-methoxymethoxy-3,5-dimethyl-benzoylamino)-azepan-1-carbonsäure-tert-butylester

MS: 547.4 $(M+H)^+$
IR: 3424, 2771, 1720, 1627, 1603, 1532 $cm^{-1}$

Beispiel 40

4-(2-Hydroxy-5-isopropyl-benzoyl)-benzoesäure (3R, 4R)-3-(4-hydroxy-benzoylamino)-azepan-4-yl ester hydrochlorid (1:1) als gelbes Pulver aus (3R,4R)-4-[4-(5-Isopropyl-2-methoxymethoxy-benzoyl)-benzoyloxy]-3-(4-methoxymethoxy-benzoylamino)-azepan-1-carbonsäure-tert-butylester

MS : 517.2 $(M+H)^+$.
IR: 3398, 1723, 1831, 1607, 1539, 1505, 1276, 840.
Anal. calc. for $C_{30}H_{32}N_2O_6$.HCl.$H_2O$ (Wa 2.33) (553.055): C 65.15, H 6.01, N 5.07; found: C 65.34, H 6.08, N 4.92.

Beispiel 41

4-(6-Hydroxy-1,4-benzodioxin-1-yl-carbonyl)-benzoesäure (3R, 4R)-3-(4-hydroxy-3,5-dimethyl-benzoylamino)-azepan-4-yl ester hydrochlorid (1:1) als gelbes Pulver aus (3R, 4R)-4[4-(6-Methoxymethoxy-(1,4-benzodioxin-5-ylcarbonyl)-benzoyloxy]-3-(4-methoxymethoxy-3,5-dimethyl-benzoylamino) azepan-1-carbonsäure-tert-butylester

MS : 561.4 $(M+H)^+$.
IR : 3421, 1720, 1632, 1606, 1740, 1268, 825.

Beispiel 42

4-(2-Fluoro-3-dimethylamino-6-hydroxy-benzoyl)-benzoesäure (3R,4R)-3-(4-hydroxy-benzoylamino)-azepan-4-yl ester hydrochlorid (1:2) als farbloses Pulver aus (3R,4R)-4-[4-(2-Fluoro-3-dimethylamino-6-methoxymethoxy-benzoyl)-benzoyloxy]-3-(4-methoxymethoxy-benzoylamino)-azepan-1-carbonsäure-tert-butylester

MS: m/e= 536.4 $(M+H)^+$
IR (KBr): 1721, 1631, 1607 $cm^{-1}$

Beispiel 43

4-(2-Fluoro-6-hydroxy-3-dimethylamino-benzoyl)-benzoesäure (3R,4R)-3-(4-hydroxy-3,5-dimethyl-benzoylamino)-azepan-4-yl ester hydrochlorid (1:2) als gelbes Pulver aus (3R,4R)-4-[4-(2-Fluoro-6-methoxymethoxy-3-dimethylamino-benzoyl)-benzoyloxy]-3-(4-methoxymethoxy-3,5-dimethyl-benzoylamino)-azepan-1-carbonsäure-tert-butylester

MS: m/e= 564.4 $(M+H)^+$
IR (KBr): 1721, 1631, 1604 $cm^{-1}$

Beispiel 44

4-(2-Fluoro-6-hydroxy-3-dimethylamino-benzoyl)-benzoesäure (3R,4R)-3-(4-hydroxy-3,5-dimethoxy-benzoylamino)-azepan-4-yl ester hydrochlorid (1:2) als farbloses Pulver aus (3R,4R)-4-[4-(2-Fluoro-6-methoxymethoxy-3-dimethylamino-benzoyl)-benzoyloxy]-3-(3,5-dimethoxy-4-methoxymethoxy-benzoylamino)-azepan-1-carbonsäure-tert-butylester

MS: m/e= 596.4 $(M+H)^+$
IR (KBr): 1721, 1631, 1603 $cm^{-1}$

Beispiel 45

4-(5-Acetyl-2-hydroxy-benzoyl)-benzoesäure (3R,4R)-3-(4-hydroxy-3,5-dimethoxy-benzoylamino)-azepan-4-yl ester hydrochlorid (1:1) als gelbes Pulver aus (3R,4R)-4-[4-(5-Acetyl-2-methoxymethoxy-benzoyl)-benzoyloxy]-3-(4-methoxymethoxy-3,5-dimethyl-benzoylamino)-azepan-1-carbonsäure-tert-butylester

MS: 545 $(M+H)^+$
IR: 3399, 1721, 1673, 1628, 1601 $cm^{-1}$

Beispiel 46

4-(2-Fluoro-6-hydroxy-3-methoxy-benzoyl)-benzoesäure (3R,4R)-3-(4-pyridinoylamino)-azepan-4-yl ester hydrochlorid (1:2) als gelbes Pulver aus (3R,4R)-4-[4-(2-Fluoro-3-methoxy-6-methoxymethoxy-benzoyl)-benzoyloxy]-3-(4-pyridinoylamino)-azepan-1-carbonsäure-tert-butylester

MS:508.3$(M+H)^+$
IR: 3429, 1724, 1672, 1640, 1608, 1550, 1499, 1276, 840$cm^{-1}$

Beispiel 47

4-(2-Hydroxy-5-methoxy-benzoyl)-benzoesäure (3R,4R)-3-(3-tert-butyl-4-hydroxy-benzoylamino)-azepan-4-yl ester hydrochlorid (1:1) als gelbes Pulver aus (3R,4R)-3-(3-tert-Butyl-4-hydroxy-benzoylamino)-4-[4-(5-methoxy-2-methoxymethoxy-benzoyl)-benzoyloxy]-azepan-1-carbonsäure-tert-butylester

MS:561.4$(M+H)^+$
IR: 3426, 1740, 1635, 1604, 1537, 1485, 1261$cm^{-1}$

Beispiel 48

4-(2-Fluoro-6-hydroxy-3-methoxy-benzoyl)-benzoesäure (3R,4R)-3-(3-Bromo-4-hydroxy-benzoylamino)-azepan-4-yl ester hydrochlorid (1:1) als gelbes Pulver aus (3R,4R)-3-(3-bromo-4-methoxymethoxy-benzoylamino)-4-[4-(2-fluoro-3-methoxy-6-methoxymethoxy-benzoyl)-benzoyloxy]-azepan-1-carbonsäure-tert-butylester

MS: 599.2 $(M-H)^-$
IR: 3416, 1722, 1642, 1601 $cm^{-1}$

Beispiel 49

4-(2-Hydroxy-5-methoxy-benzoyl)-benzoesäure (3R,4R)-3-(4-hydroxy-3-isopropyl-benzoylamino)-azepan-4-yl ester hydrochlorid (1:1) als gelbes Pulver aus (3R,4R)-3-(3-Isopropyl-4-methoxymethoxy-benzoylamino)-4-[4-(5-methoxy-2-methoxymethoxy-benzoyl)-benzoyloxy]-azepan-1-carbonsäure-tert-butylester

MS:547.6$(M+H)^+$
IR: 3421, 1721, 1635, 1604, 1537, 1485, 1277, 832$cm^{-1}$

Beispiel 50

4-(2-Hydroxy-5-methoxy-benzoyl)-benzoesäure (3R,4R)-3-(4-hydroxy-3-sec-butyl-benzoylamino)-azepan-4-yl ester hydrochlorid (1:1) als gelbes Pulver aus (3R,4R)-3-(3-sec-Butyl-4-methoxymethoxy-benzoylamino)-4-[4-(5-methoxy-2-methoxymethoxy-benzoyl)-benzoyloxy]-azepan-1-carbonsäure-tert-butylester

MS:561.6$(M+H)^+$
IR: 3393, 1719, 1636, 1604, 1538, 1485, 1278, 833$cm^{-1}$

Beispiel 51

4-(2-Fluoro-3-isopropyl-6-hydroxy-benzoyl)-benzoesäure (3R,4R)-3-(4-hydroxy-3,5-dimethyl-benzoylamino)-azepan-4-yl ester hydrochlorid (1:1) als gelbes Pulver aus (3R,4R)-4-[4-(2-Fluoro-3-isopropyl-6-methoxymethoxy-benzoyl)-benzoyloxy]-3-(4-methoxymethoxy-3,5-dimethyl-benzoylamino)-azepan-1-carbonsäure-tert-butylester

MS: 563.4 $(M+H)^+$
IR: 3412, 1722, 1638, 1533 $cm^{-1}$

Beispiel 52

4-(2-Fluoro-6-hydroxy-3-pyrrolidin-1-yl-benzoyl)-benzoesäure (3R,4R)-3-(4-hydroxy-benzoylamino)-azepan-4-yl ester hydrochlorid (1:2) als lachsrosa Pulver aus (3R,4R)-4-[4-(2-Fluoro-6-methoxymethoxy-3-pyrrolidin-1-yl-benzoyl)-benzoyloxy]-3-(4-methoxymethoxy-benzoylamino)-azepan-1-carbonsäure-tert-butylester

MS: m/e= 562.6 $(M+H)^+$
IR (KBr): 1721, 1631, 1606 $cm^{-1}$

Beispiel 53

4-(2-Fluoro-6-hydroxy-3-pyrrolidin-1-yl-benzoyl)-benzoesäure (3R,4R)-3-(4-hydroxy-3,5-dimethyl-benzoylamino)-azepan-4-yl ester hydrochlorid (1:2) als lachsrosa Pulver aus (3R,4R)-4-[4-(2-Fluoro-6-methoxymethoxy-3-pyrrolidin-1-yl-benzoyl)-benzoyloxy]-3-(4-methoxymethoxy-3,5-dimethyl-benzoylamino)-azepan-1-carbonsäure-tert-butylester

MS: m/e= 590.6 $(M+H)^+$
IR (KBr): 1721, 1631, 1604 $cm^{-1}$

Beispiel 54

4-(2-Fluoro-6-hydroxy-3-methyl-benzoyl)-benzoesäure (3R, 4R)-3-(4-hydroxy-benzoylamino)-azepan-4-yl ester hydrochlorid (1:1) als beiges Pulver aus (3R, 4R)-3-(4-Methoxymethoxy)-benzylamino)-4-[4-(2-fluoro-6-methoxymethoxy-3-methyl-benzoyl)-benzoyloxy]-azepan-1-carbonsäure-tert-butylester

MS : 505.3 $(M+H)^+$
IR: 3415, 1721, 1604, 1471, 1276, 1276, 850.

Beispiel 55

4-(3-Ethyl-2-fluoro-6-hydroxy-benzoyl)-benzoesäure (3R, 4R)-3-(4-hydroxy-benzoylamino)-azepan-4-yl ester hydrochlorid (1:1) als beiges Pulver aus (3R, 4R)-4-[4-(3-Ethyl-2-fluoro-6-methoxymethoxy-benzoyl)-benzoyloxy]-3-(4-methoxymethoxy-3,5-dimethyl-benzoylamino)azepan-1-carbonsäure-tert-butylester

MS : 549.6 $(M+H)^+$.
IR : 3401, 1722, 1639, 1604, 1533, 1485, 1273, 1219, 827.

Beispiel 56

4-(2-Fluoro-6-hydroxy-3-piperidin-1-yl-benzoyl)-benzoesäure (3R,4R)-3-(4-hydroxy-3,5-dimethyl-benzoylamino)-azepan-4-yl ester hydrochlorid (1:2) als beiges Pulver aus (3R,4R)-4-[4-(2-Fluoro-6-methoxymethoxy-3-piperidin-1-yl-benzoyl)-benzoyloxy]-3-(4-methoxymethoxy-3,5-dimethyl-benzoylamino)-azepan-1-carbonsäure-tert-butylester

MS: 604.6 $(M+H)^+$
IR: 1720, 1673, 1630, 1534 $cm^{-1}$

Beispiel 57

4-(2-Fluoro-6-hydroxy-4-methoxy-benzoyl)-benzoesäure (3R,4R)-3-(4-hydroxy-benzoylamino)-azepan-4-yl ester hydrochlorid (1:1) als farbloses Pulver aus (3R,4R)-4-[4-(2-Fluoro-4-methoxy-6-methoxymethoxy-benzoyl)-benzoyloxy]-3-(4-methoxymethoxy-benzoylamino)-azepan-1-carbonsäure-tert-butylester

MS: m/e= 523.2 $(M+H)^+$
IR (KBr): 1718, 1639, 1608 $cm^{-1}$

Beispiel 58

4-(2-Fluoro-6-hydroxy-benzoyl)-benzoesäure (3R,4R)-3-(4-hydroxy-3-methoxy-benzoylamino)-azepan-4-yl ester hydrochlorid (1:1) als farbloses Pulver aus (3R,4R)-4-[4-(2-Fluoro-6-methoxymethoxy-benzoyl)-benzoyloxy]-3-(3-methoxy-4-methoxymethoxy-benzoylamino)-azepan-1-carbonsäure-tert-butylester

MS: m/e= 523.3 $(M+H)^+$
IR (KBr): 1721, 1634, 1617 $cm^{-1}$

Beispiel 59

4-[4-(2-Fluoro-6-hydroxy)-benzoyl]-benzoesäure (3R,4R)-3-(2-hydroxy-5-methoxy-benzoylamino)-azepan-4-yl ester hydrochlorid (1:1) als gelbes Pulver aus (3R,4R)-4-[4-(2-Fluoro-6-methoxymethoxy-benzoyl)-benzoyloxy]-3-(5-methoxy-2-methoxymethoxy-benzoylamino)-azepan-1-carbonsäure-tert- butylester

MS: 523.1 $(M+H)^+$
IR: 3405, 3296, 1718, 1637, 1617, 1542, 1495 $cm^{-1}$

Beispiel 60

4-(3-Hydroxy-naphthalin-2-ylcarbonyl)-benzoesäure (3R,4R)-3-(4-hydroxy-benzoylamino)-azepan-4-yl ester hydrochlorid (1:1) als gelbes Pulver aus (3R,4R)-3-[4-(tert-Butyl-dimethylsilanyloxy)-benzoylamino]-4-(3-methoxymethoxy-naphthalen-2-ylcarbonyl)-benzoyloxy]-azepan-1-carbonsäure-tert-butylester

MS: 525.4 $(M+H)^+$
IR: 3243, 1721, 1642, 1605, 1537, 1505 $cm^{-1}$

Beispiel 61

4-(2-Fluoro-6-hydroxy-4-methoxy-benzoyl)-benzoesäure (3R,4R)-3-(4-hydroxy-3,5-dimethoxy-benzoyl-

amino)-azepan-4-yl ester hydrochlorid (1:1) als farbloses Pulver aus (3R,4R)-4-[4-(2-Fluoro-4-methoxy-6-methoxymethoxy-benzoyl)-benzoyloxy]-3-(3,5-dimethoxy-4-methoxymethoxy-benzoylamino)-azepan-1-carbonsäure-tert-butylester

MS: m/e= 583.2 $(M+H)^+$
IR (KBr): 1721, 1639, 1604 $cm^{-1}$

Beispiel 62

4-(2-Fluoro-6-hydroxy-benzoyl)-benzoesäure (3R,4R)-3-(4-hydroxy-3,5-dimethoxy-benzoylamino)-azepan-4-yl ester hydrochlorid (1:1) als farbloses Pulver aus (3R,4R)-4-[4-(2-Fluoro-6-methoxymethoxy-benzoyl)-benzoyloxy]-3-(3,5-dimethoxy-4-methoxymethoxy-benzoylamino)-azepan-1-carbonsäure-tert-butylester

MS: m/e= 553.2 $(M+H)^+$
IR (KBr): 1722, 1630, 1615 $cm^{-1}$

Beispiel 63

4-(2,3-Difluoro-6-hydroxy-benzoyl)-benzoesäure (3R,4R)-3-(4-hydroxy-3,5-dimethoxy-benzoylamino)-azepan-4-yl ester hydrochlorid (1:1) als hellgelbes Pulver aus (3R,4R)-4-[4-(2,3-Difluoro-6-methoxymethoxy-benzoyl)-benzoyloxy]-3-(3,5-dimethoxy-4-methoxymethoxy-benzoylamino)-azepan-1-carbonsäure-tert-butylester

MS: m/e= 571.3 $(M+H)^+$
IR (KBr): 1722, 1647, 1606 $cm^{-1}$

Beispiel 64

4-(2,4-Difluoro-6-hydroxy-benzoyl)-benzoesäure (3R,4R)-3-(4-hydroxy-3,5-dimethoxy-benzoylamino)-azepan-4-yl ester hydrochlorid (1:1) als farbloses Pulver aus (3R,4R)-4-[4-(2,4-Difluoro-6-methoxymethoxy-benzoyl)-benzoyloxy]-3-(3,5-dimethoxy-4-methoxymethoxy-benzoylamino)-azepan-1-carbonsäure-tert-butylester

MS: m/e= 571.2 $(M+H)^+$
IR (KBr): 1722, 1640, 1609 $cm^{-1}$

Beispiel 65

4-(2-Hydroxy-5-methoxy-benzoyl)-benzoesäure (3R,4R)-3-(5-fluoro-2-hydroxy-benzoylamino)-azepan-4-yl ester hydrochlorid(1:1) als gelbes Pulver aus (3R,4R)-3-(5-fluoro-2-hydroxy-benzoylamino)-4-[4-(5-methoxy-2-methoxymethoxy-benzoyl)-benzoyloxy]-azepan-1-carbonsäure-tert-butylester

MS: 523.2 $(M+H)^+$
IR: 3406, 1720, 1636, 1609, 1485, 1274, 1228, 826$cm^{-1}$

Beispiel 66

3,5-Difluoro-4-(2-hydroxy-5-methoxy-benzoyl)-benzoesäure (3R,4R)-3-(4-hydroxy-benzoylamino)-azepan-4-yl ester hydrochlorid (1:1) als gelbes Pulver aus (3R,4R)-4-[3,5-Difluoro-4-(5-methoxy-2-methoxymethoxy-benzoyl)-benzoyloxy]-3-(4-hydroxy-benzoylamino)-azepan-1-carbonsäure-tert-butylester

MS:541.3$(M+H)^+$
IR: 217.1, 109.1: IR: 3411, 1729, 1636, 1609, 1484, 1223, 1035 852$cm^{-1}$

Beispiel 67

4-(2-Hydroxy-5-methylthio-benzoyl)-benzoesäure (3R,4R)-3-(4-hydroxy-benzoylamino)-azepan-4-yl ester hydrochlorid (1:1) als gelbes Pulver aus (3R,4R)-3-(4-Hydroxy-benzoylamino)-4-[4-(2-methoxymethoxy-5-methylthio-benzoyl)-benzoyloxy]-azepan-1-carbonsäure-tert-butylester

MS:519.3(M-H)$^-$

IR: 3403, 1721, 1627, 1606, 1540, 1277, 1017, 829cm$^{-1}$

Beispiel 68

4-(2-Hydroxy-5-methoxy-benzoyl)-benzoesäure (3RS, 4RS)-4-(4-hydroxy-3,5-dimethyl-benzoylamino)-pyrrolidin-3-yl ester hydrochlorid (1:1) als gelbes Pulver aus (3RS,4RS)-3-[4-(5-Methoxy-2-methoxymethoxy-benzoyl)-benzoyloxy]-4-(4-methoxymethoxy-3,5-dimethyl-benzoylamino)-pyrrolidin-1-carbonsäure-tert-butylester

MS: 505.3 (M+H)$^+$

IR: 3387, 3269, 1728, 1638, 1606, 1533 cm$^{-1}$

Beispiel 69

4-(2-Hydroxy-5-methoxy-benzoyl)-benzoesäure (3RS, 4RS)-4-(4-hydroxy-benzoylamino)-pyrrolidin-3-yl ester hydrochlorid (1:1) als gelbes Pulver aus (3RS,4RS)-3-[4-(5-Methoxy-2-methoxymethoxy-benzoyl)-benzoyloxy]-4-(4-methoxymethoxy-benzoylamino)-pyrrolidin-1-carbonsäure-tert-butylester

MS: 477.3 (M+H)$^+$

IR: 3417, 1727, 1637, 1608 cm$^{-1}$

Beispiel 70

4-(2-Hydroxy-5-methoxy-benzoyl)-benzoesäure (3RS, 4SR)-4-(4-hydroxy-3,5-dimethyl-benzoylamino)-pyrrolidin-3-yl ester hydrochlorid (1:1) als gelbes Pulver aus (3RS,4SR)-3-[4-(5-Methoxy-2-methoxymethoxy-benzoyl)-benzoyloxy]-4-(4-methoxymethoxy-3,5-dimethyl-benzoylamino)-pyrrolidin-1-carbonsäure-tert-butylester

MS: 505.4 (M+H)$^+$

IR: 3415, 1716, 1650, 1605, 1540, 1486 cm$^{-1}$

Beispiel 71

4-(2-Fluoro-6-hydroxy-3-methoxy-benzoyl)-benzoesäure (3RS,4RS)-4-(4-hydroxy-3,5-dimethyl-benzylamino)-pyrrolidin-3-yl ester hydrochlorid (1:1) als gelbes Pulver aus (3RS,4RS)-3-[4-(2-Fluoro-3-methoxy-6-methoxymethoxy-benzoyl)-benzoyloxy]-4-(4-methoxymethoxy-3,5-dimethyl-benzoylamino)-pyrrolidin-1-carbonsäure-tert-butylester

MS: 523.4 (M+H)$^+$

IR: 3399, 2736, 1729, 1643, 1605, 1534, 1484 cm$^{-1}$

Beispiel 72

4-(2-Fluoro-6-hydroxy-3-methoxy-benzoyl)-benzoesäure (3RS,4RS)-4-pyridin-4-ylcarbonylamino-pyrrolidin-3-yl ester hydrochlorid (1:2) als gelbes Pulver aus (3RS,4RS)-3-[4-(2-Fluoro-3-methoxy-6-methoxymethoxy-benzoyl)-benzoyloxy]-4-pyridin-4-ylcarbonylamino-pyrrolidin-1-carbonsäure-tert-butylester

MS: 480.3 (M+H)$^+$

IR: 3431, 1727, 1693, 1538, 1491 cm$^{-1}$

Beispiel 73

4-(2-Hydroxy-5-methoxy-benzoyl)-benzoesäure (3RS, 4RS)-4-(4-hydroxy-benzoylamino)-pyrrolidin-3-yl ester hydrochlorid (1:1) als gelbes Pulver aus (3RS,4RS)-3-[4-(2-Fluoro-3-methoxy-6-methoxymethoxy-benzoyl)-benzoyloxy]-4-(4-methoxymethoxy-benzoylamino)-pyrrolidin-1-carbonsäure-tert-butylester

MS: 495.3 (M+H)$^+$

IR: 3417, 3261, 1728, 1643, 1608, 1539, 1504 cm$^{-1}$

Beispiel 74

4-(6-Hydroxy-1,3-benzodioxol-5-ylcarbonyl)-benzoesäure (3RS,4RS) -4-(4-hydroxy-3,5-dimethyl-benzoylamino)-pyrrolidin-3-yl ester hydrochlorid (1:1) als gelbes Pulver aus (3RS,4RS)-3-[4-(6-Methoxymethoxy-1,3-benzodioxol-5-ylcarbonyl)-benzoyloxy]-(-4-methoxymethoxy-benzoylamino)pyrrolidin-1-carbonsäure-tert-butylester

MS: 519.3 $(M+H)^+$
IR: 3416, 1726, 1700, 1641, 1603, 1530, 1249, 1038 $cm^{-1}$

Beispiel 75

(3R,4R)-4-hydroxy-N-[4-[4-(2-hydroxy-5-methoxy-benzoyl)benzyloxy]-azepan-3-yl]-benzamid hydrochlorid (1:1) als gelbes Pulver aus (3R,4R)-3-(4-Methoxymethoxy-benzoylamino)-4-[4-(5-methoxy-2-methoxymethoxy- benzoyl)-benzyloxy]-azepan-1-carbonsäure-tert-butylester

MS: 491.3 $(M+H)^+$
IR: 3239, 1634, 1607, 1542, 1506, 1483 $cm^{-1}$

Beispiel 76

(3R,4R)-2-Hydroxy-N-[4-[4-(2-hydroxy-5-methoxy-benzoyl]-azepan-3-yl]-5-methoxy-benzamid hydrochlorid (1:1) als gelbes Pulver aus (3R,4R)-3-(5-Methoxy-2-methoxymethoxy-benzoylamino)-4-[4-(5-methoxy-2-methoxymethoxy-benzoyl)-benzyloxy]-azepan-1-carbonsäure-tert-butylester

MS: 521.5 $(M+H)^+$
IR: 3422, 2939, 1636, 1598, 1542, 1488 $cm^{-1}$

Beispiel 77

Ein Gemisch von 14 mg 4-(2-hydroxy-5-methoxy-benzoyl)-benzoesäure (3RS,4SR)-4-(4-hydroxy-3,5-dimethylbenzoylamino-pyrrolidin-3-yl ester hydrochlorid (Beispiel 70), 0.01 ml Triethylamin und 37mg Formamidinsulfonsäure in 2 ml Dimethylformamid wurde 6 Tage bei Raumtemperatur gerührt. Das Lösungsmittel wurde unter vermindertem Druck abgedampft und der Rückstand in Wasser aufgenommen, beschallt und filtriert.Nach Trocknen unter vermindertem Druck wurden 8 mg 4-(2-Hydroxy-5-methoxy-benzoyl)-benzoesäure (3RS,4SR)-1-(amino-iminomethyl)-4-(4-hydroxy-3,5-dimethylbenzoylamino)-pyrrolidin-3-yl ester als gelbes Pulver erhalten.

MS: 547.5 $(M+H)^+$

Die Beispiele A-F erläutern die Herstellung pharmazeutischer Präparate.

Beispiel A

Hartgelatinekapseln können wie folgt hergestellt werden:

| Bestandteile | mg/Kapsel |
|---|---|
| 1. sprühgetrocknetes Pulver enthaltend 75% Verbindung I | 20 |
| 2. Natriumdioctylsulfosuccinat | 0,2 |
| 3. Natriumcarboxymethylcellulose | 4,8 |
| 4. mikrokristalline Cellulose | 86,0 |
| 5. Talk | 8,0 |
| 6. Magnesiumstearat | 1,0 |
| Total | 120 |

Das sprühgetrocknete Pulver, das auf dem Wirkstoff, Gelatine und mikrokristalliner Cellulose basiert und eine mittlere Korngrösse des Wirkstoffes von <1 µ aufweist (mittels Autokorrelationsspektroskopie gemessen), wird mit einer wässrigen Lösung von Natriumcarboxymethylcellulose und Natriumdioctylsulfosuccinat befeuchtet und geknetet. Die resultierende Masse wird granuliert, getrocknet und gesiebt, und das erhaltene Granulat mit mikrokristalliner Cellulose, Talk und Magnesiumstearat vermischt. Das Pulver wird in Kapseln der Grösse 0 abgefüllt.

Beispiel B

Tabletten können wie folgt hergestellt werden:

| Bestandteile | mg/Tablette |
|---|---|
| 1. Verbindung I als feingemahlenes Pulver | 20 |
| 2. Milchzucker pulv. | 100 |
| 3. Maisstärke weiss | 60 |
| 4. Povidone K30 | 8 |
| 5. Maisstärke weiss | 112 |
| 6. Talk | 16 |
| 7. Magnesiumstearat | 4 |
| Total | 320 |

Die feingemahlene Substanz wird mit Milchzucker und einem Teil der Maisstärke gemischt. Die Mischung wird mit einer wässrigen Lösung von Povidone K30 befeuchtet und geknetet, und die resultierende Masse granuliert, getrocknet und gesiebt. Das Granulat wird mit der restlichen Maisstärke, Talk und Magnesiumstearat vermischt und zu Tabletten geeigneter Grösse verpresst.

Beispiel C

Weichgelatinekapseln können wie folgt hergestellt werden:

| Bestandteile | mg/Kapsel |
|---|---|
| 1. Verbindung I | 5 |
| 2. Triglycerid | 450 |
| Total | 455 |

10 g Verbindung I werden unter Rühren, Inertbegasung und Lichtschutz in 90 g mittelkettigem Triglycerid gelöst. Diese Lösung wird als Kapselfüllmasse zu Weichgelatinekapseln à 5 mg Wirkstoff verarbeitet.

Beispiel D

Eine Crème kann aus den nachstehend aufgeführten Inhaltsstoffen in an sich bekannter Weise hergestellt werden:

| | Gew.-% |
|---|---|
| Verbindung der Formel I | 0,1-5 |

(fortgesetzt)

| | Gew.-% |
|---|---|
| Cetylalkohol | 5,25-8,75 |
| Arlacel 165 (Glyceryl/PEG 100-stearat) | 3,75-6,25 |
| Miglyol 818 (Capryl-/Caprin-/Linolsäure triglycerid) | 11,25-18,75 |
| Sorbit-Lösung | 3,75-6,25 |
| EDTA-$Na_2$ | 0,075-0,125 |
| Carbopol 934P (Carbomer 934P) | 0,15-0,25 |
| butyliertes Hydroxyanisol | 0,0375-0,0625 |
| Methylparaben | 0,135-0,225 |
| Propylparaben | 0,0375-0,0625 |
| NaOH (10% solution) | 0,15-0,25 |
| Wasser q.s. | 100,00 |

Beispiel E

Ein Gel kann aus den nachstehend aufgeführten Inhaltsstoffen in an sich bekannter Weise hergestellt werden:

| | Gew.-% |
|---|---|
| Verbindung der Formel I | 0,1-5 |
| Pluronic L 101 (Poloxamer 331) | 10,00 |
| Aerosil 200 (Siliciumdioxid) | 8,00 |
| PCL Liquid (Fettsäureester | 15,00 |
| Cetiol V (Decyloleat) | 20,00 |
| Neobee Oil (Triglycerid mittlerer Kettenlänge) | 15,00 |
| Euhanol G (Octyldodecanol), q.s. | 100,00 |

Durch Variation der Verhältnisse zwischen den Hilfsstoffen der Beispiele D und E können die physikalischen Eigenschaften der Präparate verändert werden.

Beispiel F

Eine Lösung kann aus folgenden Bestandteilen hergestellt werden:

| Bestandteile | mg |
|---|---|
| Verbindung der Formel I | 10 |
| Propylenglykol | 100 |
| Feinsprit 94%ig | 300 |
| Phosphorsäure ca. 85%ig | 5 |
| NaOH 1-normal ad pH 3 | |

(fortgesetzt)

| Bestandteile | mg |
|---|---|
| Wasser demineralisiert ad 1 ml | |

**Patentansprüche**

**1.** Verbindungen der Formel

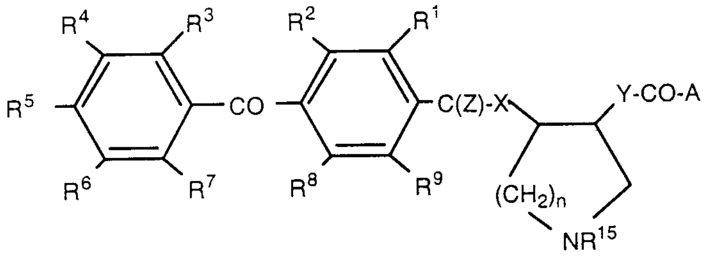

I

worin

A einen Rest

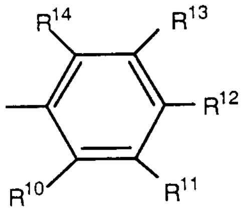

$A^1$

2-, 3- oder 4-Pyridyl oder 2- oder 3-Piperazinyl, oder durch eine oder mehrere $C_{1-6}$-Alkyl-, $C_{1-6}$-Alkoxy- und/oder Hydroxygruppen substituiertes 2-,3- oder 4-Pyridyl oder 2- oder 3-Piperazinyl;

X und Y unabhängig voneinander Sauerstoff oder NH, jedoch nicht beide NH bedeuten;

Z Sauerstoff oder, falls X Sauerstoff ist, Sauerstoff oder H,H bedeutet;

n 1,2 oder 3 ist;

$R^1$ Wasserstoff oder Fluor;

$R^2$ Wasserstoff, $C_{1-6}$-Alkoxy oder Fluor;

$R^3$ Wasserstoff, Hydroxy, $C_{1-6}$-Alkoxy, Fluor, Trifluormethyl, $C_{2-7}$-Alkoxycarbonyl, Tetrazolyl oder durch $C_{1-6}$-Alkyl, Benzyl, Cyanomethyl oder Carbamoyl-methyl substituiertes Tetrazolyl;

$R^4$ Wasserstoff, Hydroxy, $C_{1-6}$-Alkoxy, $C_{1-6}$-Alkyl, Chlor, Fluor, Trifluormethyl, Acetyl, Di-$C_{1-6}$-alkylamino, oder $C_{1-6}$-Alkoxy-$C_{1-6}$-alkyl, $C_{1-6}$-Alkylthio, $C_{1-6}$-Alkylsulfonyl, Phenyl, Pyrrolidinyl oder Piperidinyl;

$R^5$ Wasserstoff, $C_{1-6}$-Alkoxy, Fluor oder Trifluormethyl;

$R^6$ Wasserstoff, Hydroxy, $C_{1-6}$-Alkoxy, Fluor, 2,4-Difluorphenyl, $C_{1-6}$-Alkoxy-$C_{1-6}$-alkyl, $C_{1-6}$-Alkanoyl, Benzoyl, Nitrilo, Trifluormethyl, $C_{3-6}$-Cycloalkyl, 2- oder 4-Thiazolyl, 2-Thiazolidinyl, 2-Oxazolyl oder 2-Oxazolidinyl, 2- oder 4-Imidazolyl;

$R^7$ Wasserstoff, Hydroxy, $C_{1-6}$-Alkoxy, Carboxy, Amino oder Fluor;

$R^6$ und $R^7$ gemeinsam einen Rest -N=CH-CH=N- oder $-N(CH_3)CH_2CH_2N(CH_3)-$;

$R^8$ Wasserstoff, $C_{1-6}$-Alkoxy, $C_{1-6}$-Alkyl oder Fluor;

$R^9$ Wasserstoff oder Fluor;

$R^{10}$ Wasserstoff, Hydroxy, $C_{1-6}$-Alkoxy oder $C_{1-6}$-Alkyl;

$R^{11}$ Wasserstoff, $C_{1-6}$-Alkoxy, $C_{1-6}$-Alkyl, Fluor oder Brom;

$R^{12}$ Wasserstoff, Hydroxy, $C_{1-6}$-Alkoxy, Carboxy, $C_{2-7}$-Alkoxycarbonyl oder Amino;

$R^{13}$ Wasserstoff, Hydroxy, $C_{1-6}$-Alkoxy, $C_{1-6}$-Alkyl, Acetyl oder Fluor;

$R^{14}$ Wasserstoff, $C_{1-6}$-Alkyl oder Fluor;

$R^{15}$ Wasserstoff oder Amidino;

$R^3$ und $R^4$ gemeinsam einen Rest -CH=CH-CH=CH- oder Aethylendioxy;

$R^4$ und $R^5$ gemeinsam einen Rest -CH=CH-CH=CH-, Tetramethylen, Methylendioxy, Aethylendioxy oder einen Rest -N=CH-CH=CH-oder einen Rest (a)

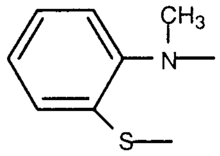

(a)

in der die Bindung zum S durch $R^5$ erfolgt; und

$R^{12}$ und $R^{13}$ gemeinsam einen Rest -CH=CH-CH=CH- oder -C(OH)=CH-CH=CH-, in der die Bindung zu dem die Hydroxygruppe tragenden C-Atom durch $R^{12}$ erfolgt;

bedeuten; und pharmazeutisch anwendbare Salze davon.

2. Verbindungen gemäss Anspruch 1, in denen $R^1$ und $R^9$ Wasserstoff, $R^2$ und $R^8$ Wasserstoff oder Fluor, und $R^7$ Wasserstoff, Hydroxy, $C_{1-6}$-Alkoxy, Amino oder Fluor darstellen.

3. Verbindungen gemäss Anspruch 1 oder 2, in denen X Sauerstoff und Y NH ist.

4. Verbindungen gemäss den Ansprüchen 1-3, in denen Z Sauerstoff ist.

5. Verbindungen gemäss den Ansprüchen 1-4, in denen n = 3 ist.

6. Verbindungen gemäss den Ansprüchen 1-5, in denen $R^1$, $R^2$, $R^8$ und $R^9$ Wasserstoff sind.

7. Verbindungen gemäss den Ansprüchen 1-6, in denen $R^7$ Hydroxy ist.

8. Verbindungen gemäss den Ansprüchen 1-7, in denen $R^3$ Wasserstoff, $C_{1-6}$-Alkyl, $C_{1-6}$-Alkoxy, Fluor oder Cyanomethyltetrazolyl; $R^4$ Wasserstoff, $C_{1-6}$-Alkyl, $C_{1-6}$-Alkoxy, $C_{1-6}$-Alkylthio, $C_{1-6}$-Alkoxy- $C_{1-6}$-alkyl, Di( $C_{1-6}$-alkyl)-amino, $C_{2-7}$-Alkanoyl, Phenyl, Pyrrolidino oder Fluor; $R^5$ Wasserstoff, $C_{1-6}$-Alkoxy, Fluor oder Trifluormethyl; $R^6$ Wasserstoff; $R^4$ und $R^5$ gemeinsam Tetramethylen, Methylendioxy, Aethylendioxy oder einen Rest -CH=CH-CH=CH-, -CH=CH-CH=N- oder

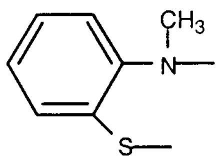

(a); und

$R^3$ und $R^4$ gemeinsam Aethylendioxy bedeuten.

9. Verbindungen gemäss den Ansprüchen 1-8, in denen A 4-Pyridyl ist.

10. Verbindungen gemäss den Ansprüchen 1-8, in denen A ein Rest $A^1$ ist.

11. Verbindungen gemäss Anspruch 10, in denen $R^{10}$ Wasserstoff, Hydroxy oder $C_{1-6}$-Alkyl; $R^{11}$ Wasserstoff, $C_{1-6}$-Alkyl, $C_{1-6}$-Alkoxy oder Brom; $R^{12}$ Wasserstoff, Hydroxy oder $C_{1-6}$-Alkoxy; $R^{13}$ Wasserstoff, $C_{1-6}$-Alkyl, $C_{1-6}$-Alkoxy, Fluor oder Acetyl; $R^{14}$ Wasserstoff oder $C_{1-6}$-Alkyl; oder $R^{12}$ und $R^{13}$ gemeinsam einen Rest -CH=CH-CH=CH- oder -C(OH)=CH-CH=CH-, in der die Bindung zu dem die Hydroxygruppe tragenden C-Atom durch $R^{12}$ erfolgt, darstellen.

12. Verbindungen gemäss den Ansprüchen 1-11, in denen $R^{15}$ Wasserstoff ist.

13. Verbindungen gemäss Anspruch 10, in denen $R^{12}$ Hydroxy und mindestens einer der Reste $R^{10}$, $R^{11}$, $R^{13}$ und $R^{14}$ $C_{1-6}$-Alkyl ist.

**14.** Die Verbindungen gemäss Anspruch 13,

4-(2-Fluoro-6-hydroxy-3-methoxy-benzoyl)-benzoesäure (3R,4R)-3-(4-hydroxy-2,3,6-trimethyl-benzoylamino)-azepan-4-yl ester hydrochlorid (1:1)

4-(2,3-Difluoro-6-hydroxy-benzoyl)-benzoesäure (3R,4R)-3-(4-hydroxy-2,3,6-trimethyl-benzoylamino)-azepan-4-yl ester hydrochlorid (1:1)

4-(2,3-Difluoro-6-hydroxy-benzoyl)-benzoesäure (3R,4R)-3 -(4-hydroxy-3,5-diisopropyl-benzoylamino)-azepan-4-yl ester hydrochlorid (1:1)

4-(2-Hydroxy-5-dimethylamino-benzoyl)-benzoesäure (3R,4R)-3-(4-hydroxy-3,5-diisopropyl-benzoylamino)-azepan-4-yl ester hydrochlorid (1:2)

4-(2-Hydroxy-5-dimethylamino-benzoyl)-benzoesäure (3R,4R)-3-(4-hydroxy-3,5-dimethyl-benzoylamino)-azepan-4-yl ester hydrochlorid (1:2)

4-(2-Fluoro-6-hydroxy-4-methoxy-benzoyl)-benzoesäure (3R,4R)-3-(4-hydroxy-3,5-diisopropyl-benzoylamino)-azepan-4-yl ester hydrochlorid (1:1)

4-(2,3-Difluoro-6-hydroxy-benzoyl)-benzoesäure (3R,4R) -3-(4-hydroxy-3,5-dimethyl-benzoylamino)-azepan-4-yl ester hydrochlorid (1:1)

4-(2,4-Difluoro-6-hydroxy-benzoyl)-benzoesäure (3R,4R) -3-(4-hydroxy-3,5-dimethyl-benzoylamino)-azepan-4-yl ester hydrochlorid (1:1)

4-(6-Hydroxy-chinolin-7-ylcarbonyl)-benzoesäure (3R,4R) -3-(4-hydroxy-3,5-dimethyl-benzoylamino)-azepan-4-yl ester hydrochlorid (1:1)

4-(2-Fluoro-6-hydroxy-3-methoxy-benzoyl)-benzoesäure (3R,4R)-3-(3,5-diethyl-4-hydroxy-benzoylamino)-azepan-4-yl ester hydrochlorid (1:1)

4-(2,3-Difluoro-6-hydroxy-benzoyl)-benzoesäure (3R,4R)-3-(3,5-diethyl-4-hydroxy-benzoylamino)-azepan-4-yl ester hydrochlorid (1:1)

4-(2-Hydroxy-5-dimethylamino-benzoyl)-benzoesäure (3R,4R)-3-(3,5-diethyl-4-hydroxy-benzoylamino)-azepan-4-yl ester hydrochlorid (1:2)

4-(2-Hydroxy-5-methoxy-benzoyl)-benzoesäure (3R,4R) -3-(4-hydroxy-3,5-dimethyl-benzoylamino)-azepan-4-yl ester hydrochlorid (1:1)

4-(6-Hydroxy-1,3-benzodioxol-5-ylcarbonyl)-benzoesäure (3R,4R) -3-(4-hydroxy-3,5-dimethyl-benzoylamino)-azepan-4-yl ester hydrochlorid (1:1)

4-(6-Hydroxy-1,4-benzodioxin-1-yl-carbonyl)-benzoesäure (3R, 4R)-3-(4-hydroxy-3,5-dimethyl-benzoylamino)-azepan-4-yl ester hydrochlorid (1:1)

4-(2-Fluoro-6-hydroxy-3-dimethylamino-benzoyl)-benzoesäure (3R,4R)-3-(4-hydroxy-3,5-dimethyl-benzoylamino)-azepan-4-yl ester hydrochlorid (1:2)

4-(5-Acetyl-2-hydroxy-benzoyl)-benzoesäure (3R,4R)-3-(4-hydroxy-3,5-dimethoxy-benzoylamino)-azepan-4-yl ester hydrochlorid (1:1)

4-(2-Hydroxy-5-methoxy-benzoyl)-benzoesäure (3R,4R)-3-(3-tert-butyl-4-hydroxy-benzoylamino)-azepan-4-yl ester hydrochlorid (1:1)

4-(2-Hydroxy-5-methoxy-benzoyl)-benzoesäure (3R,4R)-3-(4-hydroxy-3-isopropyl-benzoylamino)-azepan-4-yl ester hydrochlorid (1:1)

4-(2-Hydroxy-5-methoxy-benzoyl)-benzoesäure (3R,4R)-3-(4-hydroxy-3-sec-butyl-benzoylamino)-azepan-4-yl ester hydrochlorid (1:1)

4-(2-Fluoro-3-isopropyl-6-hydroxy-benzoyl)-benzoesäure (3R,4R)-3-(4-hydroxy-3,5-dimethyl-benzoylamino)-azepan-4-yl ester hydrochlorid (1:1)

4-(2-Fluoro-6-hydroxy-3-pyrrolidin-1-yl-benzoyl)-benzoesäure (3R,4R)-3-(4-hydroxy-3,5-dimethyl-benzoylamino)-azepan-4-yl ester hydrochlorid (1:2)

4-(3-Ethyl-2-fluoro-6-hydroxy-benzoyl)-benzoesäure (3R, 4R)-3-(4-hydroxybenzoylamino)-azepan-4-yl ester hydrochlorid (1:1)

4-(2-Fluoro-6-hydroxy-3-piperidin-1-yl-benzoyl)-benzoesäure (3R,4R)-3-(4-hydroxy-3,5-dimethyl-benzoylamino)-azepan-4-yl ester hydrochlorid (1:2)

4-(2-Hydroxy-5-methoxy-benzoyl)-benzoesäure (3RS, 4RS)-4-(4-hydroxy-3,5-dimethyl-benzoylamino)-pyrrolidin-3-yl ester hydrochlorid (1:1)

4-(2-Hydroxy-5-methoxy-benzoyl)-benzoesäure (3RS, 4SR)-4-(4-hydroxy-3,5-dimethyl-benzoylamino)-pyrrolidin-3-yl ester hydrochlorid (1:1)

4-(2-Fluoro-6-hydroxy-3-methoxy-benzoyl)-benzoesäure (3RS,4RS)-4-(4-hydroxy-3,5-dimethyl-benzylamino)-pyrrolidin-3-yl ester hydrochlorid (1:1)

4-(6-Hydroxy-1,3-benzodioxol-5-ylcarbonyl)-benzoesäure (3RS,4RS) -4-(4-hydroxy-3,5-dimethyl-benzoylamino)-pyrrolidin-3-yl ester hydrochlorid (1:1)

4-(2-Hydroxy-5-methoxy-benzoyl)-benzoesäure (3RS,4SR)-1-(amino-iminomethyl)-4-(4-hydroxy-3,5-dimethyl-benzoylamino)-pyrrolidin-3-yl ester

**15.** Die Verbindungen gemäss Anspruch 13, 4-(2-Fluoro-6-hydroxy-3-methoxy-benzoyl)-benzoesäure (3R,4R)-3-(4-hydroxy-3,5-dimethyl-benzoylamino)-azepan-4-yl ester hydrochlorid (1:1).

**16.** Verbindungen gemäss Anspruch 10, in denen $R^{12}$ Hydroxy und $R^{10}$, $R^{11}$, $R^{13}$ und $R^{14}$ Wasserstoff sind.

**17.** Die Verbindungen gemäss Anspruch 16,

4-(2-Fluoro-6-hydroxy-3-methoxy-benzoyl)-benzoesäure (3R,4R)-3-(4-hydroxybenzoylamino)-azepan-4-yl ester hydrochlorid (1:1)

3-(3-Hydroxy-5,6,7,8-tetrahydro-naphtalin-2-ylcarbonyl)-benzoylamino)-azepan-4-yl ester hydrochlorid (1:1)

4-(2-Hydroxy-5-dimethylamino-benzoyl)-benzoesäure (3R,4R)-3-(4-hydroxybenzoylamino)-azepan-4-yl ester hydrochlorid (1:2)

4-(7-Hydroxy-1,4-benzodioxin-6-ylcarbonyl)-benzoesäure (3R,4R)-3-(4-hydroxybenzoylamino)-azepan-4-yl ester hydrochlorid (1:1)

4-[2-Hydroxy-5-(1-methoxy-ethyl)-benzoyl]-benzoesäure (3R,4R)-3-(4-hydroxybenzoylamino)-azepan-4-yl ester hydrochlorid (1:1)

4-(6-Hydroxy-chinolin-7-ylcarbonyl)-benzoesäure (3R,4R)-3-(4-hydroxybenzoylamino)-azepan-4-yl ester hydrochlorid (1:1)

4-(4-Hydroxy-biphenyl-3-ylcarbonyl)-benzoesäure (3R, 4R)-3-(4-hydroxybenzoylamino)-azepan-4-yl ester hydrochlorid (1:1)

4-(5-Acetyl-2-hydroxy-benzoyl)-benzoesäure (3R, 4R)-3-(4-hydroxybenzoylamino)-azepan-4-yl ester hydrochlorid (1:1)

4-(2-Hydroxy-10-methyl-phenothiazin-1-ylcarbonyl)-benzoesäure (3R, 4R)-3-(4-hydroxy-benzoylamino)-azepan-4-yl ester hydrochlorid (1:2)

4-(2-Hydroxy-5-methyl-benzoyl)-benzoesäure (3R, 4R)-3-(4-hydroxy-benzoylamino)-azepan-4-yl ester hydrochlorid (1:1)

4-(2-Hydroxy-5-isopropyl-benzoyl)-benzoesäure (3R, 4R)-3-(4-hydroxy-benzoylamino)-azepan-4-yl ester hydrochlorid (1:1)

4-(2-Fluoro-3-dimethylamino-6-hydroxy-benzoyl)-benzoesäure (3R,4R)-3-(4-hydroxy-benzoylamino)-azepan-4-yl ester hydrochlorid (1:2)

4-(2-Fluoro-6-hydroxy-3-pyrrolidin-1-yl-benzoyl)-benzoesäure (3R,4R)-3-(4-hydroxy-benzoylamino)-azepan-4-yl ester hydrochlorid (1:2)

4-(2-Fluoro-6-hydroxy-3-methyl-benzoyl)-benzoesäure (3R, 4R)-3-(4-hydroxy-benzoylamino)-azepan-4-yl ester hydrochlorid (1:1)

4-(2-Fluoro-6-hydroxy-4-methoxy-benzoyl)-benzoesäure (3R,4R)-3-(4-hydroxy-benzoylamino)-azepan-4-yl ester hydrochlorid (1:1)

4-(3-Hydroxy-naphthalin-2-ylcarbonyl)-benzoesäure (3R,4R)-3-(4-hydroxy-benzoylamino)-azepan-4-yl ester hydrochlorid (1:1)

3,5-Difluoro-4-(2-hydroxy-5-methoxy-benzoyl)-benzoesäure (3R,4R)-3-(4-hydroxy-benzoylamino)-azepan-4-yl ester hydrochlorid (1:1)

4-(2-Hydroxy-5-methylthio-benzoyl)-benzoesäure (3R,4R)-3-(4-hydroxy-benzoylamino)-azepan-4-yl ester hydrochlorid (1:1) 4-(2-Hydroxy-5-methoxy-benzoyl)-benzoesäure (3RS, 4RS)-4-(4-hydroxy-benzoylamino)-pyrrolidin-3-yl ester hydrochlorid (1:1)

4-(2-Hydroxy-5-methoxy-benzoyl)-benzoesäure (3RS, 4RS)-4-(4-hydroxy-benzoylamino)-pyrrolidin-3-yl ester hydrochlorid (1:1)

(3R,4R)-4-hydroxy-N-[4-[4-(2-hydroxy-5-methoxy-benzoyl)benzyloxy]-azepan-3-yl]-benzamid hydrochlorid (1:1).

**18.** Verbindungen gemäss Anspruch 10, in denen mindestens einer des Reste $R^{10}$-$R^{14}$ $C_{16}$-Alkoxy ist.

**19.** Die Verbindungen gemäss Anspruch 18,

4-(2-Fluor-6-hydroxy-3-methoxy-benzoyl)-benzoesäure (3R,4R)-3-(4-hydroxy-3-methoxy-benzoylamino)-azepan-4-yl ester hydrochlorid (1:1)
4-(2-Fluor-6-hydroxy-3-methoxy-benzoyl)-benzoesäure (3R,4R)-3-(4-hydroxy-3,5-dimethoxy-benzoylamino)-azepan-4-yl ester hydrochlorid (1:1)
4-(3-Hydroxy-naphtalin-2-ylcarbonyl)-benzoesäure (3R,4R)-3-(4-hydroxy-3,5-dimethoxy-benzoylamino)-azepan-4-yl ester hydrochlorid(1:1)
4-(2-Hydroxy-5-methoxy-benzoyl)-benzoesäure (3R,4R)-3-(3,5-dimethoxy-benzoylamino)-azepan-4-yl ester hydrochlorid (1:1)
4-(2-Fluoro-6-hydroxy-3-methoxy-benzoyl)-benzoesäure (3R,4R)-3-(2-hydroxy-3-methoxy-benzoylamino)-azepan-4-yl ester hydrochlorid (1:1)
4-(2-Fluoro-6-hydroxy-3-methoxy-benzoyl)-benzoesäure (3R,4R)-3-(3,5-dimethoxy-benzoylamino)-azepan-4-yl ester hydrochlorid (1:1)
4-(2-Hydroxy-5-methoxy-benzoyl)-benzoesäure (3R,4R)-3-(3,4,5-trimethoxy-benzoylamino)-azepan-4-yl ester hydrochlorid (1:1)
4-(2-Hydroxy-5-dimethylamino-benzoyl)-benzoesäure (3R,4R)-3-(4-hydroxy-3-methoxy-benzoylamino)-azepan-4-yl ester hydrochlorid (1:2)
4-(2-Hydroxy-5-methoxy-benzoyl)-benzoesäure (3R,4R)-3-(3,5-diethoxy-benzoylamino)-azepan-4-yl ester hydrochlorid (1:1)
4-(2-Fluoro-6-hydroxy-3-dimethylamino-benzoyl)-benzoesäure (3R,4R)-3-(4-hydroxy-3,5-dimethoxy-benzoyl-amino)-azepan-4-yl ester hydrochlorid (1:2)
4-(2-Fluoro-6-hydroxy-benzoyl)-benzoesäure (3R,4R)-3-(4-hydroxy-3-methoxy-benzoylamino)-azepan-4-yl ester hydrochlorid (1:1)
4-[4-(2-Fluoro-6-hydroxy)-benzoyl]-benzoesäure (3R,4R)-3-(2-hydroxy-5-methoxy-benzoylamino)-azepan-4-yl ester hydrochlorid (1:1)
4-(2-Fluoro-6-hydroxy-4-methoxy-benzoyl)-benzoesäure (3R,4R)-3-(4-hydroxy-3,5-dimethoxy-benzoylamino)-azepan-4-yl ester hydrochlorid (1:1)
4-(2-Fluoro-6-hydroxy-benzoyl)-benzoesäure (3R,4R)-3-(4-hydroxy-3,5-dimethoxy-benzoylamino)-azepan-4-yl ester hydrochlorid (1:1)
4-(2,3-Difluoro-6-hydroxy-benzoyl)-benzoesäure (3R,4R)-3-(4-hydroxy-3,5-dimethoxy-benzoylamino)-azepan-4-yl ester hydrochlorid (1:1)
4-(2,4-Difluoro-6-hydroxy-benzoyl)-benzoesäure (3R,4R)-3-(4-hydroxy-3,5-dimethoxy-benzoylamino)-azepan-4-yl ester hydrochlorid (1:1)
(3R,4R)-2-Hydroxy-N-[4-[4-(2-hydroxy-5-methoxy-benzoyl]-azepan-3-yl]-5-methoxy-benzamid hydrochlorid (1:1)

**20.** Verbindungen gemäss Anspruch 10, in denen mindestens einer des Reste $R^{11}$, $R^{13}$ und $R^{14}$ Fluor oder $R^{11}$ Brom ist.

**21.** Die Verbindungen gemäss Anspruch 20,

4-(2-Fluoro-6-hydroxy-3-methoxy-benzoyl)-benzoesäure (3R,4R)-3-(3-Bromo-4-hydroxy-benzoylamino)-azepan-4-yl ester hydrochlorid (1:1)
4-(2-Hydroxy-5-methoxy-benzoyl)-benzoesäure (3R,4R)-3-(5-fluoro-2-hydroxy-benzoylamino)-azepan-4-yl ester hydrochlorid(1:1)

**22.** Verbindungen gemäss Anspruch 10, in denen $R^{12}$ und $R^{13}$ gemeinsam einen Rest -CH=CH-CH=CH- oder -C(OH)=CH-CH=CH-, in der die Bindung zu dem die Hydroxygruppe tragenden C-Atom durch $R^{12}$ erfolgt.

**23.** Die Verbindungen gemäss Anspruch 22,

4-(2-Fluoro-6-hydroxy-3-methoxy-benzoyl)-benzoesäure (3R,4R)-3-(3,5-dihydroxy-naphthalen-2-ylcarbonyl-amino)-azepan-4-yl ester hydrochlorid (1:1)
3-(2-Fluoro-6-hydroxy-3-methoxy-benzoyl)-benzoesäure (3R,4R)-3-((3-hydroxy-naphtalin-2-ylcarbonylamino)-azepan-4-yl ester hydrochlorid (1:1).

**24.** Die Verbindungen gemäss Anspruch 9,

4-(2-Hydroxy-5-methoxy-benzoyl)-benzoesäure (3R,4R)-3-(4-pyridinoylamino)-azepan-4-yl ester hydrochlorid (1:2)
4-(2-Fluoro-6-hydroxy-3-methoxy-benzoyl)-benzoesäure (3R,4R)-3-(4-pyridinoylamino)-azepan-4-yl ester hydrochlorid (1:2)
4-(2-Fluoro-6-hydroxy-3-methoxy-benzoyl)-benzoesäure (3RS,4RS)-4-pyridin-4-ylcarbonylamino-pyrrolidin-3-yl ester hydrochlorid (1:2)

**25.** 4-(2-Hydroxy-5-methoxy-benzoyl)-benzoesäure (3R,4R)-3-(3-acetyl-4-hydroxy-benzoylamino)-azepan-4-yl ester hydrochlorid (1:1).

**26.** Verbindungen der Formel

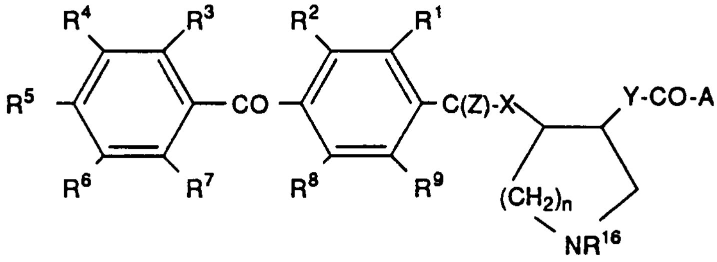

II

worin $R^{16}$ eine Schutzgruppe ist und die übrigen Symbole die in Anspruch 1 angegebene Bedeutung haben, wobei durch $R^3$, $R^4$, $R^6$, $R^7$. $R^{12}$ und $R^{13}$ dargestellte Hydroxy- und Aminogruppe in geschützter Form vorliegen können.

**27.** Die Verbindungen der Ansprüche 1-25 zur Anwendung als Heilmittel.

**28.** Pharmazeutische Präparate, enthaltend als Wirkstoff eine Verbindung der Ansprüche 1-25 und übliche pharmazeutische Hilfsstoffe.

**29.** Verwendung von Verbindungen der Ansprüche 1-25 zur Herstellung von Heilmitteln zur Therapie und Prophylaxe von durch Proteinkinasen mediierten Erkrankungen.

**30.** Verfahren zur Herstellung von Verbindungen der Ansprüche 1-25, dadurch gekennzeichnet, dass man aus einer Verbindung der Formel

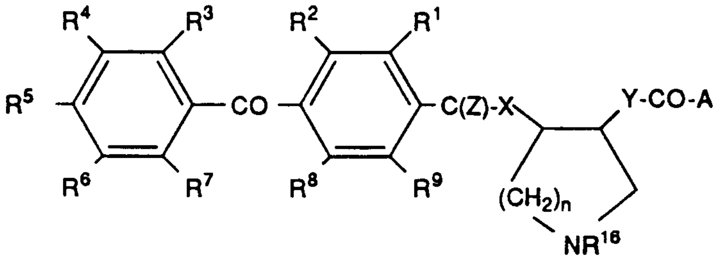

II

worin $R^{16}$ eine Schutzgruppe ist und die übrigen Symbole die in Anspruch 1 angegebene Bedeutung haben, wobei durch $R^3$, $R^4$, $R^6$, $R^7$, $R^{12}$ und $R^{13}$ dargestellte Hydroxy- und Aminogruppen in geschützter Form vorliegen können,

die Schutzgruppe $R^{16}$ und gegebenenfalls als $R^3$, $R^4$, $R^6$, $R^7$, $R^{12}$ und $R^{13}$ anwesende Hydroxy- und Aminoschutzgruppen abspaltet,
gewünschtenfalls die erhaltene Verbindung der Formel I, in der $R^{15}$ Wasserstoff darstellt, in eine Verbindung der Formel I, in der $R^{15}$ Amidino darstellt, überführt, und gewünschtenfalls eine erhaltene Verbindung der Formel I in ein pharmazeutisch anwendbares Salz überführt.

**Claims**

**1.** Compounds of the formula

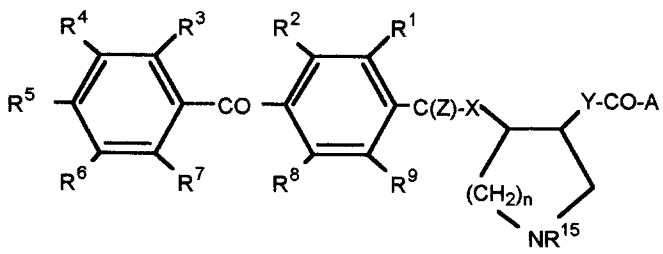

I

wherein

A signifies a residue

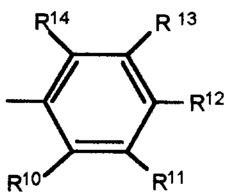

$A^1$

2-, 3- or 4-pyridyl or 2- or 3-piperazinyl, or 2-, 3- or 4-pyridyl or 2- or 3-piperazinyl substituted by one or more $C_{1-6}$-alkyl, $C_{1-6}$-alkoxy and/or hydroxy groups;

| | |
|---|---|
| X and Y | each independently signify oxygen or NH, but both do not signify NH; |
| Z | signifies oxygen or, where X is oxygen, oxygen or H,H; |
| n | is 1, 2 or 3; |
| $R^1$ | signifies hydrogen or fluorine; |
| $R^2$ | signifies hydrogen, $C_{1-6}$-alkoxy or fluorine; |
| $R^3$ | signifies hydrogen, hydroxy, $C_{1-6}$-alkoxy, fluorine, trifluoromethyl, $C_{2-7}$-alkoxycarbonyl, tetrazolyl or tetrazolyl substituted by $C_{1-6}$-alkyl, benzyl, cyano-methyl or carbamoyl-methyl; |
| $R^4$ | signifies hydrogen, hydroxy, $C_{1-6}$-alkoxy, $C_{1-6}$-alkyl, chlorine, fluorine, trifluoromethyl, acetyl, di-$C_{1-6}$-alkylamino, or $C_{1-6}$-alkoxy-$C_{1-6}$-alkyl, $C_{1-6}$-alkylthio, $C_{1-6}$-alkylsulphonyl, phenyl, pyrrolidinyl or piperidinyl; |
| $R^5$ | signifies hydrogen, $C_{1-6}$-alkoxy, fluorine or trifluoromethyl; |
| $R^6$ | signifies hydrogen, hydroxy, $C_{1-6}$-alkoxy, fluorine, 2,4-difluorophenyl, $C_{1-6}$-alkoxy-$C_{1-6}$-alkyl, $C_{1-6}$-alkanoyl, benzoyl, nitrilo, trifluoromethyl, $C_{3-6}$-cycloalkyl, 2-or 4-thiazolyl, 2-thiazolidinyl, 2-oxazolyl or 2-oxazolidinyl, 2-or 4-imidazolyl; |
| $R^7$ | signifies hydrogen, hydroxy, $C_{1-6}$-alkoxy, carboxy, amino or fluorine; |
| $R^6$ and $R^7$ | together signify a residue -N=CH-CH=N- or $-N(CH_3)CH_2CH_2N(CH_3)-$; |
| $R^8$ | signifies hydrogen, $C_{1-6}$-alkoxy, $C_{1-6}$-alkyl or fluorine; |
| $R^9$ | signifies hydrogen or fluorine; |
| $R^{10}$ | signifies hydrogen, hydroxy, $C_{1-6}$-alkoxy or $C_{1-6}$-alkyl; |
| $R^{11}$ | signifies hydrogen, $C_{1-6}$-alkoxy, $C_{1-6}$-alkyl, fluorine or bromine; |
| $R^{12}$ | signifies hydrogen, hydroxy, $C_{1-6}$-alkoxy, carboxy, $C_{2-7}$-alkoxycarbonyl or amino; |
| $R^{13}$ | signifies hydrogen, hydroxy, $C_{1-6}$-alkoxy, $C_{1-6}$-alkyl, acetyl or fluorine; |
| $R^{14}$ | signifies hydrogen, $C_{1-6}$-alkyl or fluorine; |
| $R^{15}$ | signifies hydrogen or amidino; |
| $R^3$ and $R^4$ | together signify a residue -CH=CH-CH=CH- or ethylenedioxy; |
| $R^4$ and $R^5$ | together signify a residue -CH=CH-CH=CH-, tetramethylene, methylenedioxy, ethylenedioxy or a residue -N=CH-CH=CH- or a residue (a) |

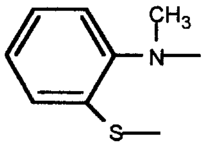

(a)

in which the bonding to the S is effected via $R^5$; and

$R^{12}$ and $R^{13}$ together signify a residue -CH=CH-CH=CH- or -C(OH)=CH-CH=CH- in which the bonding to the C atom carrying the hydroxy group is effected via

and pharmaceutically acceptable salts thereof.

**2.** Compounds according to claim 1, in which $R^1$ and $R^9$ represent hydrogen, $R^2$ and $R^8$ represent hydrogen or fluorine, and $R^7$ represents hydrogen, hydroxy, $C_{1-6}$-alkoxy, amino or fluorine.

**3.** Compounds according to claim 1 or 2, in which X is oxygen and Y is NH.

**4.** Compounds according to claims 1-3, in which Z is oxygen.

**5.** Compounds according to claims 1-4, in which n = 3.

**6.** Compounds according to claims 1-5, in which $R^1$, $R^2$, $R^8$ and $R^9$ are hydrogen.

**7.** Compounds according to claims 1-6, in which $R^7$ is hydroxy.

**8.** Compounds according to claims 1-7, in which $R^3$ signifies hydrogen, $C_{1-6}$-alkyl, $C_{1-6}$-alkoxy, fluorine or cyanomethyltetrazolyl; $R^4$ signifies hydrogen, $C_{1-6}$-alkyl, $C_{1-6}$-alkoxy, $C_{1-6}$-alkylthio, $C_{1-6}$-alkoxy-$C_{1-6}$-alkyl, di($C_{1-6}$-alkyl)amino, $C_{2-7}$-alkanoyl, phenyl, pyrrolidino or fluorine; $R^5$ signifies hydrogen, $C_{1-6}$-alkoxy, fluorine or trifluoromethyl; $R^6$ signifies hydrogen; $R^4$ and $R^5$ together signify tetramethylene, methylenedioxy, ethylenedioxy or a residue -CH=CH-CH=CH-, -CH=CH-CH=N- or

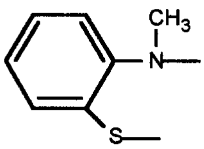

(a); and

$R^3$ and $R^4$ together signify ethylenedioxy.

**9.** Compounds according to claims 1-8, in which A is 4-pyridyl.

**10.** Compounds according to claims 1-8, in which A is a residue $A^1$.

**11.** Compounds according to claim 10, in which $R^{10}$ represents hydrogen, hydroxy or $C_{1-6}$-alkyl; $R^{11}$ represents hydrogen, $C_{1-6}$-alkyl, $C_{1-6}$-alkoxy or bromine; $R^{12}$ represents hydrogen, hydroxy or $C_{1-6}$-alkoxy; $R^{13}$ represents hydrogen, $C_{1-6}$-alkyl, $C_{1-6}$-alkoxy, fluorine or acetyl; $R^{14}$ represents hydrogen or $C_{1-6}$-alkyl; or $R^{12}$ and $R^{13}$ together represent a residue -CH=CH-CH=CH- or -C(OH)=CH-CH=CH- in which the bonding to the C atom carrying the hydroxy group is effected via $R^{12}$.

**12.** Compounds according to claims 1-11, in which $R^{15}$ is hydrogen.

**13.** Compounds according to claim 10, in which $R^{12}$ is hydroxy and at least one of residues $R^{10}$, $R^{11}$, $R^{13}$ and $R^{14}$ is $C_{1-6}$-alkyl.

**14.** The compounds according to claim 13,

4-(2-fluoro-6-hydroxy-3-methoxy-benzoyl)-benzoic acid (3R,4R)-3-(4-hydroxy-2,3,6-trimethyl-benzoylamino)-azepan-4-yl ester hydrochloride (1:1)

4-(2,3-difluoro-6-hydroxy-benzoyl)-benzoic acid (3R,4R)-3-(4-hydroxy-2,3,6-trimethyl-benzoylamino)-azepan-4-yl ester hydrochloride (1:1)

4-(2,3-difluoro-6-hydroxy-benzoyl)-benzoic acid (3R,4R)-3-(4-hydroxy-3,5-diisopropyl-benzoylamino)-azepan-4-yl ester hydrochloride (1:1)

4-(2-hydroxy-5-dimethylamino-benzoyl)-benzoic acid (3R,4R)-3-(4-hydroxy-3,5-diisopropyl-benzoylamino)-azepan-4-yl ester hydrochloride (1:2)

4-(2-hydroxy-5-dimethylamino-benzoyl)-benzoic acid (3R,4R)-3-(4-hydroxy-3,5-dimethyl-benzoylamino)-azepan-4-yl ester hydrochloride (1:2)

4-(2-fluoro-6-hydroxy-4-methoxy-benzoyl)-benzoic acid (3R,4R)-3-(4-hydroxy-3,5-diisopropyl-benzoylamino)-azepan-4-yl ester hydrochloride (1:1)

4-(2,3-difluoro-6-hydroxy-benzoyl)-benzoic acid (3R,4R)-3-(4-hydroxy-3,5-dimethyl-benzoylamino)-azepan-4-yl ester hydrochloride (1:1)

4-(2,4-difluoro-6-hydroxy-benzoyl)-benzoic acid (3R,4R)-3-(4-hydroxy-3,5-dimethyl-benzoylamino)-azepan-4-yl ester hydrochloride (1:1)

4-(6-hydroxy-quinolin-7-ylcarbonyl)-benzoic acid (3R,4R)-3-(4-hydroxy-3,5-dimethyl-benzoylamino)-azepan-4-yl ester hydrochloride (1:1)

4-(2-fluoro-6-hydroxy-3-methoxy-benzoyl)-benzoic acid (3R,4R)-3-(3,5-diethyl-4-hydroxy-benzoylamino)-azepan-4-yl ester hydrochloride (1:1)

4-(2,3-difluoro-6-hydroxy-benzoyl)-benzoic acid (3R,4R)-3- (3,5-diethyl-4-hydroxy-benzoylamino)-azepan-4-yl ester hydrochloride (1:1)

4-(2-hydroxy-5-dimethylamino-benzoyl)-benzoic acid (3R,4R)-3-(3,5-diethyl-4-hydroxy-benzoylamino)-azepan-4-yl ester hydrochloride (1:2)

4-(2-hydroxy-5-methoxy-benzoyl)-benzoic acid (3R,4R)-3-(4-hydroxy-3,5-dimethyl-benzoylamino)-azepan-4-yl ester hydrochloride (1:1)

4-(6-hydroxy-1,3-benzodioxol-5-ylcarbonyl)-benzoic acid (3R,4R)-3-(4-hydroxy-3,5-dimethyl-benzoylamino)-azepan-4-yl ester hydrochloride (1:1)

4-(6-hydroxy-1,4-benzodioxin-1-yl-carbonyl)-benzoic acid (3R, 4R)-3-(4-hydroxy-3,5-dimethyl-benzoylamino)-azepan-4-yl ester hydrochloride (1:1)

4-(2-fluoro-6-hydroxy-3-dimethylamino-benzoyl)-benzoic acid (3R,4R)-3-(4-hydroxy-3,5-dimethyl-benzoylamino)-azepan-4-yl ester hydrochloride (1:2)

4-(5-acetyl-2-hydroxy-benzoyl)-benzoic acid (3R,4R)-3-(4-hydroxy-3,5-dimethoxy-benzoylamino)-azepan-4-yl ester hydrochloride (1:1)

4-(2-hydroxy-5-methoxy-benzoyl)-benzoic acid (3R,4R)-3-(3-tert-butyl-4-hydroxy-benzoylamino)-azepan-4-yl ester hydrochloride (1:1)

4-(2-hydroxy-5-methoxy-benzoyl)-benzoic acid (3R,4R)-3-(4-hydroxy-3-isopropyl-benzoylamino)-azepan-4-yl ester hydrochloride (1:1)

4-(2-hydroxy-5-methoxy-benzoyl)-benzoic acid (3R,4R)-3-(4-hydroxy-3-sec-butyl-benzoylamino)-azepan-4-yl ester hydrochloride (1:1)

4-(2-fluoro-3-isopropyl-6-hydroxy-benzoyl)-benzoic acid (3R,4R)-3-(4-hydroxy-3,5-dimethyl-benzoylamino)-azepan-4-yl ester hydrochloride (1:1)

4-(2-fluoro-6-hydroxy-3-pyrrolidin-1-yl-benzoyl)-benzoic acid (3R,4R)-3-(4-hydroxy-3,5-dimethyl-benzoylamino)-azepan-4-yl ester hydrochloride (1:2)

4-(3-ethyl-2-fluoro-6-hydroxy-benzoyl)-benzoic acid (3R, 4R)-3-(4-hydroxy-benzoylamino)-azepan-4-yl ester hydrochloride (1:1)

4-(2-fluoro-6-hydroxy-3-piperidin-1-yl-benzoyl)-benzoic acid (3R,4R)-3-(4-hydroxy-3,5-dimethyl-benzoylamino)-azepan-4-yl ester hydrochloride (1:2)

4-(2-hydroxy-5-methoxy-benzoyl)-benzoic acid (3RS, 4RS)-4-(4-hydroxy-3,5-dimethyl-benzoylamino)-pyrrolidin-3-yl ester hydrochloride (1:1)

4-(2-hydroxy-5-methoxy-benzoyl)-benzoic acid (3RS, 4SR)-4-(4-hydroxy-3,5-dimethyl-benzoylamino)-pyrrolidin-3-yl ester hydrochloride (1:1)

4-(2-fluoro-6-hydroxy-3-methoxy-benzoyl)-benzoic acid (3RS,4RS)-4-(4-hydroxy-3,5-dimethyl-benzylamino)-pyrrolidin-3-yl ester hydrochloride (1:1)

4-(6-hydroxy-1,3-benzodioxol-5-ylcarbonyl)-benzoic acid (3RS,4RS)-4-(4-hydroxy-3,5-dimethyl-benzoylamino)-pyrrolidin-3-yl ester hydrochloride (1:1)

4-(2-hydroxy-5-methoxy-benzoyl)-benzoic acid (3RS,4SR)-1-(amino-imino-methyl)-4-(4-hydroxy-3,5-dimethyl-benzoylamino)-pyrrolidin-3-yl ester

**15.** The compounds according to claim 13,

4-(2-fluoro-6-hydroxy-3-methoxy-benzoyl)-benzoic acid (3R,4R)-3-(4-hydroxy-3,5-dimethyl-benzoylamino)-azepan-4-yl ester hydrochloride (1:1)

**16.** Compounds according to claim 10, in which $R^{12}$ is hydroxy and $R^{10}$, $R^{11}$, $R^{13}$ and $R^{14}$ are hydrogen.

**17.** The compounds according to claims 16,

4-(2-fluoro-6-hydroxy-3-methoxy-benzoyl)-benzoic acid (3R,4R)-3-(4-hydroxy-benzoylamino)-azepan-4-yl ester hydrochloride (1:1)

3-(3-hydroxy-5,6,7,8-tetrahydro-naphtalen-2-ylcarbonyl)-benzoylamino) -azepan-4-yl ester hydrochloride (1:1)

4-(2-hydroxy-5-dimethylamino-benzoyl)-benzoic acid (3R,4R)-3-(4-hydroxy-benzoylamino)-azepan-4-yl ester hydrochloride (1:2)

4-(7-hydroxy-1,4-benzodioxin-6-ylcarbonyl)-benzoic acid (3R,4R)-3-(4-hydroxy-benzoylamino)-azepan-4-yl ester hydrochloride (1:1)

4-[2-hydroxy-5-(1-methoxy-ethyl)-benzoyl]-benzoic acid (3R,4R)-3-(4-hydroxy-benzoylamino)-azepan-4-yl ester hydrochloride (1:1)

4-(6-hydroxy-quinolin-7-ylcarbonyl)-benzoic acid (3R,4R)-3-(4-hydroxy-benzoylamino)-azepan-4-yl ester hydrochloride (1:1)

4-(4-hydroxy-biphenyl-3-ylcarbonyl)-benzoic acid (3R, 4R)-3-(4-hydroxy-benzoylamino)-azepan-4-yl ester hydrochloride (1:1)

4-(5-acetyl-2-hydroxy-benzoyl)-benzoic acid (3R, 4R)-3-(4-hydroxy-benzoylamino)-azepan-4-yl ester hydrochloride (1:1)

4-(2-hydroxy-10-methyl-phenothiazin-1-ylcarbonyl)-benzoic acid (3R, 4R)-3-(4-hydroxy-benzoylamino)-azepan-4-yl ester hydrochloride (1:2)

4-(2-hydroxy-5-methyl-benzoyl)-benzoic acid (3R, 4R)-3-(4-hydroxy-benzoylamino)-azepan-4-yl ester hydrochloride (1:1)

4-(2-hydroxy-5-isopropyl-benzoyl)-benzoic acid (3R, 4R)-3-(4-hydroxy-benzoylamino)-azepan-4-yl ester hydrochloride (1:1)

4-(2-fluoro-3-dimethylamino-6-hydroxy-benzoyl)-benzoic acid (3R,4R)-3-(4-hydroxy-benzoylamino)-azepan-4-yl ester hydrochloride (1:2)

4-(2-fluoro-6-hydroxy-3-pyrrolidin-1-yl-benzoyl)-benzoic acid (3R,4R)-3-(4-hydroxy-benzoylamino)-azepan-4-yl ester hydrochloride (1:2)

4-(2-fluoro-6-hydroxy-3-methyl-benzoyl)-benzoic acid (3R, 4R)-3-(4-hydroxy-benzoyl-amino)-azepan-4-yl ester hydrochloride (1:1)

4-(2-fluoro-6-hydroxy-4-methoxy-benzoyl)-benzoic acid (3R,4R)-3-(4-hydroxy-benzoyl-amino)-azepan-4-yl ester hydrochloride (1:1)

4-(3-hydroxy-naphthalen-2-ylcarbonyl)-benzoic acid (3R,4R)-3-(4-hydroxy-benzoylamino)-azepan-4-yl ester hydrochloride (1:1)

3,5-difluoro-4-(2-hydroxy-5-methoxy-benzoyl)-benzoic acid (3R,4R)-3-(4-hydroxy-benzoylamino)-azepan-4-yl ester hydrochloride (1:1)

4-(2-hydroxy-5-methylthio-benzoyl)-benzoic acid (3R,4R)-3-(4-hydroxy-benzoylamino)-azepan-4-yl ester hydrochloride (1:1)

4-(2-hydroxy-5-methoxy-benzoyl)-benzoic acid (3RS, 4RS)-4-(4-hydroxy-benzoylamino)-pyrrolidin-3-yl ester hydrochloride (1:1)

4-(2-hydroxy-5-methoxy-benzoyl)-benzoic acid (3RS, 4RS)-4-(4-hydroxy-benzoylamino)-pyrrolidin-3-yl ester hydrochloride (1:1)

(3R,4R)-4-hydroxy-N-[4-[4-(2-hydroxy-5-methoxy-benzoyl)benzyloxy]-azepan-3-yl]-benzamide hydrochloride (1:1)

**18.** Compounds according to claim 10, in which at least one of the residues $R^{10}$-$R^{14}$ is $C_{1-6}$-alkoxy.

**19.** The compounds according to claim 18,

4-(2-fluoro-6-hydroxy-3-methoxy-benzoyl)-benzoic acid (3R,4R)-3-(4-hydroxy-3-methoxy-benzoylamino)-azepan-4-yl ester hydrochloride (1:1)

4-(2-fluoro-6-hydroxy-3-methoxy-benzoyl)-benzoic acid (3R,4R)-3-(4-hydroxy-3,5-dimethoxy-benzoylamino)-azepan-4-yl ester hydrochloride (1:1)

4-(3-hydroxy-naphtalen-2-ylcarbonyl)-benzoic acid (3R,4R)-3-(4-hydroxy-3,5-dimethoxy-benzoylamino)-azepan-4-yl ester hydrochloride (1:1)

4-(2-hydroxy-5-methoxy-benzoyl)-benzoic acid (3R,4R)-3-(3,5-dimethoxy-benzoylamino)-azepan-4-yl ester hydrochloride (1:1)

4-(2-fluoro-6-hydroxy-3-methoxy-benzoyl)-benzoic acid (3R,4R)-3- (2-hydroxy-3-methoxy-benzoylamino)-azepan-4-yl ester hydrochloride (1:1)

4-(2-fluoro-6-hydroxy-3-methoxy-benzoyl)-benzoic acid (3R,4R)-3-(3,5-dimethoxy-benzoylamino)-azepan-4-yl ester hydrochloride (1:1)

4-(2-hydroxy-5-methoxy-benzoyl)-benzoic acid (3R,4R)-3-(3,4,5-trimethoxy-benzoyl-amino)-azepan-4-yl ester hydrochloride (1:1)

4-(2-hydroxy-5-dimethylamino-benzoyl)-benzoic acid (3R,4R)-3-(4-hydroxy-3-methoxy-benzoylamino)-azepan-4-yl ester hydrochloride (1:2)

4-(2-hydroxy-5-methoxy-benzoyl)-benzoic acid (3R,4R)-3-(3,5-diethoxy-benzoylamino)-azepan-4-yl ester hydrochloride (1:1)

4-(2-fluoro-6-hydroxy-3-dimethylamino-benzoyl)-benzoic acid (3R,4R)-3-(4-hydroxy-3,5-dimethoxy-benzoylamino)-azepan-4-yl ester hydrochloride (1:2)

4-(2-fluoro-6-hydroxy-benzoyl)-benzoic acid (3R,4R)-3-(4-hydroxy-3-methoxy-benzoyl-amino)-azepan-4-yl ester hydrochloride (1:1)

4-[4-(2-fluoro-6-hydroxy)-benzoyl]-benzoic acid (3R,4R)-3-(2-hydroxy-5-methoxy-benzoylamino)-azepan-4-yl ester hydrochloride (1:1)

4-(2-fluoro-6-hydroxy-4-methoxy-benzoyl)-benzoic acid (3R,4R)-3-(4-hydroxy-3,5-dimethoxy-benzoylamino)-azepan-4-yl ester hydrochloride (1:1)

4-(2-fluoro-6-hydroxy-benzoyl)-benzoic acid (3R,4R)-3-(4-hydroxy-3,5-dimethoxy-benzoylamino)-azepan-4-yl ester hydrochloride (1:1)

4-(2,3-difluoro-6-hydroxy-benzoyl)-benzoic acid (3R,4R)-3-(4-hydroxy-3,5-dimethoxy-benzoylamino)-azepan-4-yl ester hydrochloride (1:1)

4-(2,4-difluoro-6-hydroxy-benzoyl)-benzoic acid (3R,4R)-3-(4-hydroxy-3,5-dimethoxy-benzoylamino)-azepan-4-yl ester hydrochloride (1:1)

(3R,4R)-2-hydroxy-N-[4-[4-(2-hydroxy-5-methoxy-benzoyl]-azepan-3-yl]-5-methoxy-benzamide hydrochloride (1:1)

**20.** Compounds according to claim 10, in which at least one of the residues $R^{11}$, $R^{13}$ and $R^{14}$ is fluorine or $R^{11}$ is bromine.

**21.** The compounds according to claim 20,

4-(2-fluoro-6-hydroxy-3-methoxy-benzoyl)-benzoic acid (3R,4R)-3-(3-bromo-4-hydroxy-benzoylamino)-azepan-4-yl ester hydrochloride (1:1)

4-(2-hydroxy-5-methoxy-benzoyl)-benzoic acid (3R,4R)-3-(5-fluoro-2-hydroxy-benzoyl-amino)-azepan-4-yl ester hydrochloride (1:1)

**22.** Compounds according to claim 10, in which $R^{12}$ and $R^{13}$ together are a residue -CH=CH-CH=CH- or -C(OH)=CH-CH=CH- in which the bonding to the C atom carrying the hydroxy group is effected via $R^{12}$.

**23.** The compounds according to claim 22,

4-(2-fluoro-6-hydroxy-3-methoxy-benzoyl)-benzoic acid (3R,4R)-3- (3,5-dihydroxy-naphthalen-2-ylcarbonylamino)-azepan-4-yl ester hydrochloride (1:1)

3-(2-fluoro-6-hydroxy-3-methoxy-benzoyl)-benzoic acid (3R,4R)-3-((3-hydroxy-naphtalen-2-ylcarbonylamino)-azepan-4-yl ester hydrochloride (1:1).

**24.** The compounds according to claim 9,

4-(2-hydroxy-5-methoxy-benzoyl)-benzoic acid (3R,4R)-3-(4-pyridinoylamino)-azepan-4-yl ester hydrochloride (1:2)

4-(2-fluoro-6-hydroxy-3-methoxy-benzoyl)-benzoic acid (3R,4R)-3-(4-pyridinoylamino)-azepan-4-yl ester hydrochloride (1:2)

4-(2-fluoro-6-hydroxy-3-methoxy-benzoyl)-benzoic acid (3RS,4RS)-4-pyridin-4-ylcarbonyl-amino-pyrrolidin-3-yl ester hydrochloride (1:2)

**25.** 4-(2-Hydroxy-5-methoxy-benzoyl)-benzoic acid (3R,4R)-3-(3-acetyl-4-hydroxy-benzoylamino)-azepan-4-yl ester hydrochloride (1:1)

**26.** Compounds of the formula

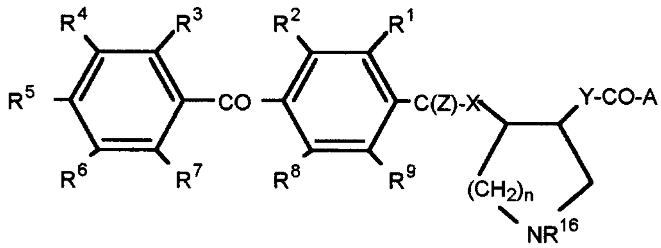

II

wherein $R^{16}$ is a protecting group and the remaining symbols have the significance given in claim 1, whereby hydroxy and amino groups represented by $R^3$, $R^4$, $R^6$, $R^7$. $R^{12}$ and $R^{13}$ can be present in protected form.

**27.** The compounds of claims 1-25 for use as medicaments.

**28.** Pharmaceutical preparations containing a compound of claims 1-25 as the active ingredient and usual pharmaceutical adjuvants.

**29.** The use of compounds of claims 1-25 for the production of medicaments for the therapy and prophylaxis of illnesses which are mediated by protein kinases.

**30.** A process for the manufacture of compounds of claims 1-25, characterized by cleaving off the protecting group $R^{16}$ and, if necessary, hydroxy and amino protecting groups present as $R^3$, $R^4$, $R^6$, $R^7$, $R^{12}$ and $R^{13}$ from a compound of the formula

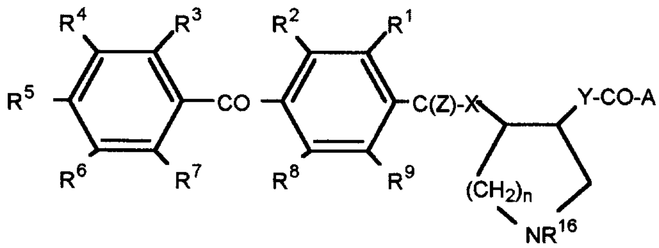

II

wherein $R^{16}$ is a protecting group and the remaining symbols have the significance given in claim 1, whereby hydroxy and amino groups represented by $R^3$, $R^4$, $R^6$, $R^7$, $R^{12}$ and $R^{13}$ can be present in protected form,

if desired, converting the compound of formula I obtained in which $R^{15}$ represents hydrogen into a compound of formula I in which $R^{15}$ represents amidino and, if desired, converting a compound of formula I obtained into a salt.

**Revendications**

**1.** Composés de formule

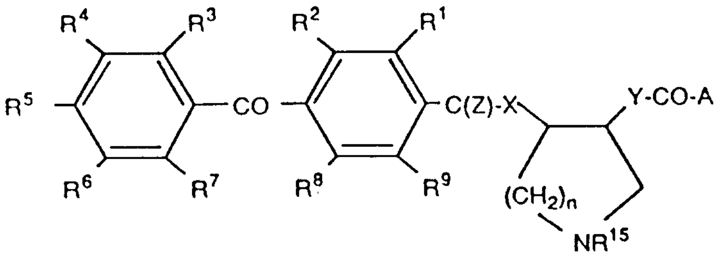

I

dans laquelle

A représente un radical

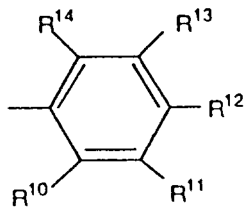

A 1

2-, 3- ou 4-pyridyle ou 2- ou 3-pipérazinyle, ou 2-, 3- ou 4-pyridyle ou 2- ou 3-pipérazinyle substitué par un ou plusieurs groupes alkyle en $C_{1-6}$, alcoxy en $C_{1-6}$ et/ou hydroxy;

X et Y représentent, indépendamment l'un de l'autre, un atome d'oxygène ou NH, mais ne représentent pas l'un et l'autre NH;

Z représente un atome d'oxygène ou, lorsque X est un atome d'oxygène, un atome d'oxygène ou H,H;

n est 1, 2 ou 3;

$R^1$ est un atome d'hydrogène ou de fluor;

$R^2$ est un atome d'hydrogène ou de fluor ou un groupe alcoxy en $C_{1-6}$;

$R^3$ représente un atome d'hydrogène ou de fluor ou un groupe hydroxy, alcoxy en $C_{1-6}$, trifluorométhyle, alcoxycarbonyle en $C_{2-7}$, tétrazolyle ou un radical tétrazolyle substitué par un groupe alkyle en $C_{1-6}$, benzyle, cyanométhyle ou carbamoylméthyle;

$R^4$ représente un atome d'hydrogène, de chlore ou de fluor, ou un groupe hydroxy, alcoxy en $C_{1-6}$, alkyle en $C_{1-6}$, trifluorométhyle, acétyle, dialkyl($C_{1-6}$)amino ou alcoxy($C_{1-6}$)-alkyle($C_{1-6}$), alkyl($C_{1-6}$)thio, alkyl($C_{1-6}$)sulfonyle, phényle, pyrrolidinyle ou pipéridinyle;

$R^5$ représente un atome d'hydrogène ou de fluor ou un groupe alcoxy en $C_{1-6}$ ou trifluorométhyle;

$R^6$ représente un atome d'hydrogène ou de fluor ou un groupe hydroxy, alcoxy en $C_{1-6}$, 2,4-difluorophényle, alcoxy($C_{1-6}$)-alkyle($C_{1-6}$), alcanoyle en $C_{1-6}$, benzoyle, nitrilo, trifluorométhyle, cycloalkyle en $C_{3-6}$, 2- ou 4-thiazolyle, 2-thiazolidinyle, 2-oxazolyle ou 2-oxazolidinyle, 2- ou 4-imidazolyle;

$R^7$ représente un atome d'hydrogène ou de fluor ou un groupe hydroxy, alcoxy en $C_{1-6}$, carboxy ou amino;

$R^6$ et $R^7$ forment ensemble un radical -N=CH-CH=N- ou $-N(CH_3)CH_2CH_2N(CH_3)-$;

$R^8$ représente un atome d'hydrogène ou de fluor ou un groupe alcoxy en $C_{1-6}$ ou alkyle en $C_{1-6}$;

$R^9$ représente un atome d'hydrogène ou de fluor;

$R^{10}$ représente un atome d'hydrogène ou un groupe hydroxy, alcoxy en $C_{1-6}$ ou alkyle en $C_{1-6}$,

$R^{11}$ représente un atome d'hydrogène, de fluor ou de brome, ou un groupe alcoxy en $C_{1-6}$ ou alkyle en

$C_{1-6}$;

$R^{12}$ représente un atome d'hydrogène ou un groupe hydroxy, alcoxy en $C_{1-6}$, carboxy, alcoxycarbonyle en $C_{2-7}$ ou amino;

$R^{13}$ représente un atome d'hydrogène ou de fluor ou un groupe hydroxy, alcoxy en $C_{1-6}$, alkyle en $C_{1-6}$ ou acétyle;

$R^{14}$ représente un atome d'hydrogène ou de fluor ou un groupe alkyle en $C_{1-6}$;

$R^{15}$ représente un atome d'hydrogène ou le groupe amidino;

$R^3$ et $R^4$ forment ensemble un radical -CH=CH-CH=CH- ou éthylènedioxy;

$R^4$ et $R^5$ forment ensemble un radical -CH=CH-CH=CH-, tétraméthylène, méthylènedioxy, éthylènedioxy ou un radical -N=CH-CH=CH- ou un radical (a)

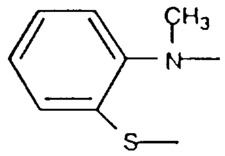

(a)

dans lequel la liaison avec S s'effectue par $R^5$; et

$R^{12}$ et $R^{13}$ forment ensemble un radical -CH=CH-CH=CH- ou -C(OH)=CH-CH=CH-, dans lequel la liaison avec l'atome de carbone portant le groupe hydroxy s'effectue par $R^{12}$;

et leurs sels pharmaceutiquement acceptables.

**2.** Composés selon la revendication 1, dans lesquels $R^1$ et $R^9$ représentent chacun un atome d'hydrogène, $R^2$ et $R^8$ représentent chacun un atome d'hydrogène ou de fluor, et $R^7$ représente un atome d'hydrogène ou de fluor ou un groupe hydroxy, alcoxy en $C_{1-6}$ ou amino.

**3.** Composés selon la revendication 1 ou 2, dans lesquels X représente un atome d'oxygène et Y est NH.

**4.** Composés selon les revendications 1 à 3, dans lesquels Z est un atome d'oxygène.

**5.** Composés selon les revendications 1 à 4, dans lesquels n est égal à 3.

**6.** Composés selon les revendications 1 à 5, dans lesquels $R^1$, $R^2$, $R^8$ et $R^9$ sont des atomes d'hydrogène.

**7.** Composés selon les revendications 1 à 6, dans lesquels $R^7$ est le groupe hydroxy.

**8.** Composés selon les revendications 1 à 7, dans lesquels $R^3$ représente un atome d'hydrogène ou de fluor ou un groupe alkyle en $C_{1-6}$, alcoxy en $C_{1-6}$ ou cyanométhyl-tétrazolyle; $R^4$ représente un atome d'hydrogène ou de fluor ou un groupe alkyle en $C_{1-6}$, alcoxy en $C_{1-6}$, alkyl($C_{1-6}$)thio, alcoxy($C_{1-6}$)-alkyle($C_{1-6}$), dialkyl($C_{1-6}$)amino, alcanoyle en $C_{2-7}$, phényle ou pyrrolidino; $R^5$ représente un atome d'hydrogène ou de fluor ou un groupe alcoxy en $C_{1-6}$ ou trifluorométhyle; $R^6$ représente un atome d'hydrogène; $R^4$ et $R^5$ forment ensemble le groupe tétraméthylène, méthylènedioxy, éthylènedioxy ou un radical -CH=CH-CH=CH-, -CH=CH-CH=N- ou

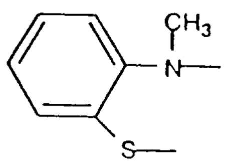

(a);

et $R^3$ et $R^4$ représentent ensemble le groupe éthylènedioxy.

**9.** Composés selon les revendications 1 à 8, dans lesquels A est le groupe 4-pyridyle.

**10.** Composés selon les revendications 1 à 8, dans lesquels A est un radical $A^1$.

**11.** Composés selon la revendication 10, dans lesquels $R^{10}$ représente un atome d'hydrogène ou un groupe hydroxy ou alkyle en $C_{1-6}$; $R^{11}$ représente un atome d'hydrogène ou de brome ou un groupe alkyle en $C_{1-6}$ ou alcoxy en $C_{1-6}$; $R^{12}$ représente un atome d'hydrogène ou un groupe hydroxy ou alcoxy en $C_{1-6}$; $R^{13}$ représente un atome d'hydrogène ou de fluor ou un groupe alkyle en $C_{1-6}$, alcoxy en $C_{1-6}$ ou acétyle; $R^{14}$ représente un atome d'hydrogène ou un groupe alkyle en $C_{1-6}$; ou $R^{12}$ et $R^{13}$ forment ensemble un radical -CH=CH-CH=CH- ou -C(OH)=CH-CH=CH-, dans lequel la liaison avec l'atome de carbone portant le groupe hydroxy s'effectue par $R^{12}$.

**12.** Composés selon les revendications 1 à 11, dans lesquels $R^{15}$ est un atome d'hydrogène.

**13.** Composés selon la revendication 10, dans lesquels $R^{12}$ est le groupe hydroxy et au moins l'un des radicaux $R^{10}$, $R^{11}$, $R^{13}$ et $R^{14}$ est un groupe alkyle en $C_{1-6}$.

**14.** Composés selon la revendication 13, qui sont les composés suivants:

4-(2-fluoro-6-hydroxy-3-méthoxybenzoyl)benzoate de (3R,4R)-3-(4-hydroxy-2,3,6-triméthylbenzoylamino)azépan-4-yle chlorhydrate (1:1),
4-(2,3-difluoro-6-hydroxybenzoyl)benzoate de (3R,4R)-3-(4-hydroxy-2,3,6-triméthylbenzoylamino)azépan-4-yle chlorhydrate (1:1),
4-(2,3-difluoro-6-hydroxybenzoyl)benzoate de (3R,4R)-3-(4-hydroxy-3,5-diisopropylbenzoylamino)azépan-4-yle chlorhydrate (1:1),
4-(2-hydroxy-5-diméthylaminobenzoyl)benzoate de (3R,4R)-3-(4-hydroxy-3,5-diisopropylbenzoylamino)azépan-4-yle chlorhydrate (1:2),
4-(2-hydroxy-5-diméthylaminobenzoyl)benzoate de (3R,4R)-3-(4-hydroxy-3,5-diméthylbenzoylamino)azépan-4-yle chlorhydrate (1:2),
4-(2-fluoro-6-hydroxy-4-méthoxybenzoyl)benzoate de (3R,4R)-3-(4-hydroxy-3,5-diisopropylbenzoylamino)azépan-4-yle chlorhydrate (1:1),
4-(2,3-difluoro-6-hydroxybenzoyl)benzoate de (3R,4R)-3-(4-hydroxy-3,5-diméthylbenzoylamino)azépan-4-yle chlorhydrate (1:1),
4-(2,4-difluoro-6-hydroxybenzoyl)benzoate de (3R,4R)-3-(4-hydroxy-3,5-diméthylbenzoylamino)azépan-4-yle chlorhydrate (1:1),
4-(6-hydroxyquinolin-7-ylcarbonyl)benzoate de (3R,4R)-3-(4-hydroxy-3,5-diméthylbenzoylamino)azépan-4-yle chlorhydrate (1:1),
4-(2-fluoro-6-hydroxy-3-méthoxybenzoyl)benzoate de (3R,4R)-3-(3,5-diéthyl-4-hydroxybenzoylamino)azépan-4-yle chlorhydrate (1:1),
4-(2,3-difluoro-6-hydroxybenzoyl)benzoate de (3R,4R)-3-(3,5-diéthyl-4-hydroxybenzoylamino)azépan-4-yle chlorhydrate (1:1),
4-(2-hydroxy-5-diméthylaminobenzoyl)benzoate de (3R,4R)-3-(3,5-diéthyl-4-hydroxybenzoylamino)azépan-4-yle chlorhydrate (1:2),
4-(2-hydroxy-5-méthoxybenzoyl)benzoate de (3R,4R)-3-(4-hydroxy-3,5-diméthylbenzoylamino)azépan-4-yle chlorhydrate (1:1),
4-(6-hydroxy-1,3-benzodioxol-5-ylcarbonyl)benzoate de (3R,4R)-3-(4-hydroxy-3,5-diméthylbenzoylamino)azépan-4-yle chlorhydrate (1:1),
4-(6-hydroxy-1,4-benzodioxin-1-ylcarbonyl)benzoate de (3R,4R)-3-(4-hydroxy-3,5-diméthylbenzoylamino)azépan-4-yle chlorhydrate (1:1),
4-(2-fluoro-6-hydroxy-3-diméthylaminobenzoyl)benzoate de (3R,4R)-3-(4-hydroxy-3,5-diméthylbenzoylamino)azépan-4-yle chlorhydrate (1:2),
4-(5-acétyl-2-hydroxybenzoyl)benzoate de (3R,4R)-3-(4-hydroxy-3,5-diméthoxybenzoylamino)azépan-4-yle chlorhydrate (1:1),
4-(2-hydroxy-5-méthoxybenzoyl)benzoate de (3R,4R)-3-(3-tert-butyl-4-hydroxybenzoylamino)azépan-4-yle chlorhydrate (1:1),
4-(2-hydroxy-5-méthoxybenzoyl)benzoate de (3R,4R)-3-(4-hydroxy-3-isopropylbenzoylamino)azépan-4-yle chlorhydrate (1:1),
4-(2-hydroxy-5-méthoxybenzoyl)benzoate de (3R,4R)-3-(4-hydroxy-3-sec-butylbenzoylamino)azépan-4-yle chlorhydrate (1:1),
4-(2-fluoro-3-isopropyl-6-hydroxybenzoyl)benzoate de (3R,4R)-3-(4-hydroxy-3,5-diméthylbenzoylamino)azépan-4-yle chlorhydrate (1:1),

4-(2-fluoro-6-hydroxy-3-pyrrolidin-1-ylbenzoyl)benzoate de (3R,4R)-3-(4-hydroxy-3,5-diméthylbenzoylamino)azépan-4-yle chlorhydrate (1:2),
4-(3-éthyl-2-fluoro-6-hydroxybenzoyl)benzoate de (3R,4R)-3-(4-hydroxybenzoylamino)azépan-4-yle chlorhydrate (1:1),
4-(2-fluoro-6-hydroxy-3-pipéridin-1-ylbenzoyl)benzoate de (3R,4R)-3-(4-hydroxy-3,5-diméthylbenzoylamino)azépan-4-yle chlorhydrate (1:2),
4-(2-hydroxy-5-méthoxybenzoyl)benzoate de (3RS,4RS)-4-(4-hydroxy-3,5-diméthylbenzoylamino)pyrrolidin-3-yle chlorhydrate (1:1),
4-(2-hydroxy-5-méthoxybenzoyl)benzoate de (3RS,4SR)-4-(4-hydroxy-3,5-diméthylbenzoylamino)pyrrolidin-3-yle chlorhydrate (1:1),
4-(2-fluoro-6-hydroxy-3-méthoxybenzoyl)benzoate de (3RS,4RS)-4-(4-hydroxy-3,5-diméthylbenzoylamino)-pyrrolidin-3-yle chlorhydrate (1:1),
4-(6-hydroxy-1,3-benzodioxol-5-ylcarbonyl)benzoate de (3RS,4RS)-4-(4-hydroxy-3,5-diméthylbenzoylamino)-pyrrolidin-3-yle chlorhydrate (1:1),
4-(2-hydroxy-5-méthoxybenzoyl)benzoate de (3RS,4SR)-1-(amino-iminométhyl)-4-(4-hydroxy-3,5-diméthylbenzoylamino)pyrrolidin-3-yle.

**15.** Composé selon la revendication 13, qui est le 4-(2-fluoro-6-hydroxy-3-méthoxybenzoyl)benzoate de (3R,4R)-3-(4-hydroxy-3,5-diméthylbenzoylamino)azépan-4-yle chlorhydrate (1:1).

**16.** Composés selon la revendication 10, dans lesquels $R^{12}$ est le groupe hydroxy et $R^{10}$, $R^{11}$, $R^{13}$ et $R^{14}$ sont des atomes d'hydrogène.

**17.** Composés selon la revendication 16, qui sont les composés suivants:

4-(2-fluoro-6-hydroxy-3-méthoxybenzoyl)benzoate de (3R,4R)-3-(4-hydroxybenzoylamino)azépan-4-yle chlorhydrate (1:1),
ester 3-(3-hydroxy-5,6,7,8-tétrahydronaphtalén-2-yl-carbonyl)benzoylamino-azépan-4-ylique chlorhydrate (1:1),
4-(2-hydroxy-5-diméthylaminobenzoyl)benzoate de (3R,4R)-3-(4-hydroxybenzoylamino)azépan-4-yle chlorhydrate (1:2),
4-(7-hydroxy-1,4-benzodioxin-6-ylcarbonyl)benzoate de (3R,4R)-3-(4-hydroxybenzoylamino)azépan-4-yle chlorhydrate (1:1),
4-[2-hydroxy-5-(1-méthoxyéthyl)benzoyl]benzoate de (3R,4R)-3-(4-hydroxybenzoylamino)azépan-4-yle chlorhydrate (1:1),
4-(6-hydroxyquinolin-7-ylcarbonyl)benzoate de (3R,4R)-3-(4-hydroxybenzoylamino)azépan-4-yle chlorhydrate (1:1),
4-(4-hydroxybiphényl-3-ylcarbonyl)benzoate de (3R,4R)-3-(4-hydroxybenzoylamino)azépan-4-yle chlorhydrate (1:1),
4-(5-acétyl-2-hydroxybenzoyl)benzoate de (3R,4R)-3-(4-hydroxybenzoylamino)azépan-4-yle chlorhydrate (1:1),
4-(2-hydroxy-10-méthylphénothiazin-1-ylcarbonyl)benzoate de (3R,4R)-3-(4-hydroxybenzoylamino)azépan-4-yle chlorhydrate (1:2),
4-(2-hydroxy-5-méthylbenzoyl)benzoate de (3R,4R)-3-(4-hydroxybenzoylamino)azépan-4-yle chlorhydrate (1:1),
4-(2-hydroxy-5-isopropylbenzoyl)benzoate de (3R,4R)-3-(4-hydroxybenzoylamino)azépan-4-yle chlorhydrate (1:1),
4-(2-fluoro-3-diméthylamino-6-hydroxybenzoyl)benzoate de (3R,4R)-3-(4-hydroxybenzoylamino)azépan-4-yle chlorhydrate (1:2),
4-(2-fluoro-6-hydroxy-3-pyrrolidin-1-ylbenzoyl)benzoate de (3R,4R)-3-(4-hydroxybenzoylamino)azépan-4-yle chlorhydrate (1:2),
4-(2-fluoro-6-hydroxy-3-méthylbenzoyl)benzoate de (3R,4R)-3-(4-hydroxybenzoylamino)azépan-4-yle chlorhydrate (1:1),
4-(2-fluoro-6-hydroxy-4-méthoxybenzoyl)benzoate de (3R,4R)-3-(4-hydroxybenzoylamino)azépan-4-yle chlorhydrate (1:1),
4-(3-hydroxynaphtalin-2-ylcarbonyl)benzoate de (3R,4R)-3-(4-hydroxybenzoylamino)azépan-4-yle chlorhydrate (1:1),
3,5-difluoro-4-(2-hydroxy-5-méthoxybenzoyl)benzoate de (3R,4R)-3-(4-hydroxybenzoylamino)azépan-4-yle

chlorhydrate (1:1),

4-(2-hydroxy-5-méthylthiobenzoyl)benzoate de (3R,4R)-3-(4-hydroxybenzoylamino)azépan-4-yle chlorhydrate (1:1),

4-(2-hydroxy-5-méthoxybenzoyl)benzoate de (3RS,4RS)-4-(4-hydroxybenzoylamino)pyrrolidin-3-yle chlorhydrate (1:1),

4-(2-hydroxy-5-méthoxybenzoyl)benzoate de (3RS,4RS)-4-(4-hydroxybenzoylamino)pyrrolidin-3-yle chlorhydrate (1:1), (3R,4R)-4-hydroxy-N-[4-[4-(2-hydroxy-5-méthoxybenzoyl)-benzyloxy]azépan-3-yl]benzamide chlorhydrate (1:1).

**18.** Composés selon la revendication 10, dans lesquels au moins l'un des radicaux $R^{10}$ à $R^{14}$ est un groupe alcoxy en $C_{1-6}$.

**19.** Composés selon la revendication 18, qui sont les composés suivants:

4-(2-fluoro-6-hydroxy-3-méthoxybenzoyl)benzoate de (3R,4R)-3-(4-hydroxy-3-méthoxybenzoylamino)azépan-4-yle chlorhydrate (1:1),

4-(2-fluoro-6-hydroxy-3-méthoxybenzoyl)benzoate de (3R,4R)-3-(4-hydroxy-3,5-diméthoxybenzoylamino)azépan-4-yle chlorhydrate (1:1),

4-(3-hydroxynaphtalin-2-ylcarbonyl)benzoate de (3R,4R)-3-(4-hydroxy-3,5-diméthoxybenzoylamino)azépan-4-yle chlorhydrate (1:1),

4-(2-hydroxy-5-méthoxybenzoyl)benzoate de (3R,4R)-3-(3,5-diméthoxybenzoylamino)azépan-4-yle chlorhydrate (1:1),

4-(2-fluoro-6-hydroxy-3-méthoxybenzoyl)benzoate de (3R,4R)-3-(2-hydroxy-3-méthoxybenzoylamino)azépan-4-yle chlorhydrate (1:1),

4-(2-fluoro-6-hydroxy-3-méthoxybenzoyl)benzoate de (3R,4R)-3-(3,5-diméthoxybenzoylamino)azépan-4-yle chlorhydrate (1:1),

4-(2-hydroxy-5-méthoxybenzoyl)benzoate de (3R,4R)-3-(3,4,5-triméthoxybenzoylamino)azépan-4-yle chlorhydrate (1:1),

4-(2-hydroxy-5-diméthylaminobenzoyl)benzoate de (3R,4R)-3-(4-hydroxy-3-méthoxybenzoylamino)azépan-4-yle chlorhydrate (1:2),

4-(2-hydroxy-5-méthoxybenzoyl)benzoate de (3R,4R)-3-(3,5-diéthoxybenzoylamino)azépan-4-yle chlorhydrate (1:1),

4-(2-fluoro-6-hydroxy-3-diméthylaminobenzoyl)benzoate de (3R,4R)-3-(4-hydroxy-3,5-diméthoxybenzoylamino)azépan-4-yle chlorhydrate (1:2),

4-(2-fluoro-6-hydroxybenzoyl)benzoate de (3R,4R)-3-(4-hydroxy-3-méthoxybenzoylamino)azépan-4-yle chlorhydrate (1:1),

4-[4-(2-fluoro-6-hydroxy)benzoyl)benzoate de (3R,4R)-3-(2-hydroxy-5-méthoxybenzoylamino)azépan-4-yle chlorhydrate (1:1),

4-(2-fluoro-6-hydroxy-4-méthoxybenzoyl)benzoate de (3R,4R)-3-(4-hydroxy-3,5-diméthoxybenzoylamino)azépan-4-yle chlorhydrate (1:1),

4-(2-fluoro-6-hydroxybenzoyl)benzoate de (3R,4R)-3-(4-hydroxy-3,5-diméthoxybenzoylamino)azépan-4-yle chlorhydrate (1:1),

4-(2,3-difluoro-6-hydroxybenzoyl)benzoate de (3R,4R)-3-(4-hydroxy-3,5-diméthoxybenzoylamino)azépan-4-yle chlorhydrate (1:1),

4-(2,4-difluoro-6-hydroxybenzoyl)benzoate de (3R,4R)-3-(4-hydroxy-3,5-diméthoxybenzoylamino)azépan-4-yle chlorhydrate (1:1),

(3R,4R)-2-hydroxy-N-[4-[4-(2-hydroxy-5-méthoxybenzoyl)-azépan-3-yl]-5-méthoxybenzamide chlorhydrate (1:1).

**20.** Composés selon la revendication 10, dans lesquels au moins l'un des radicaux $R^{11}$, $R^{13}$ et $R^{14}$ est un atome de fluor ou $R^{11}$ est un atome de brome.

**21.** Composés selon la revendication 20, qui sont les composés suivants:

4-(2-fluoro-6-hydroxy-3-méthoxybenzoyl)benzoate de (3R,4R)-3-(3-bromo-4-hydroxybenzoylamino)azépan-4-yle chlorhydrate (1:1),

4-(2-hydroxy-5-méthoxybenzoyl)benzoate de (3R,4R)-3-(5-fluoro-2-hydroxybenzoylamino)azépan-4-yle chlorhydrate (1:1).

**22.** Composés selon la revendication 10, dans lesquels $R^{12}$ et $R^{13}$ forment ensemble le radical -CH=CH-CH=CH- ou -C(OH)=CH-CH=CH-, dans lequel la liaison avec l'atome de carbone portant le groupe hydroxy s'effectue par $R^{12}$.

**23.** Composés selon la revendication 22, qui sont les composés suivants:

4-(2-fluoro-6-hydroxy-3-méthoxybenzoyl)benzoate de (3R,4R)-3-(3,5-dihydroxynaphtalén-2-ylcarbonylamino)-azépan-4-yle chlorhydrate (1:1),
3-(2-fluoro-6-hydroxy-3-méthoxybenzoyl)benzoate de (3R,4R)-3-(3-hydroxynaphtalén-2-ylcarbonylamino)azépan-4-yle chlorhydrate (1:1).

**24.** Composés selon la revendication 9, qui sont les composés suivants:

4-(2-hydroxy-5-méthoxybenzoyl)benzoate de (3R,4R)-3-(4-pyridinoylamino)azépan-4-yle chlorhydrate (1:2),
4-(2-fluoro-6-hydroxy-3-méthoxybenzoyl)benzoate de (3R,4R)-3-(4-pyridinoylamino)azépan-4-yle chlorhydrate (1:2),
4-(2-fluoro-6-hydroxy-3-méthoxybenzoyl)benzoate de (3RS,4RS)-4-pyridin-4-ylcarbonylaminopyrrolidin-3-yle chlorhydrate (1:2).

**25.** 4-(2-hydroxy-5-méthoxybenzoyl)benzoate de (3R,4R)-3-(3-acétyl-4-hydroxyvenzoylamino)azépan-4-yle chlorhydrate (1:1).

**26.** Composés de formule

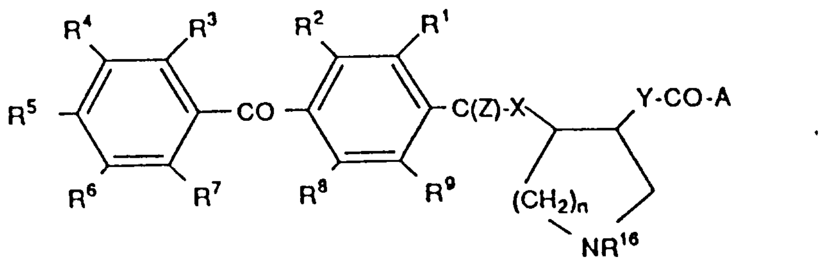

II

dans laquelle $R^{16}$ est un groupe protecteur et les autres symboles ont les significations données dans la revendication 1, les groupes hydroxy et amino représentés par $R^3$, $R^4$, $R^6$, $R^7$, $R^{12}$ et $R^{13}$ pouvant se trouver sous forme protégée.

**27.** Composés des revendications 1 à 25, pour utilisation en tant que médicaments.

**28.** Compositions pharmaceutiques, contenant comme substance active un composé des revendications 1 à 25 et des adjuvants pharmaceutiques usuels.

**29.** Utilisation des composés des revendications 1 à 25, pour la fabrication de médicaments destinés au traitement et à la prophylaxie de maladies médiées par des protéine kinases.

**30.** Procédé pour la préparation des composés des revendications 1 à 25, caractérisé en ce que l'on élimine le groupe protecteur $R^{16}$ et éventuellement des groupes protecteurs de fonction hydroxy et amino présents en tant que $R^3$, $R^4$, $R^6$, $R^7$, $R^{12}$ et $R^{13}$, d'un composé de formule

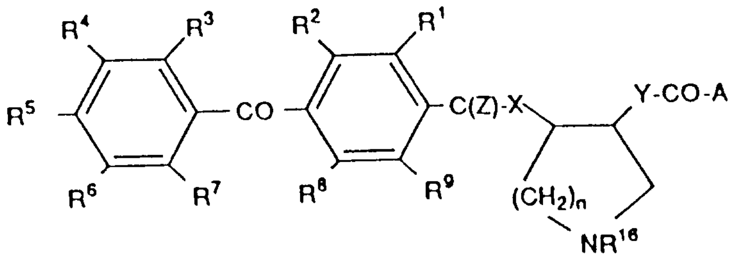

II

dans laquelle $R^{16}$ est un groupe protecteur et les autres symboles ont les significations données dans la revendication 1, les groupes hydroxy et amino représentés par $R^3$, $R^4$, $R^6$, $R^{12}$ et $R^{13}$ pouvant se trouver sous forme protégée,

si on le désire, on convertit le composé obtenu de formule I, dans lequel $R^{15}$ représente un atome d'hydrogène, en un composé de formule I dans lequel $R^{15}$ représente le groupe amidino, et, si on le désire, on convertit un composé obtenu de formule I en un sel pharmaceutiquement acceptable.